# EUROPEAN PATENT APPLICATION

(11) **EP 4 755 396 A1**
(43) Date of publication of application: **10.06.2026**
(21) Application number: 24848171.5
(22) Date of filing: 26.07.2024
(51) Int. Cl.: A61K 45/00, A61K 31/664, A61K 31/4535, A61K 31/675, A61K 31/519, A61K 31/396, A61P 35/00

(54) **TREATMENT OF P53 GENE MUTATION OR DEFECT NEGATIVE CANCER AND TUMOR PATIENTS**

(30) Priority: 28.07.2023 CN 202310939098; 13.11.2023 CN 202311512066; 27.03.2024 CN 202410365468
(71) Applicant: Ascentawits Pharmaceuticals, Ltd., Shenzhen, Guangdong 518122 (CN)
(72) Inventor: DUAN, Jianxin, Shenzhen, Guangdong 518000 (CN); QI, Tianyang, Shenzhen, Guangdong 518000 (CN); MENG, Fanying, San Francisco California 94121 (US); LIU, Xing, Shenzhen, Guangdong 518000 (CN); WANG, Yizhi, Shenzhen, Guangdong 518000 (CN); LI, Bing, Shenzhen, Guangdong 518000 (CN)
(74) Representative: Haseltine Lake Kempner LLP
(86) International application number: PCT/CN2024/107795
(87) International publication number: WO 2025/026214

(57) **Abstract**

A method of using a DNA alkylating agent prodrug compound-containing single drug or using the single drug and other drugs in combination for treatment of cancer and tumor patients with negative p53 gene mutation or defect, and its pharmaceutical use. In particular, the DNA alkylating agent prodrug compound is selected from a hypoxia-activated DNA alkylating agent prodrug compounds, an AKR1C3-activated DNA alkylating agent prodrug compounds, and a β-D-Glucosidase- or β-galactosidase-activated DNA alkylating agent prodrug compound.

## Description

### Technical field

The present invention relates to a method of treating cancer, and in particular to a treatment method of p53 gene mutation or defect negative cancer and tumor patients.

### Background

The chemical structure of DNA alkylating agent prodrug AST-3424 (WO2016145092, WO2017087428) targeting overexpressing Aldo-Keto reductase 1C3 (AKR1C3), CAS No. 2097713-69-2, is as follows:

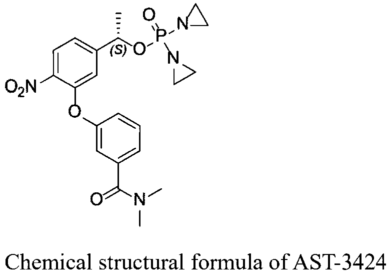

AST-3424 (also known as OBI-3424, TH-3424) enters cancer cells and is activated by AKR1C3 enzyme overexpressed by the cancer cell to release the metabolite AST-2660. AST-3424 itself is less toxic to cancer cells and its pharmacological effects are related to the expression of AKR1C3 enzyme in the animal models and in vitro pharmacological experiments: the prodrug AST-3424 is metabolized to AST-2660 under the action of AKR1C3 enzyme and NADPH, and the expression level of the enzyme is positively correlated with the drug efficacy (reference 1; reference 2; reference 3; reference 4).

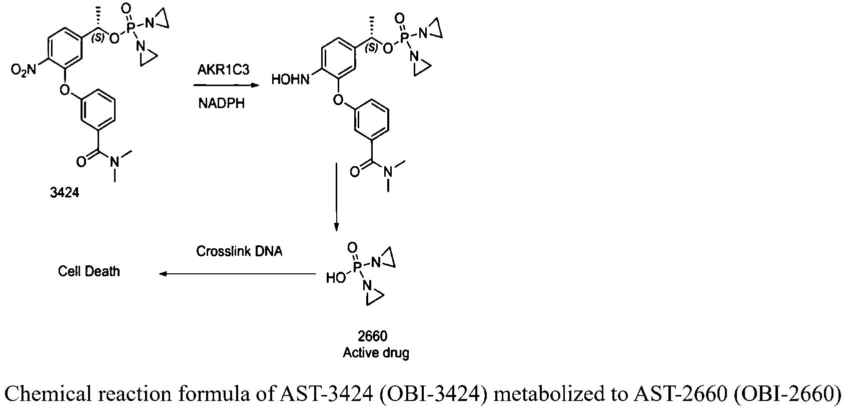

At present, the drug has entered phase I/II clinical trials in China and the United States, respectively (NCT03592264 in US, indication: liver cancer, pancreatic cancer and other solid tumors, sponsor: OBI Pharama Inc (4174), with the drug name OBI-3424; NCT04315324 in US, indication: T-ALL/T-LBL (acute T lymphoblastic leukemia/T lymphoblastic lymphoma), with the drug name OBI-3424; CTR20191399 in China, indication: various solid tumors, sponsor: Ascentawits Pharmaceuticals, LTD., with the drug name AST-3424; CTR20201915, indication: acute T-lymphocytic leukemia and acute B-lymphocytic leukemia, sponsor: Ascentawits Pharmaceuticals, LTD., with the drug name AST-3424).

At present, the clinical trials of the above-mentioned drugs are still in progress normally. Patients enrolled for treatment are selected by detecting their AKR1C3 enzyme expression levels in the corresponding phase II clinical trials.

### Summary of the Invention

In the phase II clinical trial of AST-3424 in China, the applicant found that the treatment effect to cancer and tumor patients with negative p53 gene mutation or defect is better than that of patients with positive p53 gene mutation or defect. Therefore, the applicant supposed that AST-3424 would have a better therapeutic effect in treating tumors and cancer patients with negative test results of p53 gene mutation or defect, that is, tumors and cancer patients with negative test results of p53 gene mutation or defect will have more obvious clinical benefits when receiving AST-3424 treatment.

Therefore, the applicant proposes the following applications of manufacture of anticancer drugs and methods for treating tumors and cancers.

A treatment method of using a single drug containing a DNA alkylating agent prodrug compound or in combination with other drugs for the treatment of cancer and tumor patients with negative p53 gene mutation detection or normal p53 protein expression.

An application of DNA alkylating agent prodrug compound in preparing a single drug or combined with other drugs in preparing drug for treating cancer and tumor patients with negative detection of p53 gene mutation or normal p53 protein expression.

A DNA alkylating agent prodrug compound refers to a prodrug compound that is capable of metabolizing a DNA alkylating agent in vivo.

A DNA alkylating agent prodrug compound means that the prodrug compound can be metabolically converted into DNA alkylating agent, and the DNA alkylating agent will eventually lead to alkylation of DNA in cancer cells, destroying the DNA structure of cancer cells and eventually causing cell death.

Most prodrug compounds may be metabolized and transformed in physiological environment. For anti-tumor and anti-cancer drugs, these physiological environments are usually unique microenvironments of tumor tissues or cancer cells, such as high expression of certain transporters on cell membranes, high expression of certain enzymes or proteins in intracellular or extracellular environments or concentration of certain enzymes or proteins higher than normal cells due to enrichment, or hypoxia or abnormal pH, etc. Therefore, the existence of these microenvironments is generally believed to be caused by some specific mechanisms of tumor tissues or cancer cells.

Tumor/cancer cell targeting in the above microenvironments is achieved by activating the metabolism of prodrugs into acting drugs through these microenvironments. Targets of microenvironments currently described in literatures include various enzyme activations, such as AKR1C3 enzyme activation, β-D-Glucosidase activation, Carboxylesterase activation, Esterase and caspase-3 activation, Cathepsin B activation, γ-Glutamyltranspeptidase activation, β-galactosidase activation or hypoxia activation.

The DNA alkylating agent prodrug compound is a DNA alkylating agent prodrug compound activated by the enzyme selected from the group consisting of AKR1C3 enzyme, β-D-Glucosidase, Carboxylesterase, Esterase and caspase-3, Cathepsin B, γ-Glutamyltranspeptidase, β-galactosidase or hypoxia-activation, preferably by AKR1C3 enzyme, β-D-Glucosidase, β-galactosidase, hypoxia-activation.

The DNA alkylating agent prodrug compound is selected from the group consisting of a hypoxia-activated DNA alkylating agent prodrug compounds, an AKR1C3-activated DNA alkylating agent prodrug compounds, and a β-D-Glucosidase-activated or β-galactosidase-activated DNA alkylating agent prodrug compound.

Preferably, the hypoxia-activated DNA alkylating agent prodrug compound is selected from the compound of formulae 1-3 and salt, ester, solvate and isotopic isomer thereof, the AKR1C3-activated DNA alkylating agent prodrug compound is selected from the compound of formulae 4-12 and salt, ester, solvate and isotopic isomer thereof, and the β-D-Glucosidase-activated or β-galactosidase-activated DNA alkylating agent prodrug compound is selected from the compound of formula 15 and salt, ester, solvate and isotopic isomer thereof. 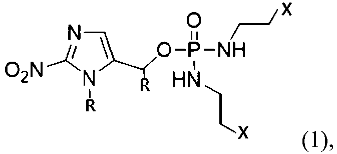 wherein R is each independently selected from H, -CH₃, -CH₂CH₃, -CF₃, and X is each independently selected from leaving groups such as Cl, Br, MsO, TsO, etc.

Formulations related to TH-302 or its analogous compounds 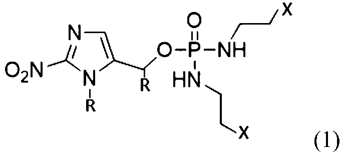 include oral preparations, freeze-dried preparations and concentrated injection solutions, and the related formulations, preparation methods, clinical combinations and administration methods are described and disclosed in detail in the related patents of Threshold Pharmaceuticals, Inc.: WO2010048330A1, WO2012142520A2, WO2008083101A1, WO2007002931A3, which are incorporated herein by reference in their entirety.

TH-302 or its analogous compound 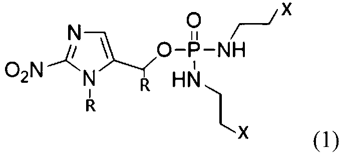 is a DNA alkylating agent anticancer drug with a wide range of cancer treatment potential and the related cancer indication experiments and clinical trials are published in the relevant patent application of Threshold Pharmaceuticals, Inc. and other pharmaceutical companies (such as WO2016011195A2, WO2004087075A1, WO2007002931A1, WO2008151253A2, WO2009018163A1, WO2009033165A2, WO2010048330A2, WO2012142520A1, WO2008083101A2, WO2020007106A1, WO2020118251A1, WO2014169035A1, WO2013116385A1, WO2019173799A2, WO2016081547A1, WO2014062856A1, WO2015069489A1, WO2012006032A2, WO2018026606A2, WO2010048330A2, WO2015171647A1, WO2013096687A1, WO2013126539A2, WO2013096684A2, WO2012009288A2, WO2012145684A2, WO2016014390A2, WO2019055786A2, WO2012135757A2, WO2015013448A2, WO2016011328A2, WO2013177633A2, WO2016011195A2, WO2015051921A2) and in the clinical trials registered by FDA (NCT02402062, NCT02020226, NCT02076230, NCT01381822, NCT02093962, NCT01440088, NCT02255110, NCT02342379, NCT01864538, NCT01149915, NCT02433639, NCT00743379, NCT01485042, NCT01721941, NCT02047500, NCT00742963, NCT01497444, NCT00495144, NCT01746979, NCT01144455, NCT01403610, NCT01522872, NCT01833546, NCT02598687, NCT03098160, NCT02496832, NCT02712567). The above patent applications and clinical trial information are incorporated herein by reference in their entirety. 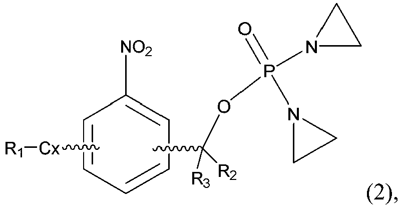 wherein, the definitions of R₁, R₂, R₃, and Cx are as described in the claims of Patent Application PCT/CN2020/114519, with Publication No. WO2021120717A1, and the synthetic preparation methods of specific compounds are also described in the above application, which is incorporated herein by reference in its entirety, and the specific definitions are:
Cx represents a 5-10 membered aromatic or heteroaromatic ring, aliphatic heterocyclic ring or cycloalkyl which shares two carbon atoms with the nitrophenyl ring to form a fused ring structure;
R₁ is attached to any ring member atom of the Cx ring and is selected from hydrogen, halogen atom, cyano or isocyano, hydroxyl, mercapto, amine, OTs, C₁-C₆ alkyl or Z-substituted alkyl, C₂-C₆ alkenyl or Z-substituted alkenyl, C₂-C₆ alkynyl or Z-substituted alkynyl, C₃-C₈ cycloalkyl or Z-substituted cycloalkyl, C₆-C₁₀ aryl or Z-substituted aryl, 4-15 membered heterocycle or Z-substituted heterocycle, 5-15 membered heteroaryl or Z-substituted heterocycle, alkoxy of 1-6 carbon atoms or Z-substituted alkoxy of 1-6 carbon atoms, -CONR⁶R⁷, - SO₂NR⁶R⁷, -SO₂R⁶, -OCOO-R⁶, -COOR⁶, -NR⁶COR⁷, -OCOR⁶, -NR⁶SO₂R⁷ or -NR⁶SO₂NR⁶R⁷;
R₂, R₃ are each independently hydrogen, C₁-C₆ alkyl or Z-substituted alkyl, C₂-C₆ alkenyl or Z-substituted alkenyl, C₂-C₆ alkynyl or Z-substituted alkynyl, C₃-C₈ cycloalkyl or Z-substituted cycloalkyl, C₆-C₁₀ aryl or Z-substituted aryl, 4-15 membered heterocycle or Z-substituted heterocycle, 5-15 membered heteroaryl or Z-substituted heteroaryl or a 3-6 membered ring formed by R₂, R₃ together with the benzyl carbon atom to which they are bonded; 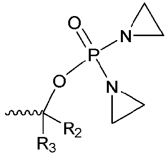 group may substitute the hydrogen atom at any position on the carbon atom of the fused ring, and the number of substitutions is 1;
the Z substituent is a halogen atom, a cyano or isocyano group, a hydroxyl group, a mercapto group, an amine group, a C₁-C₃ alkyl or substituted alkyl group, a C₁-C₃ alkoxy or substituted alkoxy group, a C₂-C₃ alkenyl or substituted alkenyl group, a C₂-C₃ alkynyl or substituted alkynyl group, a C₃-C₈ cycloalkyl or substituted cycloalkyl group;
R⁶, R⁷ are each independently hydrogen, C₁-C₆ alkyl or Z-substituted C₁-C₆ alkyl, C₂-C₆ alkenyl or Z-substituted C₂-C₆ alkenyl, C₂-C₆ alkynyl or Z-substituted C₂-C₆ alkynyl, C₃-C₈ cycloalkyl or Z-substituted C₃-C₈ cycloalkyl, C₆-C₁₀ aryl or Z-substituted C₆-C₁₀ aryl, 4-15 membered heterocyclyl or Z-substituted 4-15 membered heterocyclyl, 5-15 membered heterocyclyl or Z-substituted 5-15 membered heterocyclyl, or 5-7 membered heterocyclyl or Z-substituted 5-7 membered heterocyclyl formed by R⁶, R⁷ together with the atom to which they are bonded. 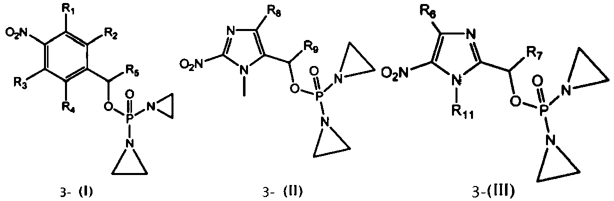 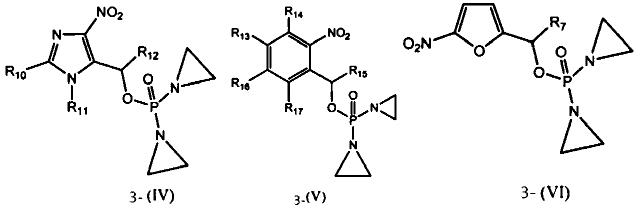 wherein, the definitions of R₁, R₂, R₃, R₄, R₅, R₆, R₇, R₈, R₉, R₁₀, R₁₁, R₁₂, R₁₃, R₁₄, R₁₅, R₁₆, and R₁₇ are as described in the claims of Patent Application PCT/US2016/039092 with Publication No. WO2016210175A1 (corresponding to Chinese Patent Application No. 2016800368985 with Publication No. CN108024974A), and the synthetic preparation methods of specific compounds are also described in the above application, which is incorporated herein by reference in its entirety, and the specific definitions are:
   R₁ is hydrogen, -N₃, CN, halo, NR²¹R²², -OR²³, -SO₂ (C₁-C₆ alkyl), C₁-C₆ alkyl, C₂-C₆ alkenyl, C₂-C₆ alkynyl, C₃-C₈ cycloalkyl, C₆-C₁₀ aryl, 4-15 membered heterocycle, 5-15 membered heteroaryl or ether;
   R²¹ and R²² are each independently hydrogen, hydroxy, C₁-C₆ alkyl, C₂-C₆ alkenyl, C₂-C₆ alkynyl, C₃-C₈ cycloalkyl, C₆-C₁₀ aryl, 4-15 membered heterocycle, 5-15 membered heteroaryl, or -SO₂ (C₁-C₆ alkyl); or a 4-15 membered heterocyclic ring or a 5-15 membered heteroaryl formed by R²¹ and R²² together with the nitrogen atom to which they are bonded;
   R²³ is hydrogen, C₁-C₆ alkyl or C₆-C₁₀ aryl;
   R₂ and R₃ are independently hydrogen or halo;
   R₄ is hydrogen, halo, C₁-C₆ alkoxy, C₁-C₆ alkyl or C₆-C₁₀ aryl,
   R₅, R₇, R₉, R₁₂ and R₁₅ are independently hydrogen, C₁-C₆ alkyl, C₂-C₆ alkenyl, C₂-C₆ alkynyl, C₃-C₈ cycloalkyl, C₆-C₁₀ aryl, 4-15 membered heterocycle, 5-15 membered heteroaryl; or C₅-C₆ cycloalkyl ring formed by R₄ and R₅ together with the intervening carbon atom therebetween;
   R₆ and R₁₀ are independently hydrogen or halo;
   R₈ is hydrogen, C₁-C₆ alkyl, C₂-C₆ alkenyl, C₂-C₆ alkynyl, or 5-15 membered heteroaryl;
   R₁₁ is each independently C₁-C₆ alkyl, C₂-C₆ alkenyl, C₂-C₆ alkynyl, C₃-C₈ cycloalkyl, or C₆-C₁₀ aryl;
   R₁₃, R₁₄, R₁₆, and R₁₇ are independently hydrogen, halo, C₁-C₆ alkyl, C₂-C₆ alkenyl, C₂-C₆ alkynyl, or C₁-C₆ alkoxy;
   wherein the alkyl, alkenyl, alkynyl, cycloalkyl, aryl, heterocyclic, heteroaryl, alkoxy and ether groups are optionally substituted. 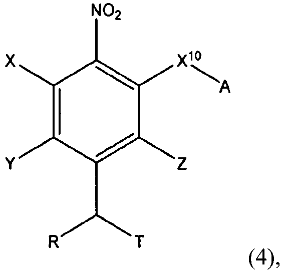 Formula (4) is an AKR1C3 enzyme-activated DNA alkylating agent prodrug compound, more specifically an AKR1C3 enzyme-activated DNA alkylating agent prodrug compound;
   wherein, the definitions of X, Y, Z, R, T, A and X₁₀ are as described in the claims of Patent Application PCT/US2016/021581 with Publication No. WO2016145092A1 (corresponding to Chinese Patent Application No. 2016800150788 with Publication No. CN107530556A), and the synthetic preparation methods of specific compounds are also described in the above application, which is incorporated herein by reference in its entirety, and the specific definitions are:
      X¹⁰ is O, S, SO or SO₂;
      A is C₆-C₁₀ aryl, 5-15 membered heteroaryl or-N=CR¹R²;
      R¹ and R² are each independently hydrogen, C₁-C₆ alkyl, C₃-C₈ cycloalkyl, C₆-C₁₀ aryl, 4-15 membered heterocycle, ether, -CONR¹³R¹⁴ or -NR¹³COR¹⁴;
      X, Y and Z are each independently hydrogen, CN, halo, C₁-C₆ alkyl, C₂-C₆ alkenyl, C₂-C₆ alkynyl, C₃-C₈ cycloalkyl, C₆-C₁₀ aryl, 4-15 membered heterocycle, ether, -CONR¹³R¹⁴ or -NR¹³COR¹⁴;
      R is hydrogen, C₁-C₆ alkyl, C₂-C₆ alkenyl, C₂-C₆ alkynyl, C₃-C₈ cycloalkyl, C₆-C₁₀ aryl, 4-15 membered heterocycle, ether, -CONR¹³R¹⁴ or -NR¹³COR¹⁴;
      R¹³ and R¹⁴ are each independently hydrogen, C₁-C₆ alkyl, C₃-C₈ cycloalkyl, C₆-C₁₀ aryl, 4-15 membered heterocyclic ring or ether;
      T comprises a phosphoramidate alkylating agent comprising one or more Z⁵-X⁵-Y⁵ moieties bonded to an -OP(Z¹) moiety, where Z⁵ is a heteroatom such as nitrogen, sulfur or oxygen, X⁵ is substituted or unsubstituted ethylene, Y⁵ is halogeno or another leaving group, or Z⁵-X⁵-Y⁵ together form an aziridinyl (NCH₂CH₂) moiety, and Z¹ is O or S; and
      wherein the alkyl, alkenyl, alkynyl, cycloalkyl, aryl, heterocyclic, heteroaryl, ether groups are substituted or unsubstituted. 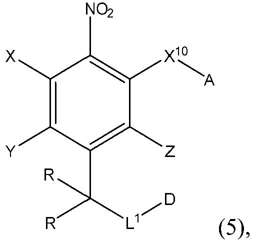
      wherein, the definitions of X, Y, Z, R, D, L¹, A and X¹⁰ are as described in the claims of Patent Application PCT/US2016/025665 with Publication No. WO2016161342A3 (corresponding to Chinese Patent Application No. 2016800200132 with Publication No. CN108136214A), and the synthetic preparation methods of specific compounds are also described in the above application, which is incorporated herein by reference in its entirety, and the specific definitions are:
         X¹⁰ is O, S, SO or SO₂;
         A is C₆-C₁₀ aryl, 5-15 membered heteroaryl or-N=CR¹R²;
         R¹ and R² are each independently hydrogen, C₁-C₆ alkyl, C₃-C₈ cycloalkyl, C₆-C₁₀ aryl, 4 to 15 membered heterocycle, 5 to 15 membered heteroaryl, ether, -CONR¹³R¹⁴ or -NR¹³COR¹⁴;
         X, Y and Z are each independently hydrogen, CN, halo, C₁-C₆ alkyl, C₂-C₆ alkenyl, C₂-C₆ alkynyl, C₃-C₈ cycloalkyl, C₆-C₁₀ aryl, 4 to 15 membered heterocycle, 5 to 15 membered heteroaryl, ether, -CONR¹³R¹⁴ or - NR¹³COR¹⁴;
         each R is independently hydrogen, C₁-C₆ alkyl, C₂-C₆ alkenyl, C₂-C₆ alkynyl, C₃-C₈ cycloalkyl, C₆-C₁₀ aryl, 4 to 15 membered heterocycle, 5 to 15 membered heteroaryl, ether, -CONR¹³R¹⁴ or -NR¹³COR¹⁴;
         R¹³ and R¹⁴ are each independently hydrogen, C₁-C₆ alkyl, C₃-C₈ cycloalkyl, C₆-C₁₀ aryl, 4 to 15 membered heterocycle, 5 to 15 membered heteroaryl or ether;
         wherein L¹ and D are defined as follows:
            L¹ is selected from: 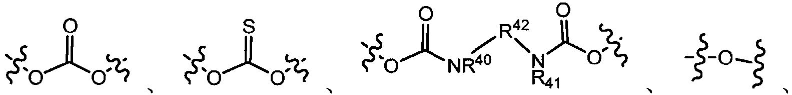 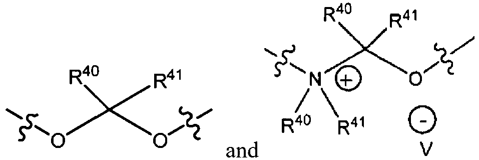
            R⁴⁰ and R⁴¹ are independently hydrogen, C₁-C₆ alkyl, C₂-C₆ alkenyl, C₂-C₆ alkynyl, C₃-C₈ cycloalkyl, C₆-C₁₀ aryl, 4 to 15 membered heterocycle, or 5 to 15 membered heteroaryl;
            R⁴² is C₂-C₃ alkylene or heteroalkylene, and optionally substituted with 1 to 3 C₁-C₆ alkyl groups;
            V (-) is any anion, preferably a pharmaceutically acceptable anion;
            D is a moiety such that D-OH is an anticancer drug, wherein OH is an aliphatic hydroxyl group or a phenolic hydroxyl group, or is an OH moiety attached to a phosphorus atom as provided herein; or L¹ is: 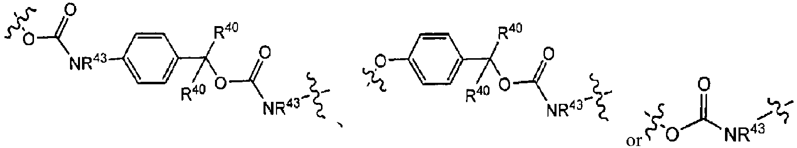
            R⁴⁰ is as defined above, R⁴³ is hydrogen or together with D forms a heterocycle, and the phenylene moiety is optionally substituted, and
            D is a moiety that makes D-NR⁴³H an anticancer drug; or
            L¹ is a bond, -O-C(R⁴⁰R⁴¹)-, -O-C(R⁴⁰R⁴¹)-NR⁴⁰R⁴¹(+)-C(R⁴⁰R⁴¹)- or 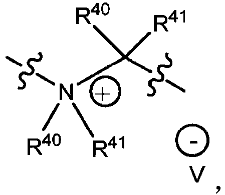
            wherein R⁴⁰, R⁴¹, and V are as defined above; and
            D is an anticancer drug containing tertiary amine or secondary amine, wherein the tertiary amine or the secondary amine is bonded to L¹; and
               and
            wherein the alkyl, alkenyl, alkynyl, cycloalkyl, aryl, heterocycle, heteroaryl, and ether are optionally substituted. 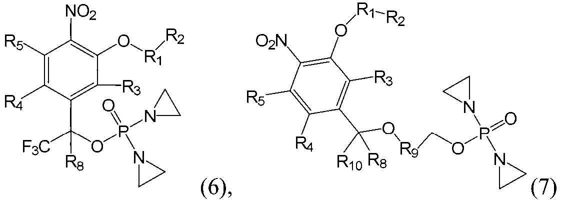
            wherein, the definitions of R₁, R₂, R₃, R₄, R₅, R₈, R₉, and R₁₀ are as described in the claims of Patent Application PCT/CN2020/089692, with Publication No. WO2020228685A1, and the synthetic preparation methods of specific compounds are also described in the above application, which is incorporated herein by reference in its entirety, and the specific definitions are:
               R₁ is C₆-C₁₀ aryl or Z-substituted aryl, a 4-15 membered heterocycle or Z-substituted heterocycle, a 5-15 membered heteroaryl or Z-substituted heteroaryl, a 7-15 membered fused ring or Z-substituted fused ring;
               R₂ is hydrogen, a halogen atom, cyano or isocyano, hydroxy, sulfhydryl, amino, OTs, OMS, C₁-C₆ alkyl or Z-substituted alkyl, C₂-C₆ alkenyl or Z-substituted alkenyl, C₂-C₆ alkynyl or Z-substituted alkynyl, C₃-C₈ cycloalkyl or Z-substituted cycloalkyl, C₆-C₁₀ aryl or Z-substituted aryl, 4-15-membered heterocycle or Z-substituted heterocycle, 5-15-membered heteroaryl or Z-substituted heteroaryl, ether having from 1 to 6 carbon atoms or Z-substituted alkoxy having from 1 to 6 carbon atoms, -CONR⁶R⁷, -SO₂NR⁶R⁷, -SO₂R⁶, -OCOO-R⁶, -COOR⁶, -NR⁶COR⁷, -OCOR⁶, -NR⁶SO₂R⁷ or -NR⁶SO₂NR⁶R⁷, or R² together with the atom in the group R¹ to which it is bonded to form a 7-15-membered fused ring or Z-substituted fused ring;
               R₃ is hydrogen, halogen, cyano or isocyano, hydroxy, sulfhydryl, amino, OTs, OMS, C₁-C₆ alkyl or Z-substituted alkyl, C₂-C₆ alkenyl or Z-substituted alkenyl, C₂-C₆ alkynyl or Z-substituted alkynyl, C₃-C₈ cycloalkyl or Z-substituted cycloalkyl, C₆-C₁₀ aryl or Z-substituted aryl, 4-15-membered heterocycle or Z-substituted heterocycle, 5-15-membered heteroaryl or Z-substituted heteroaryl, C₁-C₆ alkoxy or Z-substituted C₁-C₆ alkoxy, -CONR⁶R⁷, -SO₂NR⁶R⁷, -SO₂R⁶, -OCO-R⁶, -OCOO-R⁶, -COOR⁶, -NR⁶COR⁷, -OCOR⁶, or -NR⁶SO₂R⁷;
               R₄ and R₅ are each independently hydrogen, a halogen atom, cyano or isocyano, hydroxy, sulfhydryl, amino, OTs, OLCMS, C₁-C₆ alkyl or Z-substituted alkyl, C₂-C₆ alkenyl or Z-substituted alkenyl, C₂-C₆ alkynyl or Z-substituted alkynyl, C₃-C₈ cycloalkyl or Z-substituted cycloalkyl, C₆-C₁₀ aryl or Z-substituted aryl, 4-15-membered heterocycle or Z-substituted heterocycle, 5-15-membered heteroaryl or Z-substituted heteroaryl, C₁-C₆ alkoxy or Z-substituted C₁-C₆ alkoxy, -CONR⁶R⁷, -SO₂NR⁶R⁷, -SO₂R⁶, -OCOO-R⁶, -COOR⁶, -NR⁶COR⁶, - OCOR⁶ or -NR⁶SO₂R⁷, or R⁴ and R⁵ together with the atom in the benzene ring to which they are bonded to form a 7-15-membered fused ring or Z-substituted fused ring;
               R₆ and R₇ are each independently hydrogen, cyano or isocyano, C₁-C₆ alkyl or Z-substituted alkyl, C₂-C₆ alkenyl or Z-substituted alkenyl, C₂-C₆ alkynyl or Z-substituted alkynyl, C₃-C₈ cycloalkyl or Z-substituted cycloalkyl, C₆-C₁₀ aryl or Z-substituted aryl, 4-15-membered heterocycle or Z-substituted heterocycle, 5-15-membered heteroaryl or Z-substituted heteroaryl, C₁-C₆ alkoxy or Z-substituted C₁-C₆ alkoxy, or R⁶ and R⁷ together with the atom to which they are bonded to form 5-7-membered heterocyclyl or Z-substituted 5-7-membered heterocyclyl;
               R₈ and R₁₀ are each independently hydrogen, deuterium, aryl or Z-substituted aryl, C₁-C₆ alkyl or Z-substituted alkyl, C₂-C₆ alkenyl or Z-substituted alkenyl, C₂-C₆ alkynyl or Z-substituted alkynyl, C₃-C₈ cycloalkyl or Z-substituted cycloalkyl, and at least one of R⁸ and R¹⁰ must be hydrogen or deuterium;
               R₉ is substituted C₆-C₁₀ aryl which is substituted with at least one fluorine atom or nitro group, substituted 4-15-membered heterocycle which is substituted with at least one fluorine atom or nitro group, or substituted 5-15-membered heteroaryl which is substituted with at least one fluorine atom or nitro group;
               the substituent Z is a halogen atom, cyano or isocyano, hydroxy, sulfhydryl, amino, OTs, OMS, C₁-C₃ alkyl or substituted alkyl, C₁-C₃ alkoxy or substituted alkoxy, C₂-C₃ alkenyl or substituted alkenyl, C₂-C₃ alkynyl or substituted alkynyl, C₃-C₈ cycloalkyl or substituted cycloalkyl, an aromatic ring, heterocycle, heteroaromatic ring and fused ring or a substituted aromatic ring, heterocycle, heteroaromatic ring and fused ring, the pattern of substitution being mono- or gem-di-substitution;
               in R₉, the substitution in the substituted C₆-C₁₀ aryl, substituted 4-15-membered heterocycle or substituted 5-15-membered heteroaryl is a halogen atom, nitro, cyano or isocyano, hydroxy, amino, C₁-C₃ alkyl or alkoxy, alkenyl, alkynyl, cycloalkyl or benzene ring, substituted benzene ring, C₁-C₃ alkoxy or halogen atom-substituted alkoxy.

The compound of formula (8) is an AKR1C3 enzyme-activated alkylating agent prodrug compound, and more specifically, it is an AKR1C3 enzyme-activated DNA alkylating agent prodrug compound; 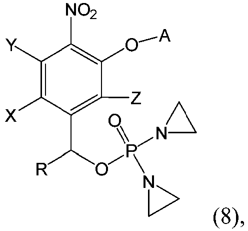 wherein:
A is substituted or unsubstituted C₆-C₁₀ aryl, biaryl or substituted biaryl, 5-15-membered heteroaryl, or - N=CR¹R²; wherein the substituent is selected from the group consisting of halogeno, -CN, -NO₂, -O-(CH₂)-O-, -CO₂H and salt thereof, -OR¹⁰⁰, -CO₂R¹⁰⁰, -CONR¹⁰¹R¹⁰², -NR¹⁰¹R¹⁰², -NR¹⁰⁰SO₂R¹⁰⁰, -SO₂R¹⁰⁰, -SO₂NR¹⁰¹R¹⁰², C₁-C₆ alkyl, and C₃-C₁₀ heterocyclyl;
wherein R¹⁰⁰, R¹⁰¹ and R¹⁰² are each independently hydrogen, C₁-C₈ alkyl, or C₆-C₁₂ aryl; or R¹⁰¹ and R¹⁰² together with the nitrogen atom to which they are attached form a 5-7-membered heterocycle;
wherein the alkyl group and the aryl group are each substituted by 1-3 halogeno groups or 1-3 C₁-C₆ alkyl groups;
R¹ and R² are each independently phenyl or methyl;
X, Y and Z are each independently hydrogen or halogeno; and
R is hydrogen or C₁-C₆ alkyl or halogen-substituted alkyl. 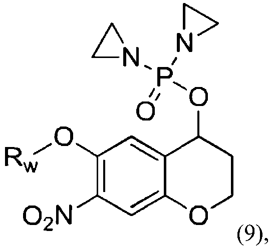
wherein, the definition of Rw has been described in the claims of Patent Application PCT/CN2020/120281 with Publication No. WO2021068952A1, which describes the synthetic preparation methods of specific compounds as well and is incorporated herein by reference in its entirety, with detailed definitions being:
   Rw is 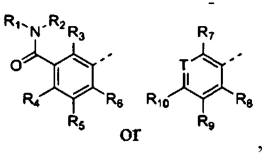
   R¹ is H, C₁₋₆ alkyl, C₃₋₆ cycloalkyl, 4-6-membered heterocycloalkyl, 5-6-membered heteroaryl or phenyl, wherein the C₁₋₆ alkyl, C₃₋₆ cycloalkyl, 4-6-membered heterocycloalkyl, 5-6-membered heteroaryl and phenyl are optionally substituted with 1, 2 or 3 R^{a} groups;
   each R^{a} is independently H, F, Cl, Br, I, -CN, -OH, C₁₋₃ alkoxy or C₁₋₃ alkyl;
   R₂ is H or C₁₋₆ alkyl;
   or R₁ and R₂, together with the N atom to which they are attached, to form a 4-6-membered heterocycloalkyl, wherein the 4-6-membered heterocycloalkyl is optionally substituted with 1, 2 or 3 R^{b} groups;
   each R^{b} is independently H, F, Cl, Br, I, -CN, -OH, -NH₂, -OCH₃, -OCH₂CH₃, -CH₃ or -CH₂CH₃;
   R₃ is H, F, Cl, Br, I, -OH, -NH₂, C₁₋₃ alkoxy or C₁₋₃ alkyl;
   or R₂ and R₃ are attached together to make the structural unit 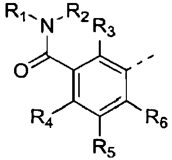 to be 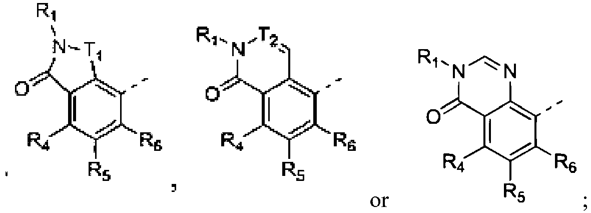
   T₁ is -(CR^{c}R^{d})ₘ- or -(CR^{c}R^{d})ₙ-O-;
   m is 1, 2 or 3;
   n is 1 or 2;
   T₂ is N or CH;
   R^{c} and R^{d} are each independently H, F, C₁₋₃ alkyl or C₁₋₃ alkoxy;
   R₄, R₅ and R₆ are each independently H, F, Cl, Br, I, C₁₋₃ alkyl or C₁₋₃ alkoxy;
   T is N or CH;
   R₇ and R₈ are each independently H, F, Cl, Br or I;
   R₉ and R₁₀ are each independently H, F, Cl, Br, I, -CN; or
   the 4-6-membered heterocycloalkyl and 5-6-membered heteroaryl each contain 1, 2, 3 or 4 heteroatoms independently selected from N, -O- and -S-. 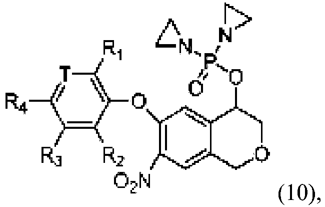
   wherein, the definitions of R₁, R₂, R₃, R₄ and T are as described in the claims of Patent Application PCT/CN2021/118597, with Publication No. WO2022057838A1, and the synthetic preparation methods of specific compounds are also described in the above application, which are incorporated herein by reference in its entirety, with detailed definitions being:
      T is N or CH;
      R₁ and R₂ are each independently H, F, Cl, Br, I or C₁₋₃ alkyl, wherein said C₁₋₃ alkyl is optionally substituted with 1, 2 or 3 Rₐ;
      each Rₐ is independently F, Cl, Br, I, -CN, -OH, or-NH₂;
      R₃ and R₄ are each independently H, F, Cl, Br, I, CN, C₁₋₃ alkyl, C₁₋₃ alkoxy, 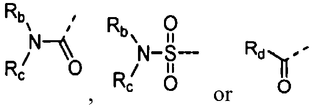
      wherein the C₁₋₃ alkyl is optionally substituted with 1, 2 or 3 Rₑ;
      R_{b} and R_{c} are each independently H, -CH₃, -CH₂CH₃, -(CH₂)₂CH₃, -CH (CH₃)₂;
      R_{d} is-CH₃, -CH₂CH₃, -(CH₂)₂CH₃, -CH(CH₃)₂;
      each Rₑ is independently F, Cl, Br, I, -CN, -OH, or-NH₂. 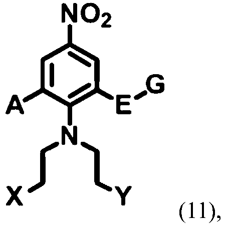
      wherein, the definitions of A, E, G, X and Y are described in the claims of Patent Application PCT/NZ2019/050030 with Publication No. WO2019190331A1 (corresponding to Chinese Patent Application No. 2019800234236 with Publication No. CN111918864A), which describes the synthetic preparation methods of specific compounds as well and is incorporated herein by reference in its entirety, with detailed definitions being:
         A is H, C₁-C₆ alkyl, C₁-C₆ alkenyl, C₁-C₆ alkynyl, CFH₂, CF₂H, CF₃, F, Cl, Br, I, OCF₃, COR or CON(R)₂;
         E is SO or SO₂;
         X is Cl, Br, I or OSO₂R;
         Y is Cl, Br, I or OSO₂R;
         each R is independently H or C₁-C₆ alkyl;
         G is a radical group selected from the group consisting of Formulae (B)-(AA): 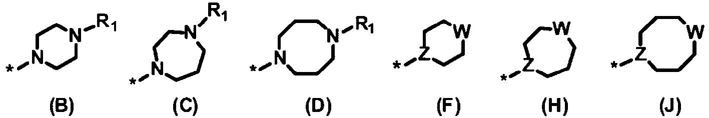 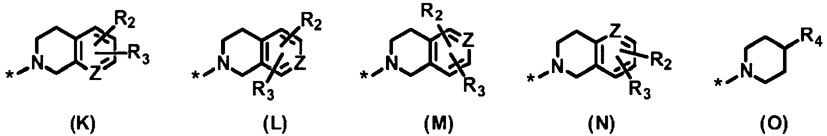 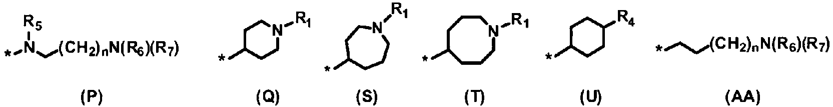
         wherein:
            R₁ is H, C₁-C₆ alkyl, CH₂(CH₂)ₙOH, CH₂CH(OH)CH₂OH, phenyl, pyridyl, benzyl, or pyridylmethyl, provided that when R₁ is phenyl, pyridyl, benzyl or pyridylmethyl, R₁ is optionally substituted at any available position with C₁-C₆ alkyl, C₁-C₆ alkenyl, C₁-C₆ alkynyl, OR₆, N(R₆)(R₇), CFH₂, CF₂H, CF₃, F, Cl, Br, I, OCF₃, COR₆, CON(R₆)(R₇), SOR₆, SON(R₆)(R₇), SO₂R₆, SO₂N(R₆)(R₇), CN, or NO₂;
            R₂ and R₃ are each independently H, C₁-C₆ alkyl, C₁-C₆ alkenyl, C₁-C₆ alkynyl, OR₆, N(R₆)(R₇), CFH₂, CF₂H, CF₃, F, Cl, Br, I, OCF₃, COR₆, CON(R₆)(R₇), SOR₆, SON(R₆)(R₇), SO₂R₆, SO₂N(R₆)(R₇), CN or NO₂;
            R₄ is N(R₆)(R₇), OH, OCH₂(CH₂)ₙN(R₆)(R₇) or CH₂(CH₂)ₙN(R₆)(R₇);
            R₅ is H or a C₁-C₆ alkyl group;
            R₆ and R₇ are each independently H or C₁₋₆ alkyl, or R₆ and R₇ together form a substituted or unsubstituted 5-membered or 6-membered heterocycle;
            Z is CH or N;
            W is CH₂, O, S, SO or SO₂;
            n is 0 to 6;
            * represents a point of attachment to Formula (I). 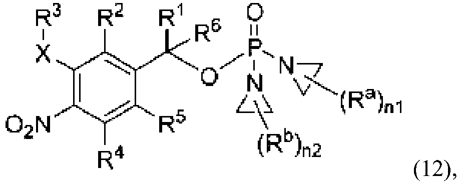 or pharmaceutically acceptable salt thereof,
            wherein, the definitions of R¹, R², R³, R⁴, R⁵, R⁶, Rₐ, R_{b}, n1, and n2 are as described in the claims of Patent Application PCT/CN2023/123253 with Publication No. WO2024078392A1, and which describes the synthetic preparation methods of specific compounds as well and is incorporated herein by reference in its entirety, with detailed definitions being:
               wherein:
               (II) R¹, R² are joined together with inserting atoms to form an optionally substituted 4-8 membered carbocyclic or heterocyclic ring, R⁴ and R⁵ being as defined in (I); or
               (III) R¹, R⁵ are joined together with inserting atoms to form an optionally substituted 4-8 membered carbocyclic or heterocyclic ring, R² and R⁴ being as defined in (I); or
               (IV) R⁴, R⁵ are joined together with inserting atoms to form an optionally substituted 4-8 membered carbocyclic or heterocyclic ring, R¹ and R² being as defined in (I) or (II); or
               (I) R¹ is hydrogen, deuterium, optionally substituted C₁₋₄ alkyl, optionally substituted C₂₋₄ alkenyl or optionally substituted C₂₋₄ alkynyl;
            R², R⁴, R⁵ are each independently hydrogen, halogen (e.g. F), optionally substituted C₁₋₄ alkyl, optionally substituted C₂₋₄ alkenyl, optionally substituted C₂₋₄ alkynyl, optionally substituted C₁₋₄ alkoxy, or optionally substituted 3-5 membered ring;
            wherein:
               X is O, S, NR¹⁰, optionally substituted C₁₋₄ alkylene or optionally substituted C₁₋₄ heteroalkylene, wherein R¹⁰ is hydrogen, optionally substituted C₁₋₄ alkyl, optionally substituted 3-6 membered ring or nitrogen protecting group;
               R³ is hydrogen, optionally substituted C₁₋₄ alkyl, or an optionally substituted 3-10 membered ring;
               R⁶ is hydrogen, deuterium, optionally substituted C₁₋₄ alkyl, optionally substituted C₂₋₄ alkenyl or optionally substituted C₂₋₄ alkynyl;
               the integers n1, n2 are each independently 0, 1, 2, 3, or 4;
               each R^{a}, R^{b} is independently at each occurrence optionally substituted C₁₋₄ alkyl or optionally substituted C₁₋₄ heteroalkylene; or two examples R^{a} or two examples R^{b} are joined together with an inserting atom to form an optionally substituted 3-6 membered ring, and any remaining examples R^{a} and/or R^{b} are as defined above. 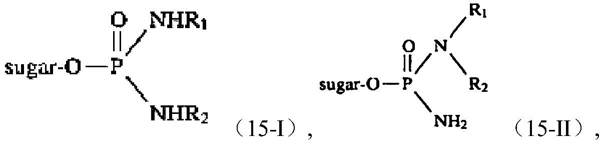
               wherein, the definitions of Sugar, R₁, and R₂ are as described in the claims of Patent Application US5622936A,
               and which describes the synthetic preparation methods of specific compounds as well and is incorporated herein by reference in its entirety, with detailed definitions being:
                  wherein the sugar moiety is attached to a phosphamide mustard residue (15-I) or an ifosfamide mustard residue (15-II), R₁ and R₂, which may be the same or different, are selected from hydrogen, C1-C4 alkyl or C1-C6 haloalkyl,
                  and the sugar moiety is an isomeric or enantiomeric form of any existing monosaccharide, disaccharide or polysaccharide.

For other structures of DNA alkylating agents activated by β-D-Glucosidase, Carboxylesterase, Esterase and caspase-3, Cathepsin B, γ-Glutamyltranspeptidase, and β-galactosidase, please refer to the review (Han HH, Wang HM, Jangili P, et al. The design of small-molecule prodrugs and activatable phototherapeutics for cancer therapy. Chem Soc Rev. 2023; 52 (3): 879-920. Published 2023 Feb 6. doi: 10.1039/d2cs00673a).

The compound with formula (1) is selected from the compounds with the following structural formulae:

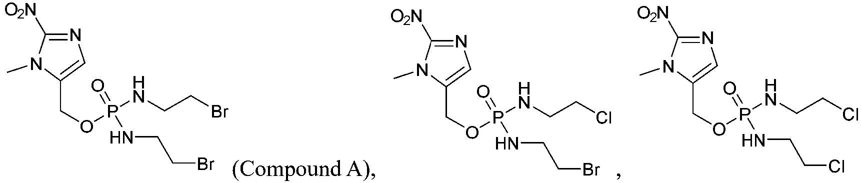

The synthetic preparation method of the compound is also described in Patent Application PCT/US2006/025881, with Publication No. WO2007002931 (corresponding to Chinese Application No. 2006800300828, with Publication No. CN101501054A), which is incorporated herein by reference in its entirety.

The compound with formula (2) is selected from the compounds with the following structural formulae:

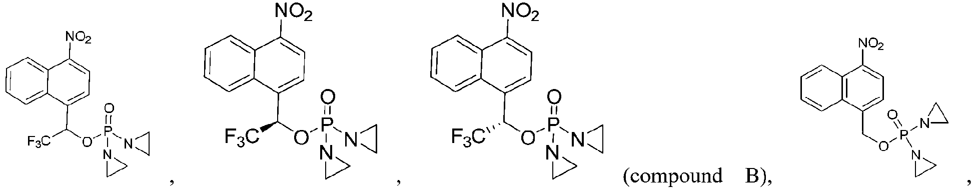

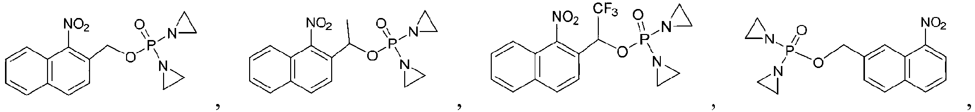

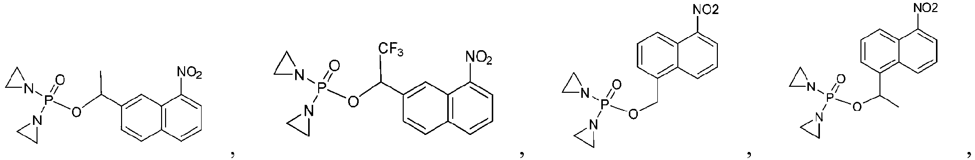

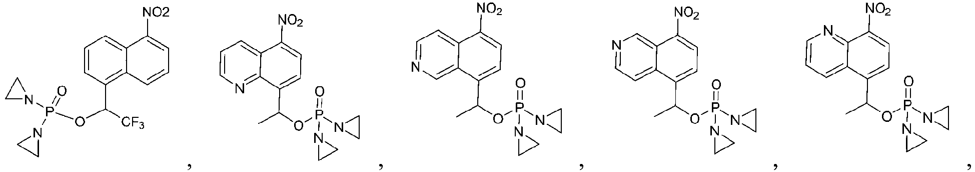

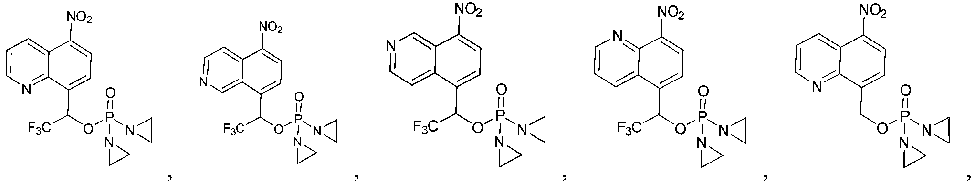

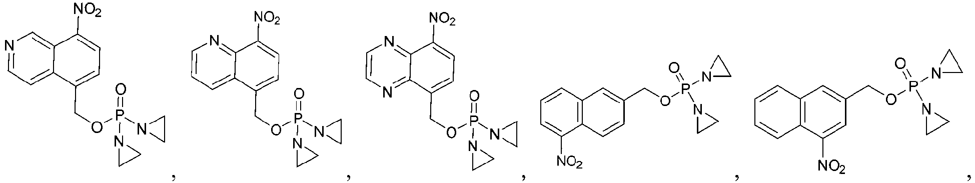

The synthetic preparation method of the compound is also described in Patent Application PCT/CN2020/114519, with Publication No. WO2021120717A1 (corresponding to Chinese Application No. 2020800673113, with Publication No. CN114466853A), which is incorporated herein by reference in its entirety.

The compound with formula (3) is selected from the compounds with the following structural formulae:

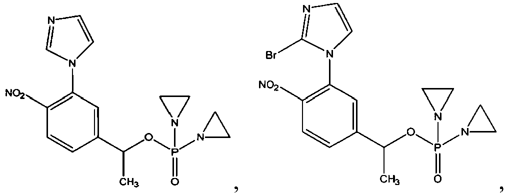

The synthetic preparation method of the compound is also described in Patent Application PCT/US2016/039092, with Publication No. WO2016210175A1 (corresponding to Chinese Application No. 2016800368985, with Publication No. CN108024974A), which is incorporated herein by reference in its entirety.

The compound with formula (4) is selected from the compounds with the following structural formulae: 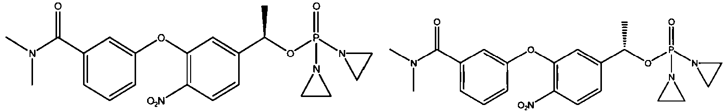 (AST-3424 compound),

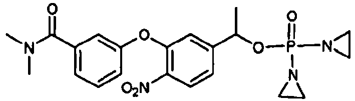

The synthetic preparation method of the compound is also described in Patent Application PCT/US2016/021581, with Publication No. WO2016145092A1 (corresponding to Chinese Application No. 2016800150788, with Publication No. CN107530556A), which is incorporated herein by reference in its entirety.

The compound with formula (5) is selected from the compounds with the following structural formulae:

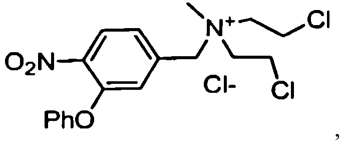

The synthetic preparation method of the compound is also described in Patent Application PCT/US2016/025665, with Publication No. WO2016161342A3 (corresponding to Chinese Application No. 2016800200132, with Publication No. CN108136214A), which is incorporated herein by reference in its entirety.

The compounds of structural formulae (6) and (7) are selected from the compounds of the following structure:

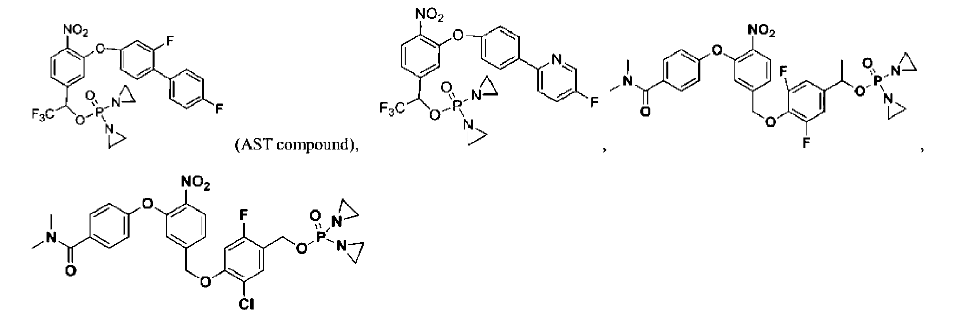

The synthetic preparation method of the compound is also described in Patent Application PCT/CN2020/089692, with Publication No. WO2020228685A9 (corresponding to Chinese Application No. 2020800358890, with Publication No. CN113853379A), which is incorporated herein by reference in its entirety.

The compound with formula (8) is selected from the compounds with the following structural formulae:

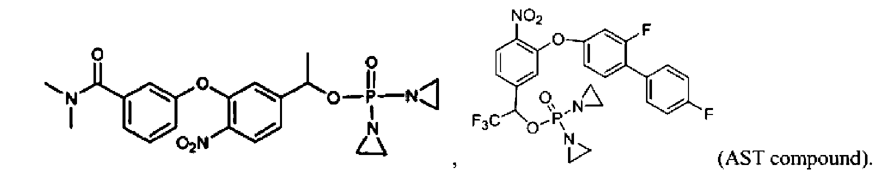

The compound with formula (9) is selected from the compounds with the following structural formulae:

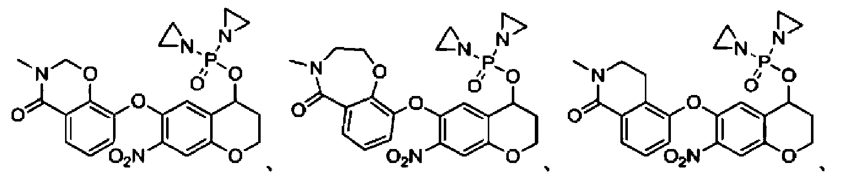

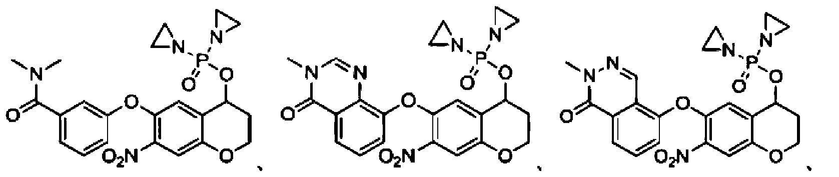

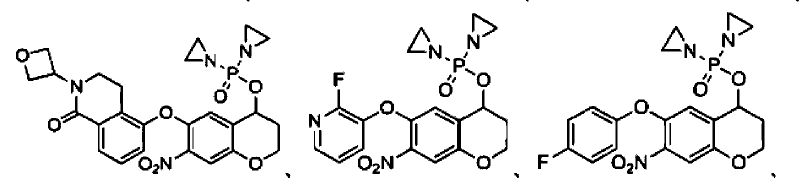

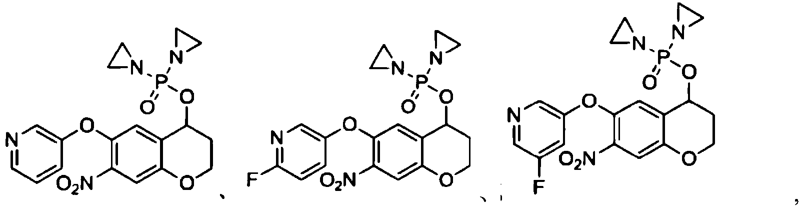

The synthetic preparation method of the compound is also described in Patent Application PCT/CN2020/120281, with Publication No. WO2021068952A1 (corresponding to Chinese Application No. 202080071652.8, with Publication No. CN114555574A), which is incorporated herein by reference in its entirety.

The compound with formula (10) is selected from the compounds with the following structural formulae:

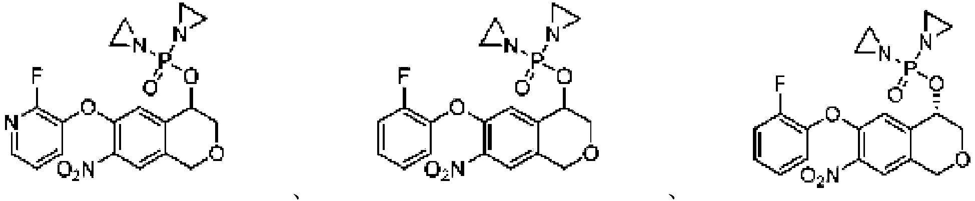

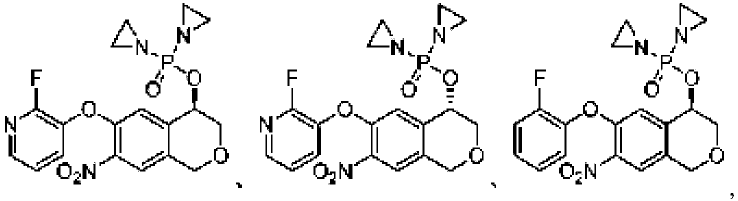

The synthetic preparation method of the compound is also described in patent application PCT/CN2021/118597, Publication No. WO2022057838 A1, which is incorporated herein by reference in its entirety.

The compound with formula (11) is selected from the compounds with the following structural formulae:

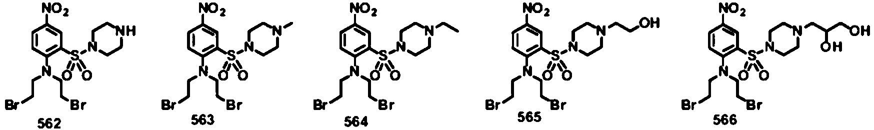

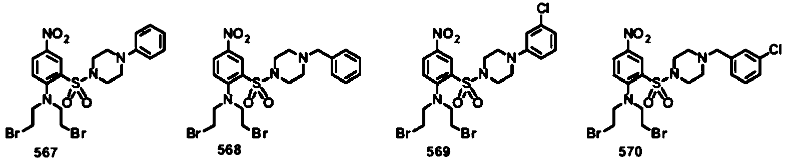

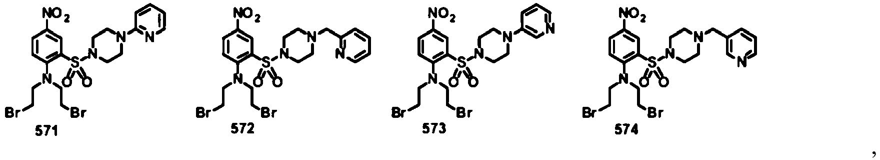

The synthetic preparation method of the compound is also described in Patent Application PCT/NZ2019/050030, with Publication No. WO2019190331A1 (corresponding to Chinese Application No. 2019800234236, with Publication No. CN111918864A), which is incorporated herein by reference in its entirety.

The AKR1C3 enzyme activating compound of formula (12) is selected from compounds 1-569 in Table 1-15 of Patent Application PCT/CN2023/123253, with Publication No. WO2024078392A1, and the structures of some compounds are listed as follows:

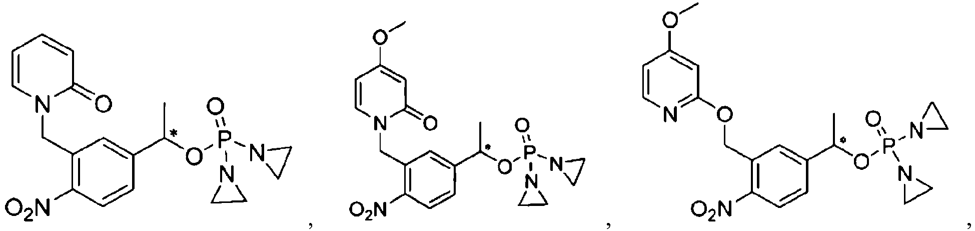

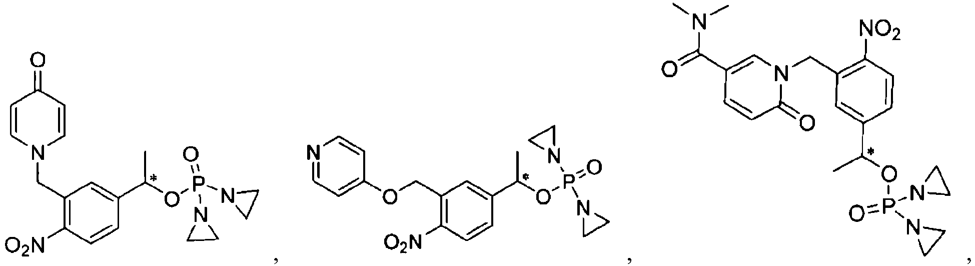

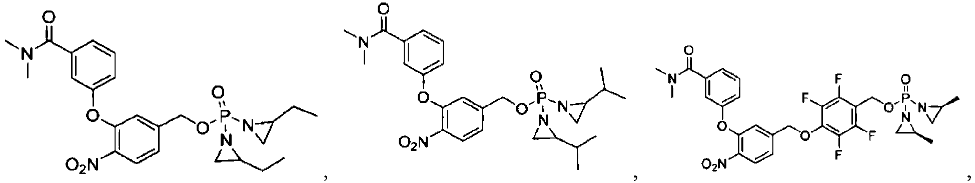

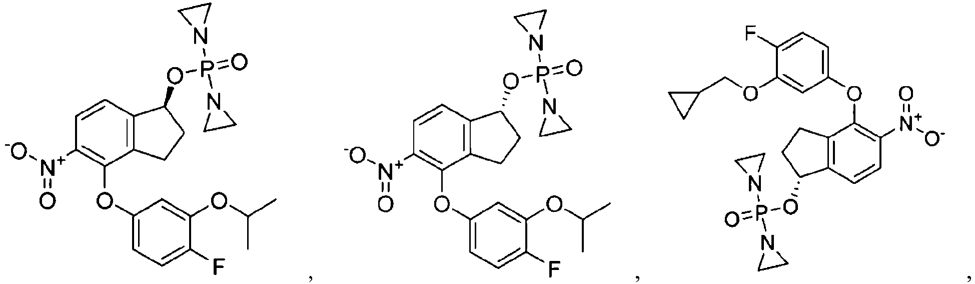

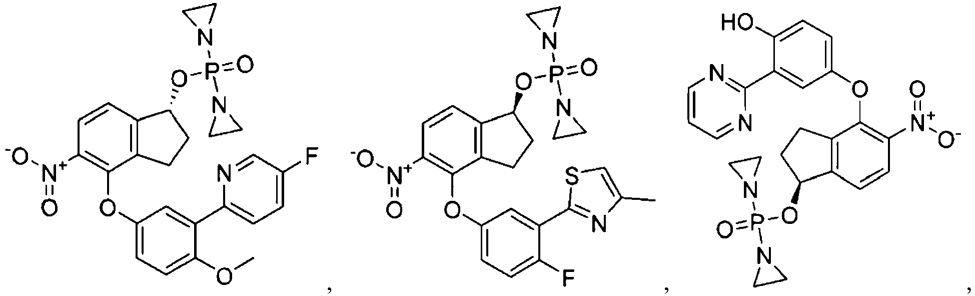

The specific synthesis method of compounds 1-569 of formula (12), together with the corresponding spectroscopic data, is disclosed in WO2024078392 A1, which is incorporated herein by reference in its entirety. The compound with formula (15) is selected from the compounds with the following structural formulae:

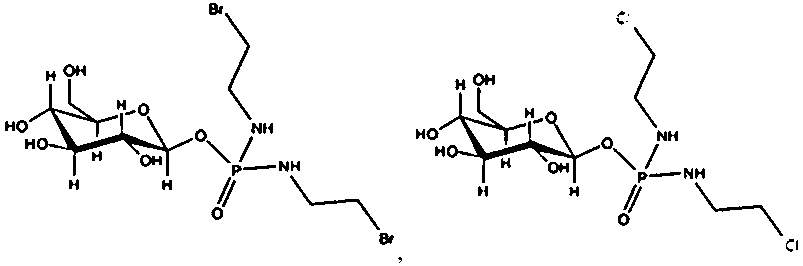

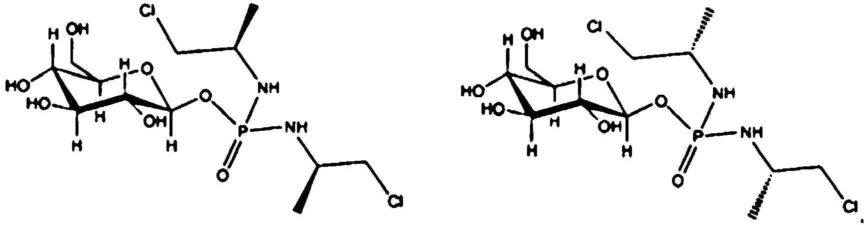

The synthetic preparation method of the compounds is also described in U.S. Patent Application No. 5,622,936 A, PCT Application No. PCT/US2007/074012 with Publication No. WO2008011588, which are incorporated herein in their entirety.
the DNA alkylating agent prodrug compound is selected from AKR1C3 activated DNA alkylating agent prodrug compound AST-3424:

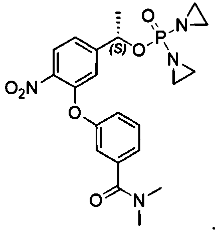

The above AST-3424 is formulated as an aqueous solution for intravenous injection,
where the solute of the aqueous solution consists of the active pharmaceutical ingredient AST-3424, glucose, ethanol, propylene glycol and pH regulator sodium bicarbonate,
wherein the concentration of the active pharmaceutical ingredient AST-3424 in the aqueous solution is 0.004-0.94 mg/ml, the pH is 7.4, the content of glucose is 4.5-5.0% by mass and the solution is an isotonic solution; or
the solute of the aqueous solution consists of the active pharmaceutical ingredient AST-3424, sodium chloride, ethanol, propylene glycol and pH regulator sodium bicarbonate,
wherein the concentration of the active pharmaceutical ingredient AST-3424 in the aqueous solution is 0.004-0.94 mg/ml, the pH is 7.4, the content of sodium chloride is 0.81-0.90% by mass, and the solution is an isotonic solution.

The preparation method of AST-3424 as a concentrated solution for injection and the preparation method of the aqueous solution for intravenous injection described above are both described in Patent Application PCT/CN2020/101870, with Publication No. WO20210085201.

The AST-3424 dosing regimen is selected from any one of the following regimen:
regimen 1, every 21-day cycle, with one dose administered on Day 1 and Day 8 each, at a dose selected from 12 mg/m², 11 mg/m², 10 mg/m², 9 mg/m², 8 mg/m², 7 mg/m², 6 mg/m², 5 mg/m², 4 mg/m² per administration;
regimen 2, every 21-day cycle, with one dose administered on Day 1, at a dose selected from 14 mg/m², 13 mg/m², 12 mg/m², 11 mg/m², 10 mg/m², 9 mg/m², 8 mg/m², 7 mg/m², 6 mg/m², 5 mg/m², 4 mg/m², 3 mg/m², 2 mg/m², 1 mg/m² per administration;
regimen 3, every 21-day cycle, with one dose administered once a day from Day 1 to Day 5, at a dose selected from 8 mg/m², 7 mg/m², 6 mg/m², 5 mg/m², 4 mg/m², 3 mg/m², 2 mg/m², 1 mg/m² per administration.

For detailed information of the above three AST-3424 dosing regimens and their doses, please refer to clinical trial NCT03592264 in the US and the clinical trials CTR20191371, CTR20191399 in China.

For AST-3424 the patient is a liver cancer patient.

Preferably, for AST-3424, the liver tumor tissue of the patient is strongly positive for AKR1C3 expression, and the liver tumor tissue of the patient is judged to be strongly positive for AKR1C3 expression if one of the following conditions is met:
I. the H score is greater than or equal to 100, preferably greater than or equal to 135, more preferably greater than or equal to 200, as detected by the AKR1C3 detection method described in WO2022048492;
II. the proportion of tumor cells having a staining intensity of 2 + and/or 3 + is ≥ 70%, as detected by the AKR1C3 detection method described in WO2022048492.

Criteria I is determined according to the OBI-3424 Phase I clinical trial NCT03592264 conducted in the United States, referred to in the academic literature (Journal of Clinical Oncology. 40.3030-3030.10.1200/JCO.2022. 40.16_suppl.3030) and in the academic literature (Tsimberidou, A.M., Verschraegen, C.F., Wesolowski, R. et al. Phase 1 dose-escalation study evaluating the safety, pharmacokinetics, and clinical activity of OBI-3424 in patients with advanced or metastatic solid tumors. Br J Cancer 129, 266-274 (2023). https://doi.org/10. 1038/s41416-023-02280-4).

Criteria II is determined according to the AST-3424 Phase I clinical trial CTR20191371 conducted in China, and the phase II clinical trial is currently conducted according to this criterion. For the enrollment criteria of phase II clinical trial, please refer to CTR20191399.

In the text described in WO2022048492, various IHC methods for measuring the expression level of AKR1 C3 enzyme are provided, wherein a preferred AKR1C3 measuring method uses immunohistochemical staining to detect the expression level of AKR1C3 in *ex vivo* formalin-fixed paraffin-embedded (FFPE) human tissue sections, comprising the following steps:
a1) Dewaxing and rehydration
   The formalin-fixed paraffin-embedded human tissue sections are dewaxed with an organic solvent, and the dewaxed sections are washed sequentially with alcohols with different water contents, and finally washed with water;
a) Antigen retrieval
   In the presence of antigen retrieval solution, formalin-fixed paraffin-embedded human tissue sections after dewaxing and rehydration are heated at 90-115°C for 17-30 minutes for antigen retrieval;
b1) Blocking non-specific antigens
   Incubate the formalin-fixed paraffin-embedded human tissue sections after antigen retrieval with blocking solution to block non-specific antigens;
b) Primary antibody incubation
   The formalin-fixed paraffin-embedded human tissue sections after blocking are mixed with AKR1C3 monoclonal antibody solution with a concentration of 0.5-5.0 µg/ml and incubated for 25-700 minutes;
c) Secondary antibody incubation
   The formalin-fixed paraffin-embedded human tissue sections incubated with the primary antibody are mixed with a secondary antibody solution with a concentration of 0.5-5.0 µg/ml, and incubated for 25-700 minutes;
d) staining and mounting
   Formalin-fixed paraffin-embedded human tissue sections are stained with hematoxylin, dehydrated and mounted after staining;
e) Observation score
   The stained human tissue sections are observed, and the expression level of AKR1C3 in the human tissue sections is evaluated according to the observed staining degree.

In the text described in WO2022048492, the score calculation method of the H-scoring system is:
H-Score = (% of cells with staining level 1+) × 1 + (% of cells with staining level 2+) × 2 + (% of cells with staining level 3+) × 3. If the IHC staining result of a certain liver cancer tissue shows that the cells with staining level 1 is 10%, the cells with staining level 2 is 20%, and the cells with staining level 3 is 50%, then the H-score is 10 +40 +150 = 200.

The drug containing AST-3424 is used in combination with a drug containing any one selected from abiraterone, prednisolone, 5- fluorouracil, sunitinib, oxaliplatin, PD-1/L1 inhibitor, apatinib, sorafenib or donafenib, or elemene for treating liver cancer.

The p53 gene is the Tumor protein p53 gene (Tumor protein p53), also known as the tp53 gene.

In the present application, p53 (protein) and p53 (gene) may be used interchangeably and are not distinguished. The negative mutation of p53 gene in the present application includes cases where no mutation is detected and cases where the mutation is detected but does not reach the defined degree of positive.

At present, relevant testing kits have been approved for commercial use and can be commercially available for testing, such as AmoyDx^{®} tp53 gene six mutations detection kit tp53, Six Mutations Detection Kit, produced by Amoy Diagnostics Co., Ltd., China, p53 gene amplification detection kit (fluorescence in situ hybridization method), FISH detection Kit for the p53 gene, produced by Celnovte Biotechnology Co., Ltd., China, and Archer^{®} VariantPlex^{™} p53 kit for Illumina^{®} produced by Integrated DNA Technologies, Inc., USA. Normal expression of p53 protein includes normal expression, high expression and overexpression higher than normal. Normal values are artificially delineated values based on clinical practice statistics.

The normal expression of p53 protein can be measured by comparing typical WB experiments or by IHC in medical clinics. Through these methods, the amount of p53 protein in the detection sample is directly measured, and then compared with the normal value or the set threshold value to determine whether it is normal expression or high expression.

It can also be screened by gene detection. Generally, if a pathogenic mutation in a gene is detected, it can be determined that p53 protein is not normally expressed or highly expressed; if no gene mutation is detected or a gene mutation is detected but not a pathogenic mutation, there is a high probability that p53 protein is normally expressed. Therefore, it is possible to determine whether the p53 protein is normally expressed/highly expressed by detecting whether the p53 gene has a pathogenic mutation.

The above-mentioned gene mutation or protein expression detection results are generally obtained by detecting tumor or cancer tissue, cells or other biological detection samples of the patient.

The biological test sample comprises a peripheral blood sample, tumor tissue or suspected tumor tissue, a thin layer cytology sample, a fine needle aspiration sample, a bone marrow sample, a lymph node sample, a urine sample, an ascites sample, a lavage sample, an esophageal brush sample, a bladder or lung wash sample, a spinal fluid sample, a brain fluid sample, a catheter aspiration sample, a nipple effusion sample, a pleural effusion sample, a fresh frozen tissue sample, a paraffin embedded tissue sample, or an extract or a treated sample obtained from any one of a peripheral blood sample, tumor tissue or suspected tumor tissue, a thin layer cytology sample, a fine needle aspiration sample, a bone marrow sample, a urine sample, an ascites sample, a lavage sample, an esophageal brush sample, a bladder or lung wash sample, a spinal fluid sample, a brain fluid sample, a catheter aspiration sample, a nipple effusion sample, a pleural effusion sample, a fresh frozen tissue sample or a paraffin embedded tissue sample. Venous whole blood or saliva is frequently used.

With respect to the compounds described herein, the chemical structure such as AST-3424 contains an organic amine structure and a P=O double bond structure, and therefore the compounds may also be administered as a salt, i.e. the present invention provides pharmaceutically acceptable salts of the compounds, which may be a basic salt, including salts of the compounds with inorganic bases (e.g. alkali metal hydroxides, alkaline earth metal hydroxides, etc.) or with organic bases (e.g. monoethanolamine, diethanolamine, or triethanolamine, etc.). Alternatively, the salt may be an acid salt, including salts of the compounds with inorganic acids such as hydrochloric acid, hydrobromic acid, hydroiodic acid, nitric acid, perchloric acid, sulfuric acid or phosphoric acid, and the like, or with an organic acid such as methanesulfonic acid, trifluoromethanesulfonic acid, ethanesulfonic acid, benzenesulfonic acid, p-toluenesulfonic acid, fumaric acid, oxalic acid, maleic acid, citric acid, and the like. Similarly, it is also possible to react with certain acids or alcohols to form esters, and therefore the compounds may also be administered as esters.

For various reasons, these compounds may also form solvates with certain solvents, which are hydrates or alcoholates, and therefore the compounds may also be administered as solvates. The selection and preparation of acceptable salts, esters, solvates, and the like of the compounds is a skill well known in the art.

The term "isotopic variant" refers to a compound containing an isotope in a non-natural ratio at one or more of the atoms comprised in such compound. In certain embodiments, an "isotopic variant" of a compound contains one or more isotopes in a non-natural ratio, including, but not limited to, hydrogen (¹H), deuterium (²H), tritium (³H), carbon-11 (¹¹C), carbon-12 (¹²C), carbon-13 (¹³C), carbon-14 (¹⁴C), nitrogen-13 (¹³N), nitrogen-14 (¹⁴N), nitrogen-15 (¹⁵O), oxygen-15 (¹⁵O), oxygen-16 (¹⁶O), oxygen-17 (¹⁷O), oxygen-18 (¹⁸O), fluorine-17 (¹⁷F), fluorine-18 (¹⁸F), phosphor-31 (³¹P), phosphor-32 (³²P), phosphor-33 (³³P), sulfur-32 (³²S), sulfur-33 (³³S), sulfur-34 (³³S), sulfur-35 (³⁵S), sulfur-36 (³⁶S), chlorine-35(³⁵Cl), chlorine-36(³⁶Cl), chlorine-37(³⁷Cl), bromine-79 (⁷⁹Br), bromine-81 (⁸¹Br), iodine-123 (¹²³I), iodine-125 (¹²⁵I), iodine-127 (¹²⁷I), iodine-129 (¹²⁹I), and iodine-131 (¹³¹I). In certain examples, the "isotopic variant" of the compound is in a stable form, i.e., non-radioactive. In certain examples, an "isotopic variant" of a compound contains one or more isotopes in a non-natural ratio, including, but not limited to, hydrogen (¹H), deuterium (²H), carbon-12 (¹²C), carbon-13 (¹³C), nitrogen-14 (¹⁴N), nitrogen-15 (¹⁵N), oxygen-16 (¹⁶O), oxygen-17 (¹⁷O), oxygen-18 (¹⁸O), fluorine-17 (¹⁷F), phosphorus-31 (³¹P), sulfur-32 (³²S), sulfur-33 (³³S), sulfur-34 (³⁴S), sulfur-36 (³⁶S), chlorin-35 (³⁵Cl), chloro-37 (³⁷Cl), bromine-79 (⁷⁹Br), bromine-81 (⁸¹Br) and iodine-127 (¹²⁷I). In certain examples, the "isotopic variant" of the compound is in an unstable form, i.e., radioactive. In certain examples, an "isotopic variant" of a compound contains one or more isotopes in a non-natural ratio, including, but not limited to, tritium (³H), carbon-11 (¹¹C), carbon-14 (¹⁴C), nitrogen-13 (¹³N), oxygen-14 (¹⁴O), oxygen-15 (¹⁵O), fluorine-18 (¹³F), phosphorus-32 (³²P), phosphorus-33 (³³P), sulfur-35 (³⁵S), chloro-36 (³⁶Cl), iodine-123 (¹²³I), iodine-125 (¹²⁵I), iodine-129 (¹²⁹I), and iodine-131 (¹³¹I). It is to be understood that in the compounds provided herein, any hydrogen may be, for example, ²H, i.e. D, or any carbon may be, for example, ¹³C, or any nitrogen may be, for example, ¹⁵N, and any oxygen may be ¹⁸O, as practicable in the judgment of those skilled in the art. In certain examples, an "isotopic variant" of a compound contains deuterium (D) in a non-natural ratio.

The pharmaceutical as described herein refers to a pharmaceutical product or a preparation in which the pharmaceutical product contains a hypoxia-activating compound of formula (1) or a salt or solvate thereof as an active ingredient in a specific dosage range, and/or the pharmaceutical product is administered in a specific dosage form and in a specific mode of administration.

The prepared pharmaceutical product, medicament, and preparation may also contain pharmaceutically acceptable adjuvants or excipients. The medicament may be in any dosage form for clinical administration, such as tablets, suppositories, dispersible tablets, enteric-coated tablets, chewable tablets, orally disintegrating tablets, capsules, dragees, granules, dry powders, oral solutions, small needles for injection, lyophilized powder injections, or large infusions. Depending on the specific dosage form and mode of administration, the pharmaceutically acceptable adjuvants or excipients in the medicament may include one or more of the following: diluents, solubilizers, disintegrants, suspending agents, lubricants, binders, fillers, flavoring agents, sweetening agents, antioxidants, surfactants, preservatives, encapsulating agents, and pigments.

Monotherapy refers to single drug therapy. Combination therapy refers to combined drug therapy. Single drug therapy refers to the use of only one anticancer drug in a course of treatment. Combination therapy refers to the simultaneous or sequential use of two or more anticancer drugs in a course of treatment.

Generally speaking, for combination therapy, different administration dosages and administration cycles need to be explored according to the characteristics of the disease and the types of drugs to be used in combination. Only according to the above conditions, the combined drug therapy regimen obtained from the exploration may achieve better therapeutic effect as compared with single drug therapy.

The drug dosage, administration cycle, and dosing schedule of monotherapy and combination therapy regimens are obtained through clinical trials.

Examples of AST-3424 used in combination with other drugs have been described in the following references: wherein, for AST-3424 used in combination with abiraterone acetate or abiraterone + prednisolone combination, sunitinib and gemcitabine, please refer to Patent Application PCT/CN2021/078115, with Publication No. WO2022178821 and academic literature (Meng, Fanying et al. "A novel selective AKR1C3-activated prodrug AST-3424/OBI-3424 exhibits broad anti-tumor activity." American journal of cancer research vol. 11, 7 3645-3659. 15 Jul. 2021). Herein, AST-3424, the combination of abiraterone acetate or abiraterone and prednisolone is a triple therapy, using three drugs, that is, on the basis of the existing combination of abiraterone acetate or abiraterone + prednisolone, the third drug AST-3424 is used for the combination treatment of three drugs.

For AST-3424 used in combination with 5-fluorouracil, please refer to Patent Application PCT/CN2021/078115, with Publication No. WO2022178821 and academic literature (Meng, Fanying et al. "A novel selective AKR1C3-activated product AST-3424/OBI-3424 exhibits broad anti-tumor activity." American journal of cancer research vol. 11, 7 3645-3659.15 Jul. 2021) and academic literature (Zhang, Yu et al. "The In-Vitro Antitumor Effects of AST-3424 Monotherapy and Combination Therapy With Oxaliplatin or 5-Fluorouracil in Primary Liver Cancer." Frontiers in oncology vol. 12 885139. 22 Jul. 2022, doi: 10.3389/fonc.2022. 885139). For AST-3424 used in combination with oxaliplatin, please refer to academic literature (Zhang, Yu et al. "The In-Vitro Antitumor Effects of AST-3424 Monotherapy and Combination Therapy With Oxaliplatin or 5-Fluorouracil in Primary Liver Cancer." Frontiers in oncology vol. 12 885139. 22 Jul. 2022, doi: 10.3389/fonc.2022. 885139).

For AST-3424 used in combination with PD-1/L1 inhibitors, please refer to Patent Application PCT/US2021/29552, with Publication No. WO2022231580, and academic conference poster (Chun-Chung Wang, Wan-Fen Li, Chih-Chan Lee, Lu-Tzu Chen, Jhih-Jie Yang, Jiann-Shiun Lai, Ming-Tain Lai; Abstract 6111; OBI-3424, an AKR1C3-activated prodrug, exhibits in vivo synergistic anti-tumor effect in combination with pembrolizumab by induction of immunogenic cell death. Cancer Res 15 June 2022; 82 (12 _ Supplement): 6111. https://doi.org/10.1158/1538-7445.AM2022-6111. The poster can be downloaded from the official website of OBI Pharma Inc at https://www.obipharma.com/zh-hant/news-zh-hant/news-2022-zh-hant/poster-presentations-at-aacr-2022-annual-meeting-for-obi-3424-and-globo-h-science//)

For AST-3424 used in combination with apatinib, sorafenib or donafenib, or elemene, please refer to the academic literature (Xun, Chen et al. "A novel AKR1C3 specific prodrug AST-3424 and its combination therapy in hepatocellular carcinoma." Journal of pharmacological sciences vol. 152, 2 (2023): 69-75. doi: 10.1016/j.jphs.2023. 03.004). Donafenib is a deuterated drug of sorafenib, and the combination treatment of AST-3424 and sorafenib has been described in the above references, so those skilled in the art can readily infer that AST-3424 can also be used in combination with its deuterated drug donafenib.

### BRIEF DESCRIPTION OF THE DRAWINGS

Figure 1 shows the *in vitro* proliferation inhibition rate curves of AST-3424/AST in combination with Nutlin-3 on H460 cells.
Figure 2 shows the *in vitro* proliferation inhibition rates of AST-3424 alone, Nutlin-3 alone and the two in combination with different administration sequences on H460 cells.
Figure 3 shows the effects of AST-3424 alone, Nutlin-3 alone and the two in combination with different administration sequences on colony formations of H460/HPAF-II cells.
Figure 4 shows the effects of AST-3424 alone, Nutlin-3 alone and the two in combination with different doses on the apoptosis process of H460 cells.
Figure 5 shows the first experimental result of the effects of the combination of AST-3424 and Nutlin-3 on the cell cycle G2/M arrest of H460/HPAF-II cells. The bar chart shows G2/M, S, and G0/G1 phases respectively from top to bottom.
Figure 6 shows the second experimental result of the effects of the combination of AST-3424 and Nutlin-3 on the cell cycle G2/M arrest. The bar chart shows G2/M, S, and G0/G1 phases respectively from top to bottom.
Figure 7 shows the bar chart of the *in vitro* proliferation inhibition results of AST alone, Nutlin-3 alone and the two in combination on H460 cells. The three bars from left to right in the chart indicate the inhibition rate of AST alone, Nutlin-3 alone and AST+Nutlin-3 in combination respectively.
Figure 8 shows the bar chart of the *in vitro* proliferation inhibition results of AST alone, RITA alone and the two in combination on H460 cells. The three bars from left to right in the chart indicate the inhibition rate of AST alone, RITA alone and AST+RITA in combination respectively.
Figure 9 shows the bar chart of the *in vitro* proliferation inhibition results of AST alone, Nutlin-3 alone and the two in combination on HPAF-II cells. The three bars from left to right in each group in the figure indicate the inhibition rate of AST alone, Nutlin-3 alone and AST+Nutlin-3 in combination respectively. The six groups of charts from left to right correspond to the experimental groups 1-6 in the table respectively.
Figure 10 shows the bar chart of the *in vitro* proliferation inhibition results of AST alone, RITA alone and the two in combination on HPAF-II cells. The three bars from left to right in the chart indicate the inhibition rate of AST alone, RITA alone and AST+RITA in combination respectively.
Figure 11 shows the WB detection results of the cell protein lysate in the first experiment of treating H460 cells with AST-3424 alone, Nutlin-3 alone and the two in combination. In this figure, the upper shows the WB-detected protein bands, and the lower shows the ratios of the corresponding proteins to the internal reference protein β-actin.
Figure 12 shows the WB detection results of the protein in the cell lysate in the second experiment of treating H460 cells with AST-3424 alone, Nutlin-3 alone and the two in combination. In this figure, the upper shows the WB-detected protein bands, and the middle and the lower show the ratios of the corresponding proteins to the internal reference protein β-actin.
Figure 13 shows the WB detection results of the protein in the cell lysate in the third experiment of treating H460 cells with AST-3424 alone, Nutlin-3 alone and the two in combination. In this figure, the upper shows the WB-detected protein bands, and the middle and the lower show the ratios of the corresponding proteins to the internal reference protein β-actin.
Figure 14 shows the WB detection results of the RAD51 protein in the cell lysate in the first experiment of treating H460 cells with AST-3424 alone, Nutlin-3 alone and the two in combination. In this figure, the left shows the WB-detected protein bands, and the right shows the ratios of the corresponding proteins to the internal reference protein β-actin.
Figure 15 shows the WB detection results of the RAD51 protein in the cell lysate in the second experiment of treating H460 cells with AST-3424 alone, Nutlin-3 alone and the two in combination. In this figure, the upper shows the WB-detected protein bands, and the lower shows the ratios of the corresponding proteins to the internal reference protein β-actin.
Figure 16 shows the WB detection results of the RAD51 protein in the cell lysate in the third experiment of treating H460 cells with AST-3424 alone, Nutlin-3 alone and the two in combination. In this figure, the upper shows the WB-detected protein bands, and the lower shows the ratios of the corresponding proteins to the internal reference protein β-actin.
Figure 17 shows the WB detection results of the RAD51 protein in the cell lysate in the experiment of treating H460 cells with AST-3424 in combination with Nutlin-3 and MG-132. In this figure, the upper shows the WB-detected protein bands, and the lower shows the ratios of the corresponding proteins to the internal reference protein β-actin.
Figure 18 shows the WB detection results of the RAD51 protein in the cell lysate in the experiment of treating H460 cells with AST-3424 in combination with Nutlin-3 and Cycloheximide. In this figure, the upper shows the WB-detected protein bands, and the lower shows the ratios of the corresponding proteins to the internal reference protein β-actin.
Figure 19 shows the change curves of the relative levels of RAD51 after treating with different drugs for 24h, i.e., 1% DMSO, 0.1 nM AST-3424, 5 µM Nutlin-3, 0.1 nM AST-3424 + 5 µM Nutlin-3, followed by adding 4 µM Cycloheximide for different time periods.
Figure 20 shows the WB detection results of the γH2AX protein in the cell lysate in the first experiment of treating H460 cells with AST-3424 alone, Nutlin-3 alone and the two in combination. In this figure, the upper shows the WB-detected protein bands, and the lower shows the ratios of the corresponding proteins to the internal reference protein β-actin.
Figure 21 shows the WB detection results of the γH2AX protein in the cell lysate in the second experiment of treating H460 cells with AST-3424 alone, Nutlin-3 alone and the two in combination. In this figure, the upper shows the WB-detected protein bands, and the lower shows the ratios of the corresponding proteins to the internal reference protein β-actin.
Figure 22 shows the WB detection results of the γH2AX protein in the cell lysate in the third experiment of treating H460 cells with AST-3424 alone, Nutlin-3 alone and the two in combination. In this figure, the upper shows the WB-detected protein bands, and the lower shows the ratios of the corresponding proteins to the internal reference protein β-actin.
Figure 23 shows the WB detection results of the p53, Rad51, MDM2, p21 proteins in the cell lysate in the experiment of treating HPAF-II cells with AST alone, Nutlin-3 alone and the two in combination. In this figure, the upper shows the WB-detected protein bands, and the lower shows the bar charts of ratios of the corresponding proteins to the internal reference protein β-actin.
Figure 24 shows the WB detection results of the cell protein lysate in the experiment of treating HPAF-II cells with AST-3424 alone, Nutlin-3 alone and the two in combination. In this figure, the upper shows the WB-detected protein bands, and the lower shows the bar charts of ratios of the corresponding proteins to the internal reference protein β-actin.
Figure 25 shows the *in vitro* proliferation inhibition rate curves of AST-3424 on H460, H460 P53 KO #1, H460 P53 KO #7 and H460 P53 KO #12 cells.
Figure 26 shows the *in vitro* proliferation inhibition rate curves of AST on H460, H460 P53 KO #1, H460 P53 KO #7 and H460 P53 KO #12 cells.
Figure 27 shows the *in vitro* proliferation inhibition rate curves of compound A on H460, H460 P53 KO #1, H460 P53 KO #7 and H460 P53 KO #12 cells.
Figure 28 shows the *in vitro* proliferation inhibition rate curves of compound B on H460, H460 P53 KO #1, H460 P53 KO #7 and H460 P53 KO #12 cells.
Figure 29 shows the WB-detected protein bands showing the effects of ±Nutlin-3 on Total P53, MDM2, P21, AKR1C3 and Actin proteins in H460 and H460 P53 KO cells.
Figure 30 shows the ratios of the corresponding protein in the WB-detected proteins to the internal reference protein β-actin showing the effects of ±Nutlin-3 on Total P53, MDM2, P21 and AKR1C3 proteins in H460 and H460 P53 KO cells,.
Figure 31 shows the *in vitro* proliferation inhibition rate curves of compound C and AST-3424 on NCI-H460 cells under normoxic condition.
Figure 32 shows the *in vitro* proliferation inhibition rate curves of compound C and AST-3424 on NCI-H460 P53KO #1 cells under normoxic condition.

### DETAILED DESCRIPTION OF THE INVENTION

The present invention will be described below with the reference to specific examples. Those skilled in the art will understand that these examples are only used to illustrate the present invention and do not limit the scope of the present invention in any way.

"Administering" or "administration of" a medicament to patients (and the grammatical equivalents of this phrase) refers to direct administration, which means a medicament may be administered to patients by a medical professional or self-administration, and/or indirect administration, which may be the act of prescribing a medicament. For example, a physician who instructs patients to self-administer a medicament and/or provides patients with a prescription for a medicament is administering the medicament to the patient.

"Cancer" refers to leukemia, lymphoma, carcinoma, and other malignant tumors (including solid tumors) with potentially unrestrained growth that can expand locally by invasion and systemically by metastasis. Examples of cancer include (but are not limited to) cancers of the adrenal gland, bone, brain, breast, bronchus, colon and/or rectum, gallbladder, head and neck, kidney, larynx, liver, lung, nervous tissue, pancreas, prostate, accessory thyroid gland, skin, stomach, and thyroid gland. Certain other examples of cancer include acute and chronic lymphocytic and granulocytic neoplasms, adenocarcinoma, adenoma, basal cell carcinoma, uterine cervical epithelial dysplasia and carcinoma in situ, Ewing's sarcoma, epidermoid carcinoma, giant cell tumor, glioblastoma multiforme, pilocytic tumor, intestinal ganglioneuroma, hyperplastic corneal nerve tumor, islet cell carcinoma, Kaposi's sarcoma, leiomyoma, leukemia, lymphoma, malignant carcinoid, malignant melanoma, malignant hypercalcemia, marfanoid habitus tumor, medullary epithelial carcinoma, metastatic skin cancer, mucosal neuroma, myeloma, mycosis fungoides, neuroblastoma, osteosarcoma, osteogenic and other sarcomas, ovarian tumor, pheochromocytoma, polycythemia vera, primary brain tumor, small cell lung cancer, ulcerative and papillary squamous cell carcinoma, hyperplasia, seminoma, soft tissue sarcoma, retinoblastoma, rhabdomyosarcoma, renal cell tumor, local skin lesion, reticulum cell sarcoma, and Wilm's tumor.

"Patient" and "individual" may be used interchangeably and refer to a mammal in need of treatment for cancer. Typically, the patient is a human. Typically, the patient is a human diagnosed with cancer. In certain embodiments, a "patient" or "individual" may refer to a non-human mammal, such as a non-human primate, a dog, a cat, a rabbit, a pig, a mouse, or a rat used for screening, characterizing, and evaluating drugs and therapies. "Solid tumors" refer to solid tumors including, but not limited to, metastatic tumors in the bone, brain, liver, lung, lymph node, pancreas, prostate, skin, and soft tissue (sarcoma).

"Therapeutically effective amount" of a drug refers to an amount of a drug that, when administered to patients with cancer, will have the intended therapeutic effect (e.g., alleviation, amelioration, palliation, or elimination of one or more clinical manifestations of cancer in the patient). A therapeutic effect does not necessarily occur by administration of one dose, and may occur only after administration of a series of doses. Thus, a therapeutically effective amount may be administered in one or more administrations.

"Treating", "treatment of" or "therapy of" conditions or patients refers to taking steps to obtain beneficial or desired results (including clinical results). For purposes of the present invention, beneficial or desired clinical results include (but are not limited to) alleviation or amelioration of one or more symptoms of cancer; diminishment of extent of disease; delay or slowing of disease progression; amelioration, remission, or stabilization of disease status; or other beneficial results. In some cases, treatment of cancer may result in partial response or stable disease.

"Tumor cells" or "cancer cells" refers to tumor cells or cancer cells of any appropriate species (e.g., a mammal, such as a murine, a canine, a feline, an equine, or a human).

"Patient" and "individual" may be used interchangeably and refer to a mammal in need of treatment for cancer. Typically, the patient is a human. Typically, the patient is a human diagnosed with cancer. In certain embodiments, a "patient" or "individual" may refer to a non-human mammal, such as a non-human primate, a dog, a cat, a rabbit, a pig, a mouse, or a rat used for screening, characterizing, and evaluating drugs and therapies. "Treatment" or "treatment of patients" is to give, administer or use a therapeutically effective amount of a medicament in relation to the present invention to patients.

"Administering", "administration of" or "use of" a medicament to patients refers to giving directly or direct administration, which means a medicament may be used or administered to patients by a medical professional or self-use or self-administration, and/or indirect use or administration, which may be the act of prescribing a medicament. For example, a physician who instructs patients to self-use or self-administer a medicament and/or provides patients with a prescription for a medicament is giving or administering the medicament to the patient. "Treating", "treatment of" or "therapy of" conditions or patients refers to taking steps to obtain beneficial or desired results (including clinical results). For purposes of the present invention, beneficial or desired clinical results include (but are not limited to) alleviation or amelioration of one or more symptoms of cancer; diminishment of extent of disease; delay or slowing of disease progression; amelioration, remission, or stabilization of disease status; or other beneficial results. In some cases, treatment of cancer may result in partial response or stable disease.

The experimental methods in the following examples are conventional methods unless specified otherwise. The medicinal raw materials, reagent materials and the like used are all commercially available products unless specified otherwise.

The above description of specific embodiments of the present invention does not limit the present invention. Those skilled in the art can make various modifications and changes according to the present invention, and any modification and change within the spirit of the present invention shall be covered in the scope of the claims appended to the present application.

### Example 1 Phase II Clinical Trial of AST-3424 Conducted in China

The clinical trial registration number is CTR20191399.

This trial was approved by the ethics committee of the medical institution participating in the clinical trial and was conducted in accordance with the principles of the Declaration of Helsinki. The informed consent has been obtained from all subjects.

### Enrollment Criteria

1. Male or female, aged ≥18 years old.
2. Histopathologically confirmed advanced HCC that cannot be controlled by surgical resection or local therapy.
3. History of prior standard systemic therapy including, but not limited to, Sorafenib and/or Oxaliplatin-based systemic chemotherapy, Lenvatinib, Regorafenib and/or Nivolumab, who have experienced disease progression, toxicity intolerance, or refusal to continue treatment with these drugs.
4. At least one measurable lesion meeting RECIST 1.1 criteria. A lesion with a history of prior radiation therapy shall not be regarded as the measurable lesion unless a definite radiological progression is observed after radiation therapy.
5. Capable of providing pathological paraffin blocks or sections (including archived pathological paraffin blocks and sections) for AKR1C3 expression analysis, and with confirmation of strongly positive AKR1C3 expression in liver tumor tissues (the central laboratory immunohistochemistry result shows that the proportion of tumor cells with AKR1C3 staining intensity of 2+ and/or 3+ is ≥ 70%).
6. Eastern Cooperative Oncology Group (ECOG) performance status score of 0 or 1.
7. Expected survival time ≥ 12 weeks.
8. With or without HBV or HCV infection. a. For subjects with HBV infection, HBV-DNA level shall be <2,000 IU/ml, and antiviral therapy with entecavir, tenofovir disoproxil fumarate, or tenofovir alafenamide fumarate shall be administered according to the national guidelines for the prevention and treatment of chronic hepatitis B. The therapy shall be maintained during the study and continued for 6 months after the last dose of the study drug. b. For subjects with HCV infection (in the presence of detectable HCV-RNA or anti-HCV antibodies), treatment may be conducted according to medical practice.
9. Child-Pugh score ≤ 6 points.
10. No history of hepatic encephalopathy.
11. Prior to the initiation of the study drug, all toxicities from previous anticancer therapies (except alopecia, fatigue, or peripheral neuropathy) shall have returned to Grade 1 or baseline levels (NCI CTCAE Version 5).
12. Subject's laboratory tests shall meet the following criteria. During 14 days before screening laboratory tests, no correction of indicators by blood transfusion, hematopoietic growth factor infusion or albumin infusion is allowed to meet the inclusion criteria: a. Hemoglobin ≥ 90 g/L; b. Platelet count ≥ 80 × 10⁹/L; c. Absolute neutrophil count (ANC) ≥ 1.5 × 10⁹/L; d. Serum total bilirubin ≤ 3 mg/dL; E. ALT and AST ≤ 5.0 × ULN; f. International normalized ratio (INR) ≤ 2.3 or prothrombin time prolongation ≤ 6 seconds; g. Albumin ≥ 29 g/L; h. Creatinine clearance > 50 mL/min as measured according to the Cockcroft-Gault equation.
13. No history of alcohol, drug or substance abuse within the past year.
14. Fertile female subjects shall be in non-lactating and have a negative pregnancy test result within 5 days prior to the initiation of treatment (the subject with a positive urine pregnancy test result needs to be reconfirmed by a serum pregnancy test).
15. Fertile female and male subjects shall agree to use effective contraceptive measures (e.g. surgical sterilization or condom or diaphragm contraception combined with spermicidal gel or intrauterine contraceptive device [IUD], etc.) with their partners from the start of study participation until 6 months after the last dose of the study drug.
16. Voluntary participation in this study, fully understanding of the relevant risks, good compliance, and signing the informed consent. Subjects may also sign an informed consent form for future biomedical research (FBR). However, the subjects who would not participate in FBR are still allowed participate in the main trial.

### Exclusion criteria:

1. Untreated active central nervous system (CNS) metastases or leptomeningeal disease. Subjects with CNS metastases that have been adequately treated and confirmed to be stable for at least 4 weeks during screening by clinical tests and brain imaging (MRI or CT) are allowed to participate in the study.
2. History of other malignant tumors within 2 years, except for adequately treated basal cell carcinoma, carcinoma in situ at other sites or natural medical history and other tumors whose treatment will not interfere with the safety or efficacy assessment of the current study.
3. Major surgery (other than diagnostic surgery) within 4 weeks prior to the first dose of the study drug.
4. Radiation therapy, surgical treatment, chemotherapy, immunotherapy, biological therapy for cancer, targeted therapy, or hormonal therapy within 4 weeks prior to the first dose of the study drug (a 6-week washout period for nitrosourea or Mitomycin C therapy; a 2-week washout period for oral fluorouracil drugs therapy; a 2-week washout period for small-molecule targeted therapy).
5. Participation in study for (diagnostic or therapeutic) drugs or medical devices within 4 weeks prior to the first dose of the study drug.
6. Needs to concomitant use of strong CYP3A4 inhibitors or inducers during the study.
7. Uncontrolled active bacterial, viral, or fungal infections requiring systemic therapy.
8. Known infection with human immunodeficiency virus (HIV) or syphilis-positive status.
9. Clinically significant ascites, defined as ascites identified by physical examination and needs to be controlled by abdominal paracentesis or additional pharmacological intervention for symptom maintenance (subjects with ascites detectable only by imaging test may be enrolled).
10. Female subjects who are pregnant, breastfeeding or planning to become pregnant.
11. Concomitant diseases or symptoms that may interfere with the conduct of the study, or physical abnormalities that, in the opinion of the investigator, pose an excessive risk to the subject, including, but not limited to, within three months a history of gastrointestinal bleeding or a higher risk of bleeding, active peptic ulcer or gastritis, changes in mental status, or psychiatric disorders that may interfere with the subject's understanding of the informed consent form.
12. A history of allergy to ethanol or propylene glycol.
13. Subjects who are unwilling or unable to comply with the study protocol for any reason.

### Study drug:

Concentrated injection solution of AST-3424: manufactured by a pharmaceutical enterprise entrusted by ASCENTAWITS PHARMACEUTICALS, LTD.. Specification: 1 mL: 10 mg; containing 0.75 mL ethanol and 0.25 mL propylene glycol.

Dosing Regimen:
in each 21-day cycle, administration is performed on day 1 and day 8; the dose is 6 mg/m² per administration; and the subject will be permitted to receive treatment for a maximum of 34 cycles.

### Specific operation of drug administration:

Before administration, 0.1 ml of 5% sodium bicarbonate solution for injection was added to 100 ml of commercially available sterile 5% dextrose aqueous solution for injection (D5W) in an intravenous infusion bag without DEHP (di(2-ethylhexyl) phthalate). Add the calculated volume (accurate to 0.01 mL) of the concentrated injection solution of AST-3424 to the pH-adjusted D5W bag to prepare the AST-3424 injection for intravenous infusion.

The solutes of the intravenous aqueous injection consist of the active pharmaceutical ingredient AST-3424, glucose, ethanol, propylene glycol and sodium bicarbonate as pH regulator, wherein the solution has an active pharmaceutical ingredient AST-3424 concentration of 0.004-0.94 mg/ml, a pH of 7.4, a glucose content of 4.5-5.0% by mass, and the solution is an isotonic solution.

If the subject is intolerant to glucose injection, normal saline may be used instead:
Before administration, 0.1 ml of 5% sodium bicarbonate solution for injection was added to 100 ml of commercially available sterile 0.9% normal saline for injection in an intravenous infusion bag without DEHP (di(2-ethylhexyl) phthalate). Add the calculated volume (accurate to 0.01 mL) of the concentrated injection solution of AST-3424 to the pH-adjusted normal saline bag to prepare the AST-3424 injection for intravenous infusion.

The solutes of the intravenous aqueous injection consist of the active pharmaceutical ingredient AST-3424, sodium chloride, ethanol, propylene glycol and sodium bicarbonate as pH regulator, wherein the solution has an active pharmaceutical ingredient AST-3424 concentration of 0.004-0.94 mg/ml, a pH of 7.4, a sodium chloride content of 0.81-0.90% by mass, and the solution is an isotonic solution.

The exact calculation method for the required volume of concentrated injection solution of AST-3424 is as follows:
For a subject with a height of 175cm and a weight of 75kg, the corresponding equivalent body surface area BSA (m²) = ([Height (cm) × Weight (kg)]/3600)^{1/2}= 1.90, then the corresponding dose is 1.90 × 6.0 = 11.40 mg, then the concentrated injection solution of AST-3424 of the above specifications to be drawn as 11.40 ÷ 10 × 1 = 1.14 ml.

The prepared intravenous AST-3424 injection should be injected within 8 hours.

### Clinical evaluation

Efficacy evaluation includes clinical efficacy assessment.

The clinical efficacy evaluation criteria are RECIST 1.1, the efficacy evaluation criteria for solid tumors. Lesions are evaluated by MRI/CT, and the same evaluation method should be used for the same lesion throughout the study. Subjects shall have measurable tumor lesions at the baseline.

Efficacy evaluation indicators include complete response (CR), partial response (PR), stable disease (SD) and progressive disease (PD).

Complete response (CR): All target lesions disappear, and all pathological lymph nodes (including target and non-target nodes) must be reduced to < 10mm in short diameter.

Partial response (PR): The sum of the diameters of the target lesions is reduced by at least 30% compared to the baseline level.

Progressive disease (PD): The sum of the diameters of the target lesions is increased by at least 20% compared to the minimum of the sum of all measured target lesion diameters over the course of the experimental study (baseline value if the baseline measurement is the smallest). In addition, an absolute increase of at least 5 mm in the sum of the diameters shall be achieved (the appearance of one or more new lesions is also considered as disease progression).

Stable disease (SD): The degree of reduction in target lesions does not meet the criteria for PR, and the degree of increase does not meet the criteria for PD, falling between the two. The minimum value of the sum of diameters can be used as a reference in the study.

### Study Endpoint

The efficacy of AST-3424 monotherapy for HCC and other malignant tumors is preliminarily evaluated based on the objective response rate (ORR), disease control rate (DCR), duration of response (DOR), and progression-free survival (PFS) of the subjects.

Objective response rate (ORR): The percentage of subjects who achieve complete response (CR) and partial response (PR) after treatment among all evaluable subjects.

Disease Control Rate (DCR) refers to the percentage of subjects with confirmed complete response (CR), partial response (PR), and stable disease (SD) among all evaluable subjects.

### Trial Results

A total of 17 subjects with liver cancer were enrolled, among whom 8 completed the clinical efficacy evaluation. Of these 8 subjects: 1 achieved PR, accounting for 12.5%; 5 achieved SD, accounting for 62.5%; and 2 achieved PD, accounting for 25%. The overall DCR was 75% and the ORR was 12.5%. In particular, 8 of them were tested for p53 gene mutations or defects, wherein negative (-) indicates no mutation/defect detected and positive (+) indicates mutation/defect detected (which may or may not affect protein expression). The detailed results are shown in Table 1 below.

**Table 1: AST-3424 Phase II clinical partial case data as of July 21, 2023**

| Case screening number | Positive/negative for p53 gene mutation or defect test | Efficacy evaluation |
|---|---|---|
| 03003 | Negative (-) | PR |
| 04001 | Negative (-) | SD, lesion reduction |
| 05003 | Negative (-) | SD, lesion reduction |
| 04003 | Positive (+), unclear in whether it affects protein expression | SD, lesion increase |
| 06003 | Positive (+), which may affect protein expression | SD, lesion increase |
| 06004 | Positive (+), which may affect protein expression | SD, lesion increase |
| 03006 | Positive (+), which may affect protein expression | PD |
| 05001 | Negative (-) | PD |

Further analysis of the relationship between efficacy and negative/positive p53 gene mutation:
Among the 4 negative (-) cases, 1 achieved PR, 2 achieved SD with reduction in lesion size, and 1 achieved PD. Namely, in this subgroup, the DCR was 75% and the ORR was 25%.

Among the 4 positive (+) cases, 3 achieved SD with increase in lesion size, and 1 achieved PD. Namely, in this subgroup, the DCR was 75% (all the 3 cases presented lesion enlargement); and the ORR of this subgroup was 0%.

On the whole, after administration of AST-3424 at the same dose level, there were notable differences in the therapeutic efficacy evaluation between the negative/positive p53 gene mutation subgroups: the ORR was 25% in the subgroup of liver cancer subjects with negative (-) p53 gene mutation or defect, while the ORR of the corresponding positive (+) subgroup was 0%. Furthermore, although both subgroups had a DCR of 75% (3 out of 4 cases), practically 2 out of the 3 subjects evaluated as SD, in the subgroup of liver cancer subjects with negative (-) p53 gene mutation or defect, exhibited reduction in lesion size (with a high probability of converting to PR or even CR upon subsequent continuous administration). In contrast, all the 3 subjects evaluated as SD, in the subgroup of liver cancer subjects with positive (+) p53 gene mutation or defect, presented enlargement in lesion size.

Therefore, based on the clinical results at the current stage, it is reasonable for a person skilled in the art to hold that AST-3424 exerts a significantly superior therapeutic effect on cancer or tumor patients with negative (-) p53 gene mutation or defect compared with those with positive (+) p53 gene mutation or defect. Accordingly, the applicant infers that AST-3424 will yield a better therapeutic effect in the treatment of tumor/cancer patients with a negative (-) test result for p53 gene mutation or defect, i.e., such patients will obtain more significant clinical benefits from the treatment with AST-3424.

As of September 1, 2023, the progress of the above-mentioned ongoing clinical trials is as follows.

### Trial Results

A total of 20 subjects with liver cancer were enrolled, among whom 18 completed the clinical efficacy evaluation. Of these 18 subjects: 1 achieved PR; 10 achieved SD; and 7 achieved PD. Finally, the ORR was 5.6% (1/18) and the DCR was 61.1% (11/18).

In particular, 10 of them were tested for p53 gene mutations or defects, wherein negative (-) indicates no mutation/defect detected and positive (+) indicates mutation/defect detected (which may or may not affect protein expression). The detailed results are shown in Table 6 below.

**Table 6: AST-3424 Phase II clinical partial case data as of September 01, 2023**

| Case screening number | Percentage of 2 + & 3 + in AKR1C3 IHC assay | Positive/negative for p53 gene mutation or defect test | Efficacy evaluation | PFS |
|---|---|---|---|---|
| 03003 | 90% | Negative (-) | PR | >6.6 |
| 04001 | 95% | Negative (-) | SD, lesion reduction | 7.8 |
| 05003 | 90% | Negative (-) | SD, lesion reduction | >2.8 |
| 04003 | 85% | Positive (+), unclear in whether it affects protein expression | SD, lesion increase | 2.9 |
| 06003 | 95% | Positive (+), which may affect protein expression | SD, lesion increase | 1.4 |
| 06004 | 80% | Positive (+), which may affect protein expression | SD, lesion increase | 1.4 |
| 03006 | 75% | Positive (+), which may affect protein expression | PD | 1.4 |
| 05001 | 100% | Negative (-) | PD | 1.7 |
| 03009 | 100% | Negative (-) | SD, lesion increase | >1.6 |
| 02005 | 95% | Positive (+), which may affect protein expression | PD | 1.38 |

PFS, cut-off date: 2023.09.01. The PFS is an estimated value, calculated at 30.437 days per month.

Further analysis of the relationship between efficacy and negative/positive p53 gene mutation:
Among the 5 negative (-) cases, 1 achieved PR, 3 achieved SD, and 1 achieved PD. Namely, in this subgroup, the DCR was 80% (4/5), the ORR was 20% (115). The mean PFS was calculated as 4.1 months.

Among the 5 positive (+) cases, 3 achieved SD with increase in lesion size, and 2 achieved PD. Namely, in this subgroup, the DCR was 60% (all the 3 cases presented lesion enlargement); and the ORR was 0%. The mean PFS was calculated as 1.756 months.

On the whole, after administration of AST-3424 at the same dose level, there were notable differences in the therapeutic efficacy evaluation between the negative/positive p53 gene mutation subgroups: in the subgroup of liver cancer subjects with negative (-) p53 gene mutation or defect, the ORR was 20%, the DCR was 80%, and the PFS was 4.1 months; the corresponding data of the positive (+) subgroup were 0, 60%, and 1.756 months. The subgroup of patients with negative (-) p53 gene mutation had a better treatment effect.

As of March 11, 2024, the progress of the above-mentioned ongoing clinical trials is as follows.

### Trial Results

As of March 11, 2024, a total of 30 subjects have been enrolled: 5 are still receiving treatment in the trial, and 25 have discontinued trial participation (1 withdrew informed consent, 10 died, and 14 are under survival follow-up). The current longest PFS was >11.5 months, and the longest OS was >17.6 months. A total of 11 subjects were enrolled by the end of January 2023, among whom 6 had an OS of more than 12 months. The details are shown in Table 7 below.

**Table 7: Clinical data on the correlation between therapeutic efficacy of AST-3424 on hepatocellular carcinoma and gene mutation status as of March 11, 2024**

| Cases | AKR1C3 | p53 | Efficacy evaluation | PFS | OS |
|---|---|---|---|---|---|
| 03003 | 90% | Negative (-) | PR | >11.5 | >13.8 |
| 04001 | 95% | Negative (-) | SD, lesion reduction | 7.8 | 12.3 |
| 05003 | 90% | Negative (-) | SD, lesion reduction | 2.8 | >9.6 |
| 04003 | 85% | Positive (+), unclear in whether it affects protein expression | SD, lesion increase | 2.9 | >17.6 |
| 06003 | 95% | Positive (+), which may affect protein expression | SD, lesion increase | 1.4 | 11.4 |
| 06004 | 80% | Positive (+), which may affect protein expression | SD, lesion increase | 1.4 | >14.0 |
| 03006 | 75% | Positive (+), which may affect protein expression | PD | 1.4 | >11.3 |
| 05001 | 100% | Negative (-) | PD | 1.7 | 10.8 |
| 03009 | 100% | Negative (-) | SD, lesion increase | 3.2 | 6.3 |
| 02005 | 95% | Positive (+), which may affect protein expression | PD | 1.4 | >7.3 |
| 03007 | 100% | Positive (+), which may affect protein expression | PD | 1.6 | 2.2 |
| 03008 | 95% | Positive (+), which may affect protein expression | PD | 1.5 | >8.7 |
| 05004 | 80% | Positive (+), which may affect protein expression | PD | 1.2 | >7.1 |
| 06009 | 85% | Negative (-) | SD, lesion increase | 2.7 | 2.9 |
| 04011 | 85% | Negative (-) | PD | 1.5 | >5.7 |
| 06013 | 75% | Positive (+), which may affect protein expression | PD | 1.6 | >2.3 |
| 04013 | 100% | Positive (+), unclear in whether it affects protein expression | SD, lesion increase | 1.0 | >1.5 |
| 03010 | 90% | Positive (+), unclear in whether it affects protein expression | SD, lesion increase | 1.6 | >1.8 |
| 06014 | 100% | Negative (-) | SD, lesion increase | 1.6 | >2.0 |
| 06010 | 85% | Negative (-) | PR | 4.7 | >5.7 |
| 04008 | 100% | Not detected | SD, lesion increase | 3.2 | >5.9 |
| 06007 | 100% | Not detected | Not evaluated | 2.4 | 2.4 |
| 03004 | 100% | Not detected | SD, lesion reduction | 1.4 | >2.1 |
| 02004 | 100% | Not detected | Not evaluated | 3.3 | 3.3 |
| 04005 | 95% | Not detected | SD, lesion increase | 3.2 | 10.6 |
| 07006 | 100% | Not detected | SD, lesion increase | 2.7 | >17.0 |
| 07005 | 95% | Not detected | SD, lesion increase | 2.7 | >17.6 |
| 07001 | 80% | Not detected | PD | 1.4 | 5.7 |

Among the 26 enrolled subjects with efficacy clinical evaluation results, 20 had p53 gene mutation test results. The efficacy was observed according to the p53 test results in groups:
Wild-type (WT) with no mutation, i.e., the p53 gene mutation test result is negative (-);
Variant of uncertain significance (VUS): i.e., the p53 gene mutation test result is positive (+), and it is not clear whether it affects protein expression;
Mutations that may affect protein function (MUT), i.e., the p53 gene mutation test result is positive (+), which may affect protein expression. The statistical analyses were conducted based on the above three groups. The results are shown in Tables 8, 9 and 10 below.

**Table 8: Efficacy data of 8 P53 mutation-positive (MUT) subjects**

| Cases | AKR1C3 | p53 | Efficacy evaluation | PFS | OS |
|---|---|---|---|---|---|
| 06003 | 95% | Positive (+), which may affect protein expression | SD, lesion increase | 1.4 | 11.4 |
| 06004 | 80% | Positive (+), which may affect protein expression | SD, lesion increase | 1.4 | >14.0 |
| 03006 | 75% | Positive (+), which may affect protein expression | PD | 1.4 | >11.3 |
| 02005 | 95% | Positive (+), which may affect protein expression | PD | 1.4 | >7.3 |
| 03007 | 100% | Positive (+), which may affect protein expression | PD | 1.6 | 2.2 |
| 03008 | 95% | Positive (+), which may affect protein expression | PD | 1.5 | >8.7 |
| 05004 | 80% | Positive (+), which may affect protein expression | PD | 1.2 | >7.1 |
| 06013 | 75% | Positive (+), which may affect protein expression | PD | 1.6 | >2.3 |

The statistical results indicated that among the 8 cases, 2 have died and 6 were under survival follow-up. The current longest PFS was 1.6 months, the longest OS was >14.0 months, the mean PFS was 1.4 months, and the mean OS was more than 9.3 months. Among the 8 cases with positive (+) p53 gene mutation which may affect protein expression, 0 achieved PR, 2 achieved SD, and 7 achieved PD. Namely, in this subgroup, the DCR was 25% (2/8) and the ORR was 0% (0/5).

**Table 9: Efficacy data of 9 P53 mutation negative (WT) subjects**

| Cases | AKR1C3 | p53 | Efficacy evaluation | PFS | OS |
|---|---|---|---|---|---|
| 03003 | 90% | Negative (-) | PR | >11.5 | >13.8 |
| 04001 | 95% | Negative (-) | SD, lesion reduction | 7.8 | 12.3 |
| 05003 | 90% | Negative (-) | SD, lesion reduction | 2.8 | >9.6 |
| 05001 | 100% | Negative (-) | PD | 1.7 | 10.8 |
| 03009 | 100% | Negative (-) | SD, lesion increase | 3.2 | 6.3 |
| 06009 | 85% | Negative (-) | SD, lesion increase | 2.7 | 2.9 |
| 04011 | 85% | Negative (-) | PD | 1.5 | >5.7 |
| 06014 | 100% | Negative (-) | SD, lesion increase | 1.6 | >2.0 |
| 06010 | 85% | Negative (-) | PR | 4.7 | >5.7 |

The statistical results indicated that among the 9 cases, 4 have died and 5 were under survival follow-up. The current longest PFS was more than 11.5 months, the longest OS was >13.8 months, the mean PFS was more than 4.1 months, and the mean OS was more than 7.7 months. Among the 9 cases with negative (-) p53 gene mutation, 2 achieved PR, 5 achieved SD, and 2 achieved PD. Namely, in this subgroup, the DCR was 77.8% (7/9) and the ORR was 22.2% (2/9).

**Table 10: Efficacy data of 3 subjects with positive P53 mutation, unclear in whether it affects protein expression (VUS)**

| Cases | AKR1C3 | p53 | Efficacy evaluation | PFS | OS |
|---|---|---|---|---|---|
| 04003 | 85% | Positive (+), unclear in whether it affects protein expression | SD, lesion increase | 2.9 | >17.6 |
| 04013 | 100% | Positive (+), unclear in whether it affects protein expression | SD, lesion increase | 1 | >1.5 |
| 03010 | 90% | Positive (+), unclear in whether it affects protein expression | SD, lesion increase | 1.6 | >1.8 |

Therefore, based on the clinical results at the current stage, it is reasonable for a person skilled in the art to hold that AST-3424 exerts a significantly superior therapeutic effect on cancer or tumor patients with negative (-) p53 gene mutation or defect compared with those with positive (+) p53 gene mutation or defect. Accordingly, the applicant infers that AST-3424 will yield a better therapeutic effect in the treatment of tumor/cancer patients with a negative (-) test result for p53 gene mutation or defect, i.e., such patients will obtain more significant clinical benefits from the treatment with AST-3424.

The interim results of the two clinical trials in example 1 seem to confirm that DNA alkylating agent prodrugs such as AST-3424 have a better therapeutic effect in patients with negative p53 gene mutation, i.e., with normal expression of p53 protein. In order to further confirm the above fact, the applicant conducted further experimental demonstration.

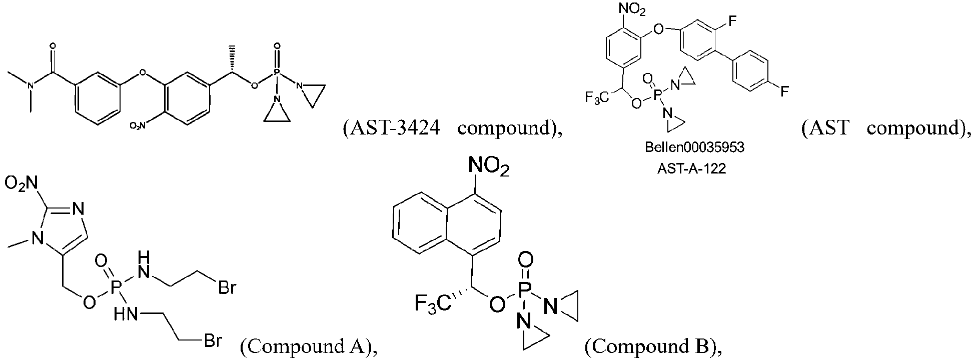

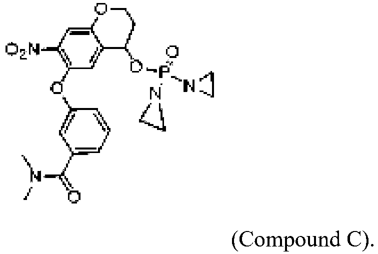

Unless otherwise specified, the aforementioned compounds were synthesized and prepared by the applicant with reference to the corresponding patent applications.

Further review of relevant literature reveals the following:
The main function of the p53 protein is to monitor cell DNA damage. It can induce cells to enter cycle arrest, promote DNA damage repair, and facilitate tumor cell apoptosis, ultimately inhibiting tumor growth. (Marei, H.E., Althani, A., Afifi, N. et al. p53 signaling in cancer progression and therapy. Cancer Cell Int 21, 703 (2021). https://doi.org/10.1186/s12935-021-02396-8) _{∘}

The p53 mutation/defect will lead to a series of reactions and manifestations:
The p53 mutation/defect can impair the apoptotic pathway, thereby resulting in subsequent drug resistance (Sturm I, Bosanquet A G, Hermann S, et al. Mutation of p53 and consecutive selective drug resistance in B-CLL occurs as a consequence of prior DNA-damaging chemotherapy[J].Cell Death & Differentiation, 2003, 10(4):477.DOI:10.1038/sj.cdd.4401194.).

The p53 mutation/defect would impair cell cycle arrest, allowing damaged cells to continue dividing and surviving, thereby resulting in drug resistance (Zhao, D., Tahaney, W.M., Mazumdar, A. et al. Molecularly targeted therapies for p53-mutant cancers. Cell. Mol. Life Sci.2017 , 74, 4171-4187. https://doi.org/10.1007/s00018-017-2575-0).

The p53 mutation/defect causes improper activation of DNA repair pathways, leading to the accumulation of DNA damage in cancer cells and subsequent drug resistance. (Williams AB, Schumacher B. p53 in the DNA-Damage-Repair Process. Cold Spring Harb Perspect Med. 2016,6(5):a026070. doi:10.1101/cshperspect.a026070.

The p53 mutation/defect accelerates and enhances the generation efficiency of cancer stem cells (CSC) with drug-resistant properties, thereby resulting in drug resistance. The p53 gain-of-function mutations can also promote CSC generation and subsequent chemoresistance (Ozaki T, Nakamura M, Shimozato O. Novel Implications of DNA Damage Response in Drug Resistance of Malignant Cancers Obtained from the Functional Interaction between p53 Family and RUNX2. Biomolecules. 2015,5(4):2854-2876. doi:10.3390/biom5042854). The p53 gain-of-function mutations can promote tumor progression, further leading to drug resistant and treatment-refractory state (Alvarado-Ortiz E, de la Cruz-López KG, Becerril-Rico J, Sarabia-Sánchez MA, Ortiz-Sánchez E, Garcia-Carrancá A. Mutant p53 Gain-of-Function: Role in Cancer Development, Progression, and Therapeutic Approaches. Front Cell Dev Biol. 2021,8:607670. Published 2021 Feb 11. doi:10.3389/fcell.2020.607670).

In other words, p53 plays an important role in the process of DNA damage repair and apoptosis. Once the p53 gene is mutated or defective, it would lead to abnormal p53 protein, which would not play the above role, making tumors or cancer cells resistant to drugs.

DNA alkylating agent prodrug compounds such as AST-3424 release DNA alkylating agents (e.g., AST-2660, Br-IPM and so on) to crosslink with DNA in vivo, which results in DNA damage and breakage, ultimately leading to cell death. The p53 mutation/defect would inhibits the ability of the p53 protein to promote tumor cell apoptosis after DNA damage. Based on this, it can be inferred that cells with negative TP53 gene mutation and normal p53 protein expression, after p53 protein activation or upregulation, can promote the apoptosis of tumor cells with DNA damage. A series of experimental phenomena associated with the above-mentioned enhanced toxicity of cell apoptosis and DNA damage can be observed.

To this end, the following experiments were conducted using p53 wild-type H460 cells, A549 cells, p53 mutant HPAF-II cells, and p53 knockout cells. Unless otherwise specified, the H460 cells in examples 2 to 13 below are NCI-H460 cells.

### Example 2: in vitro cell proliferation effect experiment and cell colony formation effect experiment

Nutlin-3 is a small-molecule MDM2-p53 inhibitor that indirectly activates p53 function by inhibiting the interaction between MDM2 and p53. Therefore, cell proliferation effect experiments and cell colony formation effect experiments were conducted with AST-3424/AST combined with Nutlin-3 which activates/upregulates p53 function.

The CAS number of Nutlin-3 is 548472-68-0.

H460 are p53 wild-type cells, i.e., negative for p53 gene mutation with normal expression of p53 protein. HPAF-II cells are p53 mutant cells (p.P151S, deleterious), i.e., positive for p53 gene mutation with abnormal expression of p53 protein.

Effects of compounds on H460 cell proliferation in vitro under normoxia

Overview of the experimental process
1) H460 cell suspension was added to a 96-well plate at 100 µL per well, with a cell density of 2000 /well.
2) The cells were cultured overnight in an incubator at 37°C, 5% CO₂.
3) Compound Treatment

Monotherapy: After 24 hours of cell plating, each well was supplied with 99.5 µL of growth medium then added with 0.5µL of test compounds at different concentrations, shaken gently to ensure even mixing, and then placed at 37°C, 5% CO₂ in an incubator.

Combination therapy: After 24 hours of cell plating, each well was supplied with 99 µL of growth medium then added with 0.5µL of the combined action compound Nutlin-3, shaken gently to ensure even mixing, and then placed in an incubator for 2 hours. Then each well was added with 0.5µL of test compounds at different concentrations, shaken gently to ensure even mixing, and then placed at 37°C, 5% CO₂ in an incubator.

4) The cell plate was placed in the incubator for 72 hours.

5) The cell plate to be tested was placed at room temperature to equilibrium for 30 minutes, and 100µL of medium was discarded per well.

6) 25µL of CTG reagent was added to each well, placed at a fast shaker to shake for 2 minutes, and placed at room temperature away from light for 30 minutes.

7) The chemiluminescence signal value was read using a multifunctional microplate reader with a reading time of 1000 ms.

8) IC₅₀ was calculated with Graph Pad Prism 5 software.

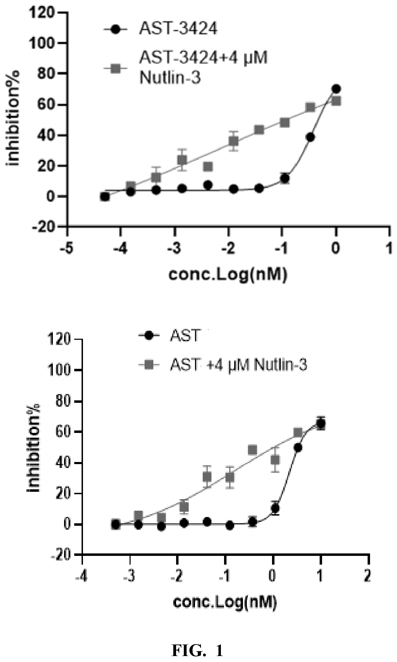

The experimental results are shown in figure 1.

The experiment results show that adding Nutlin-3 which indirectly activates p53 function in advance, followed by AST-3424, can allow the proliferation inhibition rate of AST-3424 to increase significantly.

Further combination therapy experiments with different administration sequences of AST-3424 were carried out. Dosing regimens were set for the experiments.

Monotherapy: Each well was added with AST-3424 alone, Nutlin-3 alone, 5 µL of each compound in different concentrations (400 times) and 5 µL of medium. The cells were treated (DMSO 0.25% and 0.50%).

Combination therapy: Cells were treated with 5 µL each of AST-3424 and Nutlin-3 in different administration sequences: AST-3424 pre-treatment for 2 hours followed by co-treatment with Nutlin-3 for 6 hours; Nutlin-3 pre-treatment for 2 hours followed by co-treatment with AST-3424 for 6 hours; and co-treatment with AST-3424 and Nutlin-3 for 6 hours.

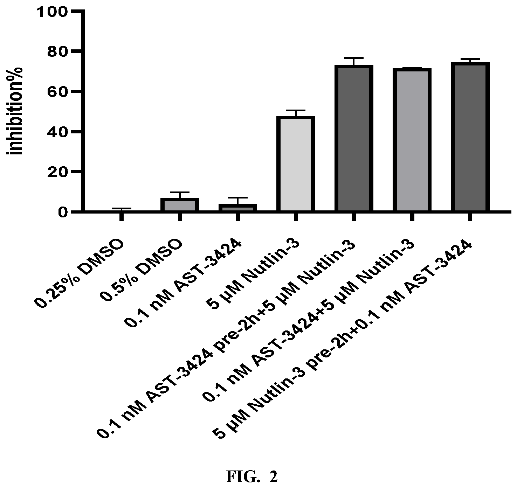

The experimental results are shown in Figure 2.

The results of the cell proliferation experiments (3 days) indicated that the proliferation inhibition rate of AST-3424 was significantly increased after the addition of Nutlin-3 which indirectly activates p53 function. This effect was independent of the administration sequence of Nutlin-3.

Experiment of effect of AST-3424 ± Nutlin-3 with different administration sequences on colony formation of H460/HPAF-II cells

### Overview of the experimental process:

H460: 1000cells/2ml/well/6-well plate
HPAF-II: 3000 cells/2 ml/well/6-well plate
   1) H460 cell suspension was added to a 6-well plate at 2 mL per well, with a cell density of 1000 /well. HPAF-II cell suspension was added to a 6-well plate at 2 mL per well, with a cell density of 3000 /well.
   2) The cells were inoculated with 1990 µL of medium and cultured overnight in an incubator at 37°C, 5% CO₂.
   3) Compound Treatment

Monotherapy: Each well was added with AST-3424 alone, Nutlin-3 alone, 5 µL of each compound in different concentrations (400 times) and 5 µL of medium. The cells were treated (DMSO 0.50%) for 6h.

Combination therapy: Cells were treated with 5 µL each of AST-3424 and Nutlin-3 in different administration sequences: AST-3424 pre-treatment for 2 hours followed by co-treatment with Nutlin-3 for 6 hours; Nutlin-3 pre-treatment for 2 hours followed by co-treatment with AST-3424 for 6 hours; and co-treatment with AST-3424 and Nutlin-3 for 6 hours.

4) After 6 hours of co-treatment, the cells were washed twice to remove the compounds. 5 mL of medium was added to each well.

5) The cells were cultured for 7 days (medium change every 3 days and observation end time could be adjusted according to the actual cell proliferation).

6) Staining was performed when the vast majority of cell communities reached about 50 cells at 0 nM (0.5% DMSO). The medium was discarded and the cells were stained with 0.5%[w/v] crystal violet for 40 min after the cells were fixed. The plates were washed twice with tap water and dried before counting.

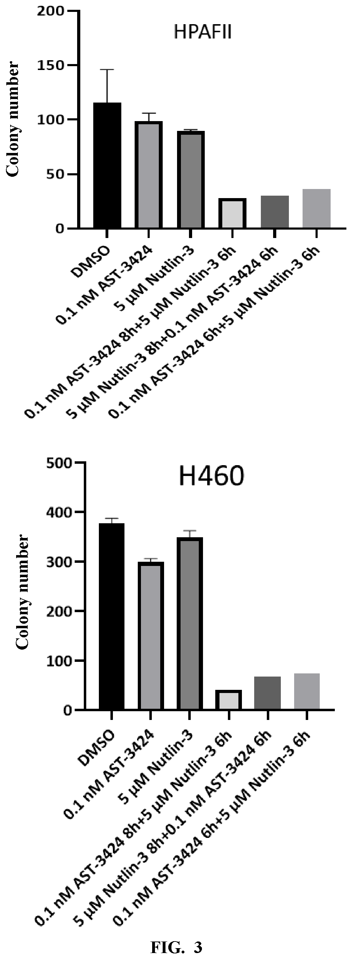

7) Cell colony counting was performed, and the number of colonies containing more than 50 cells was recorded. The experimental results are shown in Figure 3.

Compared with monotherapy, the combination of Nutlin-3 and AST-3424 significantly inhibited the colony formation of both H460 and HPAF-II cells, exhibiting an additive effect.

Among the three groups of H460 cells treated with the combination therapy, the group with AST-3424 added first showed fewer colonies than the other two groups.

Among the three groups of HPAF-II cells treated with the combination therapy, there was no significant difference between different administration sequences.

For H460 cells, the colony numbers in the three combination therapy groups with different administration sequences and the DMSO group were compared respectively. The percentages of colony numbers in the combination therapy groups relative to the DMSO group were calculated to reflect the inhibitory capacity of the combination therapy on cell colony formation. A lower percentage indicates a stronger inhibitory effect of the drug on cell colony formation. The percentages of colony numbers in the three combination therapy groups with different administration sequences relative to the DMSO group were 11.12%, 23.03% and 21.45%, respectively. Similarly, analysis of HPAF-II cell data showed that the percentages of colony numbers in the three combination therapy groups with different administration sequences relative to the DMSO group were 24.14%, 30.41% and 40.30%, respectively.

Based on these results, it can be inferred that the combination with Nutlin-3 exhibited a significantly superior colony inhibitory effect on p53 wild-type H460 cells compared to p53 mutant HPAF-II cells.

The above experimental results show that Nutlin-3 can indeed enhance the *in vitro* cytotoxicity of AST-3424/AST against cancer cells, and the enhancement of *in vitro* cytotoxicity is more significant in p53 wild-type H460 cells.

### Example 3: Effect of Nutlin-3 combined with AST-3424 on apoptosis and cell cycle G2/M arrest

Effects of AST-3424 alone and Nutlin-3 alone or the two in combination on the apoptosis process of H460 cells Overview of the experimental process:
1) H460 cell suspension was added to a 96-well white plate at 99 µL per well, with a cell density of 15000 /well. Meanwhile, 96-well transparent plates with the same cell density were plated for photographing and observation before detection.
2) The cells were cultured overnight in an incubator at 37°C, 5% CO₂.
3) Compound Treatment

Monotherapy: After 24 hours of cell plating, 0.5 µL of medium was supplied to each well. According to the protocol, the designated cell wells were supplied with 0.5 µL of the corresponding concentration of the compound, shaken gently to ensure even mixing, and then placed at 37°C, 5% CO₂ in an incubator.

Combination therapy: After 24 hours of cell plating, the designated cell wells were supplied with 0.5 µL of the corresponding concentration of the compound Nutlin-3, shaken gently to ensure even mixing, and then placed at 37°C, 5% CO₂ in an incubator for 2 hours. Then the designated cell wells were supplied with 0.5 µL of AST-3424.

4) The cell plate was placed in the incubator for 24 hours.

5) The cell plate to be tested was placed at room temperature to equilibrium for 5 minutes.

6) CaspaseGlo^{®}3/7 reagent was prepared and equilibrated to room temperature. The buffer and the substrate of the reagent were mixed homogeneously at 1:1 to prepare the CaspaseGlo^{®}3/7 reagent. The prepared reagent can be stored at 4°C for 3 days.

7) 100 µL of CaspaseGlo ^{®} 3/7 Reagent was added to each well of medium containing 100 µ L of blank (cell-free wells), negative control cells or treated cells. Due to the sensitivity of this assay, care was taken not to contact the pipette tip with the well containing the sample to avoid cross-contamination. The plate was covered with a plate sealer or lid.

8) Gently mix the reagent solution using a shaker at 300-500 rpm for 30 seconds. The plates were placed at room temperature away from light for 1 hour.

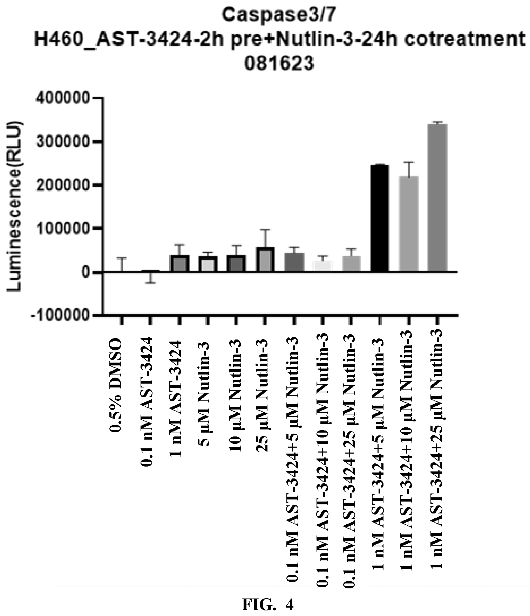

9) The luminescence of each sample was measured using a plate reading photometer. The luminescence values of each group were calculated and analyzed as bar graphs with Relative Luminescence Units (RLU). The results are shown in Figure 4.

Compared with each monotherapy, the combination of AST-3424 (1 nM) and Nutlin-3 in effective concentrations significantly increased the content of Caspase 3/7, a marker of cancer cell apoptosis, indicating that the combination enhances cell apoptosis. The marker content varied with different doses of Nutlin-3, demonstrating that the aforementioned increase in apoptosis is Nutlin-3 dose-dependent. Namely, as the concentration of Nutlin-3 increased, the content of the apoptosis marker detected after adding the combination of AST-3424 and Nutlin-3 also increased, further confirming the Nutlin-3 dose-dependence of the apoptosis enhancement.

In other words, the addition of Nutlin-3 which indirectly activates p53 protein function can enhance the apoptotic effect of AST-3424.

Effects of AST-3424 alone and Nutlin-3 alone or the two in combination on cell cycle G2/M arrest

The whole cell replication cycle can be described as G0/G1, S and G2/M phases. In tumor pathological study, the cell ratio in S phase is usually used as an indicator to judge the tumor proliferation status.

Overview of the experimental process:
1) H460/HPAF-II cell suspensions were added to a 24-well plate, with a cell density of 100000 /well and 995 µL of medium per well.
2) The cells were cultured overnight in an incubator at 37°C, 5% CO₂.
3) Compound Treatment

After 24 hours of cell plating, according to the protocol, each cell line experiment was grouped as: Monotherapy: After 24 hours of cell plating, each well was supplied with 5µL of test compounds at different concentrations: 1% DMSO, 5 µM Nutlin-3 and 0.1 nM AST-3424, shaken gently to ensure even mixing, and then placed at 37°C, 5% CO₂ in an incubator.

### Combination therapy:

To cells AST-3424 pre-treatment for 2 hours followed by co-treatment with Nutlin-3 for 24 hours.

To cells Nutlin-3 pre-treatment for 2 hours followed by co-treatment with AST -3424 for 24 hours.

Co-treatment with AST-3424 and Nutlin-3 for 24 hours.

4) The cells were digested, centrifuged at 1000 g at 4°C, washed once with pre-cooled PBS, centrifuged at 1000 g at 4°C, and fixed with 1 mL of pre-cooled 70% ethanol at -20°C overnight.

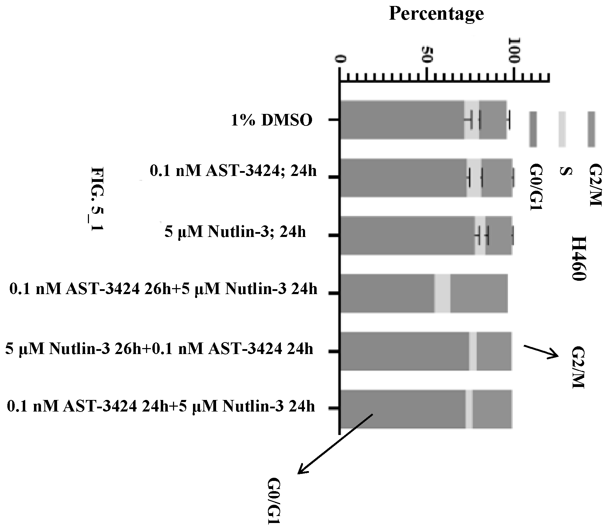

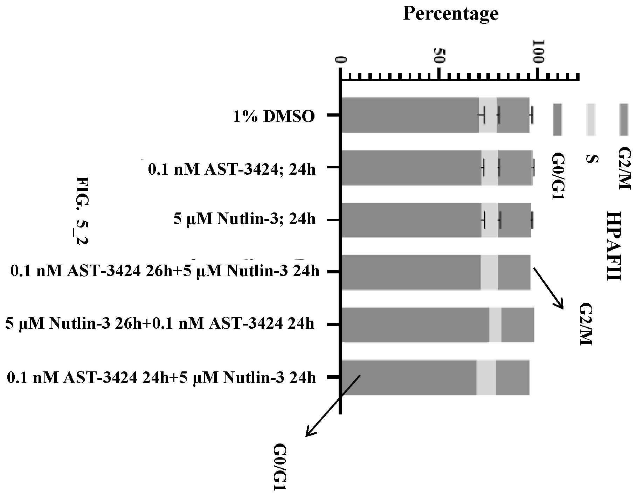

5) The cells were centrifuged at 3000g at 4°C, washed once with pre-cooled PBS (phosphate buffer), centrifuged at 3000g at 4°C. Prepare PI staining solution. The cells were stained at 37°C away from light for 30 minutes, and stored in an ice bath away from light. Flow cytometric detection was performed on the same day, and the proportion of cells in different cell cycle phases was analyzed using FlowJo. The results are shown in Figure 5. The experimental results showed that monotherapy with 0.1 nM AST-3424 or 5 µM Nutlin-3 did not significantly alter the cell cycle of p53 wild-type H460 cells. However, the combination of adding 0.1 nM AST-3424 first and then 5µM Nutlin-3 significantly reduced the G0/G1 phase and increased the G2/M phase. The S phase decreased slightly in the other two groups. However, the cell cycle of HPAF-II cells with p53 pathogenic mutation was less affected.

Numerous studies have shown that cell cycle arrest at the G2/M phase can induce cell apoptosis.

A second experiment was further conducted, in order to further study the effects of different concentrations of AST-3424 alone, Nutlin-3 alone and the two in combination and different administration sequences in the combination therapy on the cell cycle of H460/A549 cells (p53 wild-type, protein normal expression).

The remaining operations were similar to the above experiment, and the compound treatment conditions were as follows:

### Monotherapy

For H460 cell line, 1% DMSO, adding 5 µM Nutlin-3 for treatment for 22 hours, adding 0.03 nM AST-3424 for treatment for 24 hours, adding 0.1 nM AST-3424 for treatment for 24 hours, adding 0.3 nM AST-3424 for treatment for 24 hours, and adding 1 nM AST-3424 for treatment for 24 hours.

For A549 cell line, 1% DMSO, adding 5 µM Nutlin-3 for treatment for 22 hours, adding 0.3 nM AST-3424 for treatment for 24 hours, adding 1 nM AST-3424 for treatment for 24 hours, adding 9 nM AST-3424 for treatment for 24 hours, and adding 9 nM AST-3424 for treatment for 24 hours.

### Combination therapy

For H460 cell line,
Adding 0.03 nM AST-3424 for pre-treatment for 2 hours, and then adding 5 µM Nutlin-3 for co-treatment for 22 hours;
Adding 0.1 nM AST-3424 for pre-treatment for 2 hours, and then adding 5 µM Nutlin-3 for co-treatment for 22 hours;
Adding 0.3 nM AST-3424 for pre-treatment for 2 hours, and then adding 5 µM Nutlin-3 for co-treatment for 22 hours;
Adding 1 nM AST-3424 for pre-treatment for 2 hours, and then adding 5 µM Nutlin-3 for co-treatment for 22 hours.

For A549 cell line,
Adding 0.3 nM AST-3424 for pre-treatment for 2 hours, and then adding 5 µM Nutlin-3 for co-treatment for 22 hours;
Adding 1 nM AST-3424 for pre-treatment for 2 hours, and then adding 5 µM Nutlin-3 for co-treatment for 22 hours;
Adding 3 nM AST-3424 for pre-treatment for 2 hours, and then adding 5 µM Nutlin-3 for co-treatment for 22 hours;
Adding 9 nM AST-3424 for pre-treatment for 2 hours, and then adding 5 µM Nutlin-3 for co-treatment for 22 hours.

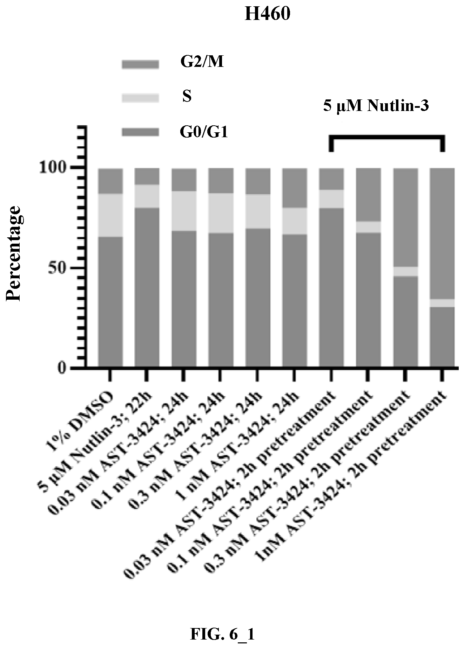

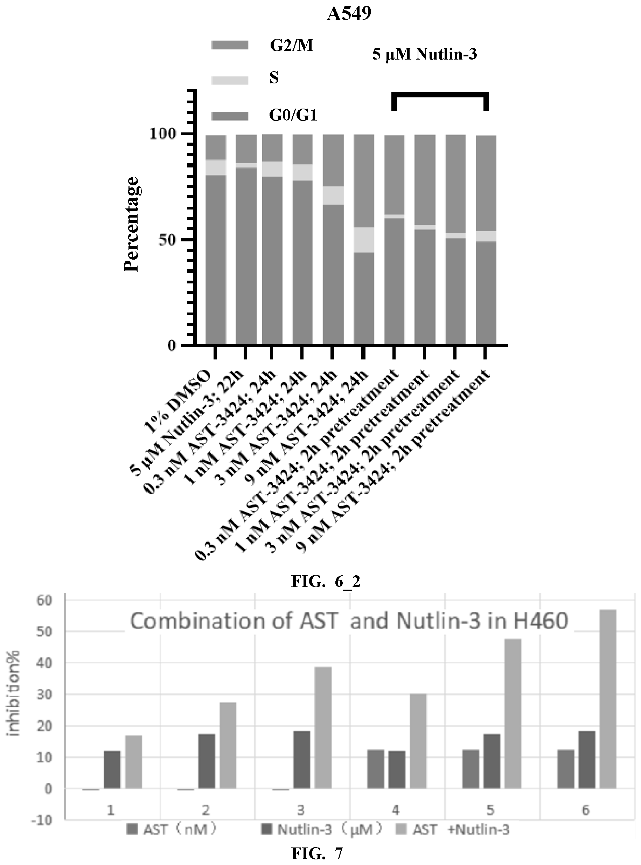

The results are shown in Figure 6.

Further experimental results showed that AST-3424 induced concentration-dependent G2/M cell cycle arrest in p53 wild-type cancer cells (H460 and A549).

The combination of AST-3424 and Nutlin-3 significantly enhanced G2/M phase arrest, decreased the G0/G1 phase, and exhibited AST-3424 concentration dependence in p53 wild-type cancer cells (H460 and A549). The enhanced G2/M phase arrest observed in the combination therapy with AST-3424 and Nutlin-3 (AST-3424 pre-treatment for 2 hours) was consistent with the results of the above initial experiment with the same administration sequence.

The above results indicate that Nutlin-3 which indirectly activates p53 function can alter the cell cycle of p53 wild-type cancer cells (H460 and A549), lead to G2/M cell cycle arrest and exert an AST-3424 concentration-dependence . However, Nutlin-3 has no effect on the cell cycle of HPAF-II cells with p53 pathogenic mutation. In other words, the combination of Nutlin-3, which indirectly activates p53 function, and AST-3424 can regulate the cell cycle of p53 wild-type cancer cells (H460 and A549), enhance G2/M cell cycle arrest and exert AST-3424 concentration-dependence, but exerts no effect on the cell cycle of HPAF-II cells with p53 pathogenic mutation.

Based on Examples 2 and 3, it can be seen that the p53 protein has a determinant effect on the efficacy of AST-3424/AST. DNA alkylating agents such as AST-3424/AST exert a stronger proliferation inhibitory effect on cells with negative p53 gene mutation and normal p53 protein. The action mechanisms thereof include G2/M cell cycle arrest and cell colony inhibition.

In order to further confirm the above conclusion, the p53-MDM2 inhibitor RITA (which is also a p53-HDM-2 inhibitor) was selected to activate p53 function, and further *in vitro* cytotoxicity experiments were carried out. The CAS number of RITA is 213261-59-7.

### Example 4: Effect of AST alone, Nutlin-3 alone and RITA alone and combination thereof on HPAF-II/H460 cytotoxicity

### In vitro proliferation inhibition experiment

### Overview of the experimental process

1) H460 and HPAF-II cell suspensions were added to a 96-well plate at 100 µL per well, with a cell density of 2000 /well and 20000/well, respectively.
2) The cells were cultured overnight in an incubator at 37°C, 5% CO₂.
3) Compound Treatment

Combination therapy: After 24 hours of cell plating, 99 µL of growth medium was supplied to each well. Each well was added with 0.5 µL of Nutlin-3 or RITA, shaken gently to ensure even mixing, and then placed in an incubator for 2 hours. Then each well was added with 0.5µL of test compounds at different concentrations, shaken gently to ensure even mixing, and then placed at 37°C, 5% CO₂ in an incubator.

Monotherapy: After 24 hours of cell plating, 99.5 µL of growth medium was supplied to each well. Then each well was added with 0.5µL of test compounds at different concentrations, shaken gently to ensure even mixing, and then placed at 37°C, 5% CO₂ in an incubator.

4) The cell plate was placed in the incubator for 72 hours.

5) The cell plate to be tested was placed at room temperature to equilibrium for 30 minutes, and 100µL of medium was discarded per well.

6) 25µL of CTG reagent was added to each well, placed at a fast shaker to shake for 2 minutes, and placed at room temperature away from light for 30 minutes.

7) The chemiluminescence signal value was read using a multifunctional microplate reader with a reading time of 1000 ms. The corresponding inhibition rate data were calculated.

The drug groups and experimental results of AST alone, Nutlin-3 alone and the two in combination in H460 cell proliferation inhibition experiment are shown in Table 2 below, and Figure 7 was plotted according to the inhibition rate data in Table 2.

**Table 2: Drug groups and inhibition rate results of AST alone, Nutlin-3 alone and the two in combination in H460 cell proliferation inhibition experiment**

| Experimental group | 1 | 2 | 3 | 4 | 5 | 6 |
|---|---|---|---|---|---|---|
| AST (nM) | 0.5 | 0.5 | 0.5 | 1.5 | 1.5 | 1.5 |
| Nutlin-3 (µM) | 0.25 | 0.5 | 1 | 0.25 | 0.5 | 1 |
| AST+Nutlin-3 | 0.5+0.25 | 0.5+0.5 | 0.5+1 | 1.5+0.25 | 1.5+0.5 | 1.5+1 |

| Experimental group and inhibition rate | Inhibition rate result | | | | | |
|---|---|---|---|---|---|---|
| | 1 | 2 | 3 | 4 | 5 | 6 |
| AST | -0.29 | -0.29 | -0.29 | 12.19 | 12.19 | 12.19 |
| Nutlin-3 | 11.8 | 17.27 | 18.3 | 11.8 | 17.27 | 18.3 |
| AST+Nutlin-3 | 17.08 | 27.33 | 38.53 | 30.07 | 47.56 | 56.99 |

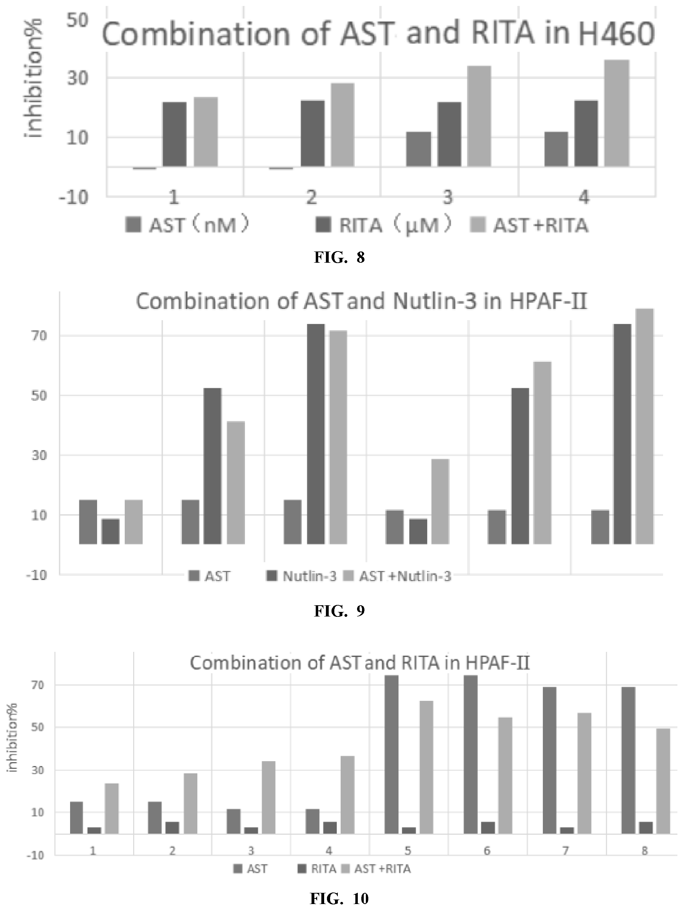

The drug groups and experimental results of AST alone, RITA (p53-HDM-2 inhibitor, indirectly upregulating p53 expression and function) alone and the two in combination in H460 cell proliferation inhibition experiment are shown in Table 3 below, and Figure 8 was plotted according to the inhibition rate data in Table 3.

**Table 3: Drug groups and inhibition rate results of AST alone, RITA alone and the two in combination in H460 cell proliferation inhibition experiment**

| Experimental group | 1 | 2 | 3 | 4 |
|---|---|---|---|---|
| AST (nM) | 0.5 | 0.5 | 1.5 | 1.5 |
| RITA (µM) | 0.025 | 0.05 | 0.025 | 0.05 |
| AST + RITA | 0.5+0.025 | 0.5+0.05 | 1.5+0.025 | 1.5+0.05 |

| Experimental group and inhibition rate | Inhibition rate result | | | |
|---|---|---|---|---|
| | 1 | 2 | 3 | 4 |
| AST | -0.29 | -0.29 | 12.19 | 12.19 |
| RITA | 22.31 | 22.4 | 22.31 | 22.4 |
| AST + RITA | 23.91 | 28.34 | 34.21 | 36.57 |

The drug groups and experimental results of AST alone, Nutlin-3 alone and the two in combination in HPAF-II cell proliferation inhibition experiment are shown in Table 4 below, and Figure 9 was plotted according to the inhibition rate data in Table 4

**Table 4: Drug groups and inhibition rate results of AST alone, Nutlin-3 alone and the two in combination in HPAF-II cell proliferation inhibition experiment**

| Experimental group | 1 | 2 | 3 | 4 | 5 | 6 |
|---|---|---|---|---|---|---|
| AST (nM) | 20 | 20 | 20 | 60 | 60 | 60 |
| Nutlin-3 (µM) | 7.5 | 10 | 15 | 7.5 | 10 | 15 |
| AST+Nutlin-3 | 20+7.5 | 20+10 | 20+15 | 60+7.5 | 60+10 | 60+15 |

| Experimental group and inhibition rate | Inhibition rate result | | | | | |
|---|---|---|---|---|---|---|
| | 1 | 2 | 3 | 4 | 5 | 6 |
| AST | 14.9 | 14.9 | 14.9 | 11.75 | 11.75 | 11.75 |
| Nutlin-3 | 8.54 | 52.46 | 73.93 | 8.54 | 52.46 | 73.93 |
| AST+Nutlin-3 | 14.84 | 41.52 | 72.03 | 28.7 | 61.42 | 79.12 |

The drug groups and experimental results of AST alone, RITA alone and the two in combination in HPAF-II cell proliferation inhibition experiment are shown in Table 5 below, and Figure 10 was plotted according to the inhibition rate data in Table 5.

**Table 5: Drug groups and inhibition rate results of AST alone, RITA alone and the two in combination in HPAF-II cell proliferation inhibition experiment**

| Experimental group | 1 | 2 | 3 | 4 | 5 | 6 | 7 | 8 |
|---|---|---|---|---|---|---|---|---|
| AST (nM) | 60 | 60 | 200 | 200 | 1667 | 1667 | 5000 | 5000 |
| RITA (µM) | 2.5 | 5 | 2.5 | 5 | 2.5 | 5 | 2.5 | 5 |
| AST + RITA | 60+2.5 | 60+5 | 200+2.5 | 200+5 | 1667+2.5 | 1667+5 | 5000+2.5 | 5000+5 |

| Experimental group and inhibition rate | Inhibition rate | | | | | | | |
|---|---|---|---|---|---|---|---|---|
| | 1 | 2 | 3 | 4 | 5 | 6 | 7 | 8 |
| AST | 14.9 | 14.9 | 11.75 | 11.75 | 74.32 | 74.32 | 68.75 | 68.75 |
| RITA | 2.86 | 5.56 | 2.86 | 5.56 | 2.86 | 5.56 | 2.86 | 5.56 |
| AST + RITA | 23.91 | 28.34 | 34.21 | 36.57 | 62.61 | 54.91 | 56.82 | 49.75 |

Clearly, compared with each monotherapy, the combination therapy of Nutlin-3 or RITA with AST significantly enhances the proliferation inhibitory effect on p53 wild-type H460 cells, whereas the combination therapy regimen exerts almost no enhancing effect on p53 mutant HPAF-II cells. In other words, the aforementioned experiments further confirm that the p53 protein has a determinant effect on the efficacy of AST-3424/AST. DNA alkylating agents such as AST-3424/AST exert a stronger proliferation inhibitory effect on cells with negative p53 gene mutation and normal p53 protein.

In particular, taking AST monotherapy as an example, to achieve an inhibition rate of 11-12%, only a concentration of 1.5 nM is required for p53 wild-type H460 cells, while 60 nM is needed for p53 mutant HPAF-II cells. This indicates that DNA alkylating agents such as AST/AST-3424 have a stronger proliferation inhibitory effect on p53 wild-type cancer cells.

The expression levels of AKR1 C3 protein in H460 and HPAF-II cells are very close, indicating that the degree of activation of AST-3424/AST is similar in the two types of cells. However, taking AST monotherapy and AST-3424 monotherapy as examples, the applicant's previous cancer cell proliferation inhibition test data are as follows:
The ICso values of AST in H460 and HPAF-II cells are 6.87 nM and 329.1 nM respectively.

The IC₅₀ values of AST-3424 in H460 and HPAF-II cells are 0.47 nM and 107.3 nM respectively.

The above data further verified that DNA alkylating agents such as AST/AST-3424 have a stronger proliferation inhibitory effect on p53 wild-type cancer cells.

Based on Examples 2, 3 and 4, it can be known that p53 protein has a determinant effect on the efficacy of AST-3424/AST. DNA alkylating agents such as AST-3424/AST exert a stronger proliferation inhibitory effect on cells with negative p53 gene mutation and normal p53 protein. The action mechanisms thereof include G2/M cell cycle arrest and cell colony inhibition.

To further explain the above experimental phenomena, the effect of monotherapy or combination therapy on the p53 protein pathway was detected.

### Example 5: experiment of effect of AST-3424 ± Nutlin-3 on p53 protein pathway

The first experiment of the effect of AST-3424 alone and Nutlin-3 alone and the two in combination on Ser15-p53, Ser20-p53, Total p53, MDM2 and p21 proteins in H460 cells

Overview of the experimental process:
1) H460 cell suspension was added to a 24-well plate, with a cell density of 80000 /well and 995 µL of medium per well.
2) The cells were cultured overnight in an incubator at 37°C, 5% CO₂.
3) Compound Treatment

After 24 hours of cell plating, according to the protocol, each cell line experiment was grouped as: Monotherapy: After 24 hours of cell plating, each well was supplied with 5µL of test compounds at different concentrations: 0.5% DMSO, 0.1nM AST-3424 and 5 µM Nutlin-3, shaken gently to ensure even mixing, and then placed at 37°C, 5% CO₂ in an incubator.

Combination therapy: After 24 hours of cell plating, each well was supplied with 5µL of test compounds at different concentrations. The cells were pre-treated with 5µM Nutlin-3 for 2 hours and then were added with 0.1nM AST-3424, shaken gently to ensure even mixing, and then placed at 37°C, 5% CO₂ in an incubator.

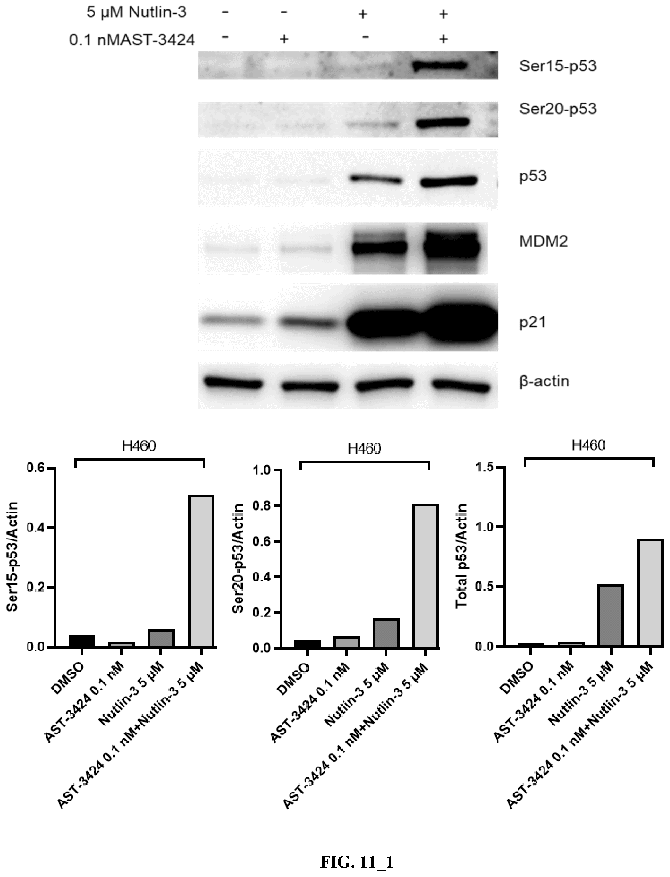

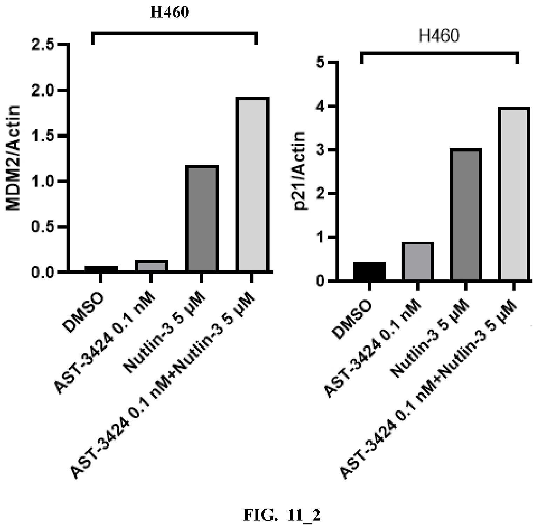

4) Cell protein lysates were collected for WB analysis. The results of the WB detection band are shown in Figure 11.

WB detection refers to the Western Blot (WB) experiment.

Compared with AST-3424 alone or Nutlin-3 alone, the combination of AST-3424 and Nutlin-3 significantly promoted the phosphorylation of p53 (Ser15-p53 and Ser20-p53) and the protein expression of total-p53, and upregulated the expression of MDM2 and p21, the downstream genes of p53.

In order to further investigate the effects of the combination of AST-3424 at different concentrations and Nutlin-3, the second experiment was conducted.

The second experiment of the effect of AST-3424 alone and Nutlin-3 alone and the two in combination on Ser15-p53, Ser20-p53, Total p53, MDM2 and p21 proteins in H460 cells

The remaining operations were similar to the first experiment, except that the compound treatments for each group were as follows:
Monotherapy group, 1% DMSO, adding 5 µM Nutlin-3 for treatment for 24 hours, adding 0.1 nM AST-3424 for treatment for 24 hours, adding 0.3 nM AST-3424 for treatment for 24 hours, adding 1 nM AST-3424 for treatment for 24 hours.

Combination therapy group:
Adding 0.1 nM AST-3424 for pre-treatment for 2 hours, and then adding 5 µM Nutlin-3 for co-treatment for 22 hours;
Adding 0.3 nM AST-3424 for pre-treatment for 2 hours, and then adding 5 µM Nutlin-3 for co-treatment for 22 hours;
Adding 1 nM AST-3424 for pre-treatment for 2 hours, and then adding 5 µM Nutlin-3 for co-treatment for 22 hours.

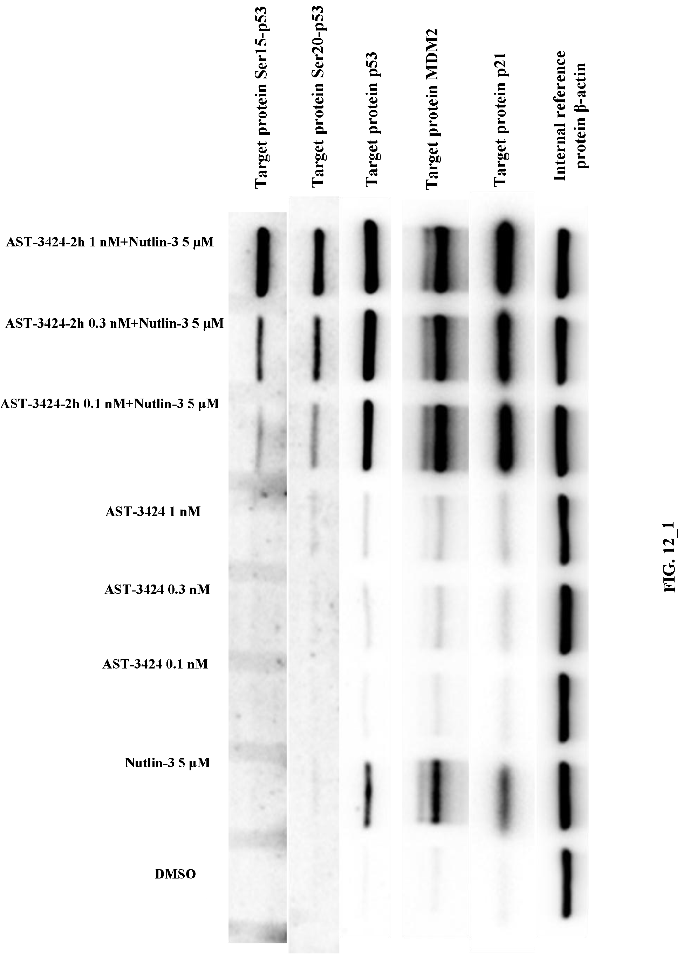

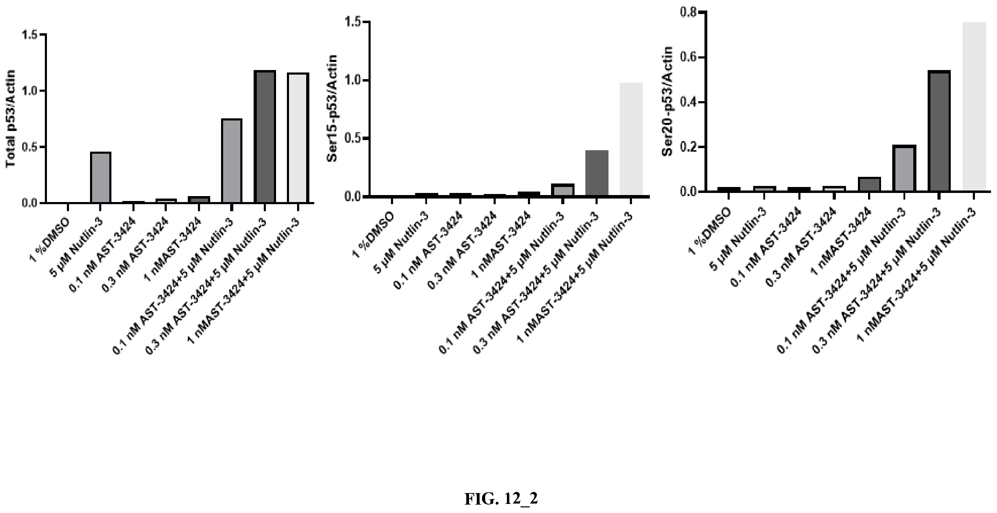

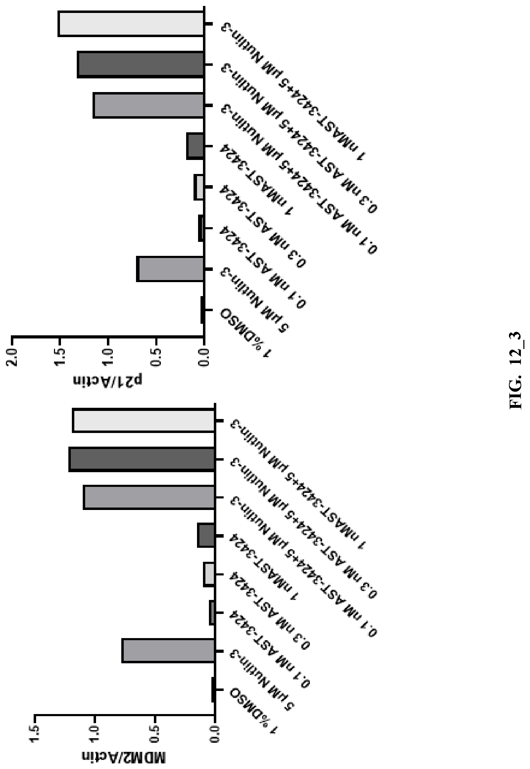

The results are shown in Figure 12.

Experimental results indicated that Nutlin-3 upregulated the expression levels of p53 and its downstream proteins p21 and MDM2 in H460 cells (p53 wild-type with normal protein expression), which was consistent with the data reported in the literature Meijer A, Kruyt FA, van der Zee AG, et al. Nutlin-3 preferentially sensitises wild-type p53-expressing cancer cells to DR5-selective TRAIL over rhTRAIL. Br J Cancer. 2013;109(10):2685-2695. doi:10.1038/bjc.2013.636). Compared with each monotherapy, the combination of AST-3424 at increasing concentrations and Nutlin-3 significantly promoted the phosphorylation of p53 (Ser15-p53 and Ser20-p53) and the protein expression of total-p53, and upregulated the expression of MDM2 and p21, the downstream genes of p53. Meanwhile, the effect on the expression of the aforementioned proteins was AST-3424 dose-dependent.

In order to further investigate the effect of administration sequence on the efficacy of the combination of AST-3424 and Nutlin-3, the third experiment was conducted.

The third experiment of the effect of AST-3424 alone and Nutlin-3 alone and the two in combination on Ser15-p53, Ser20-p53, total p53, MDM2 and p21 proteins in H460 cells
The remaining operations were similar to the first experiment, except that the compound treatments for each group were as follows:
Monotherapy group, 0.5% DMSO, 5 µM Nutlin-3 for 24 hours, 0.1 nM AST-3424 for 24 hours.
Combination therapy group:
   Adding 0.1 nM AST-3424 for pre-treatment for 2 hours, and then adding 5 µM Nutlin-3 for co-treatment for 22 hours;
   Adding 0.1 nM AST-3424 and 5 µM Nutlin-3 for co-treatment for 24 hours;
   Adding 5 µM Nutlin-3 for pre-treatment for 2 hours, and then adding 0.1 nM AST-3424 for co-treatment for 22 hours.

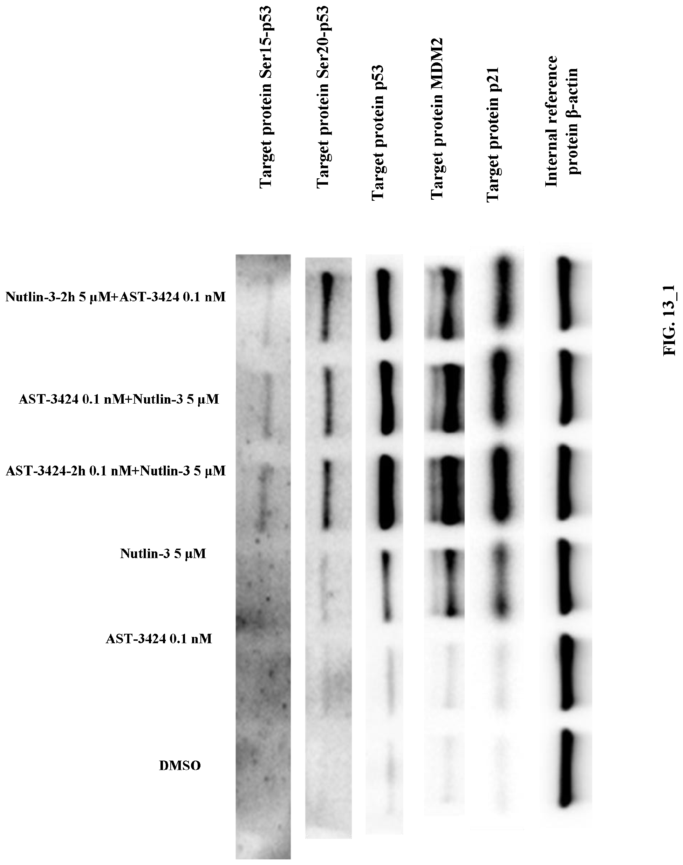

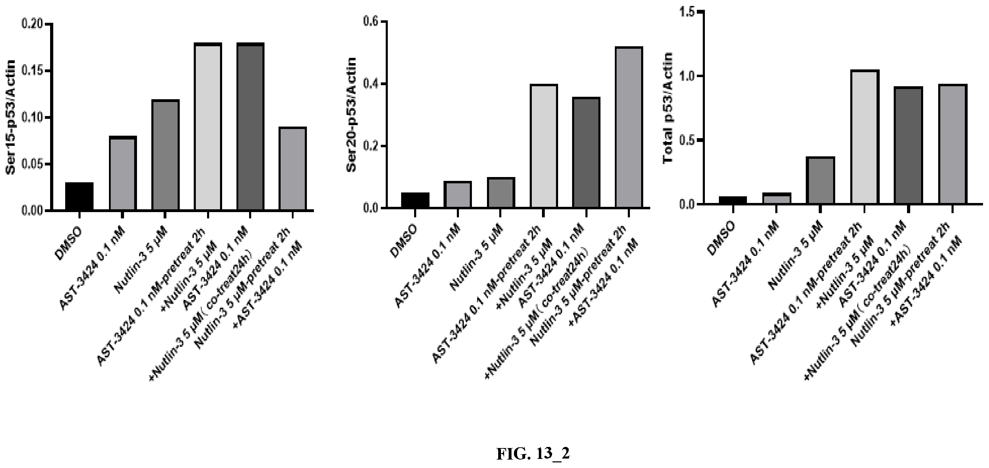

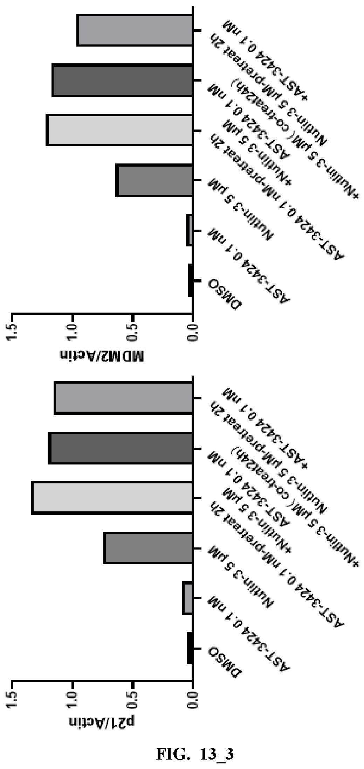

The results are shown in Figure 13.

The experimental results showed that compared with each monotherapy, the combination of AST-3424 and Nutlin-3 significantly promoted the phosphorylation of p53 (Ser15-p53 and Ser20-p53) and the protein expression of total-p53, and upregulated the expression of MDM2 and p21, the downstream genes of p53. However, there was no significant difference in the effects of different administration sequences on the expression of the aforementioned proteins.

### Example 6: The effects of Nutlin-3 alone, AST-3424 alone and the two in combination on RAD51 protein amount

The combination of AST-3424 and Nutlin-3 significantly inhibited the protein expression of RAD51

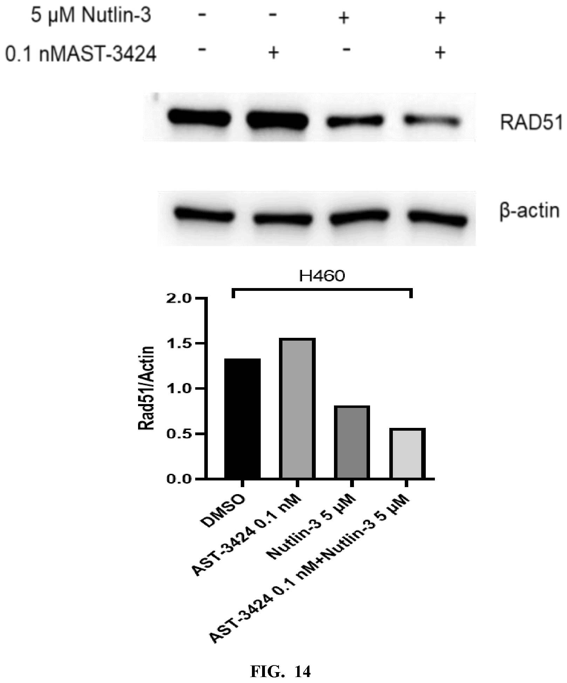

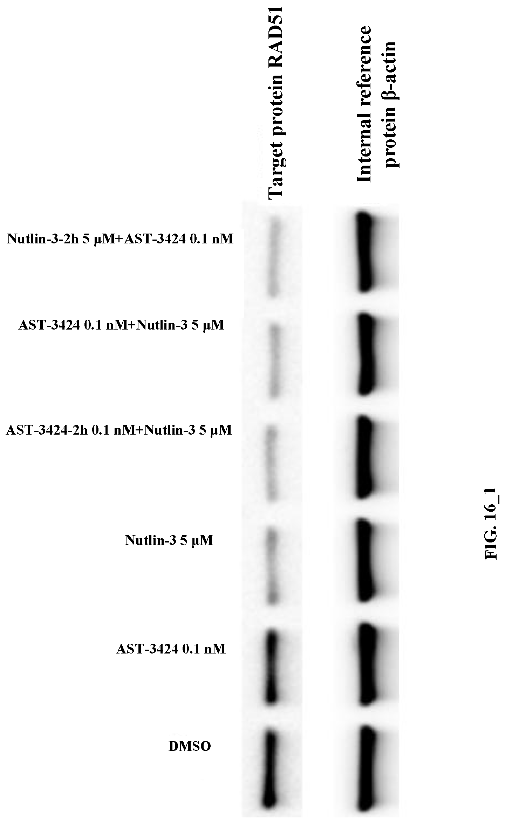

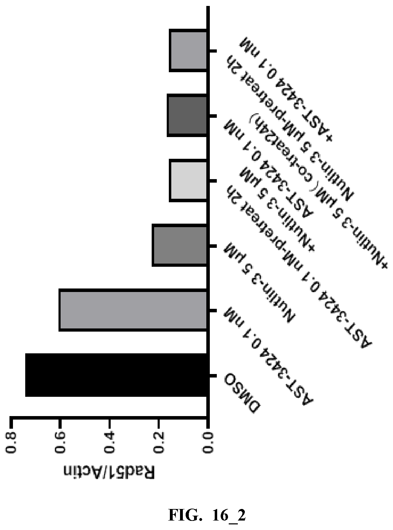

In the first, the second and the third experiments of the effect of AST-3424 in combination with Nutlin-3 on Ser15-p53, Ser20-p53, Total p53, MDM2 and p21 proteins in H460 cells in example 5, the applicant also performed WB detection of RAD51 protein, with the results shown in Figures 14, 15 and 16.

The results of all the above three experiments indicated that compared with AST-3424 alone and Nutlin-3 alone, the combination therapy significantly reduced the RAD51 protein amount and was AST-3424 dose-dependent.

The combination of AST-3424 with Nutlin-3 increases RAD51 protein degradation

### Overview of the experimental process:

1) H460 cell suspension was added to a 24-well plate, with a cell density of 100000 /well and 995 µL of medium per well.
2) The cells were cultured overnight in an incubator at 37°C, 5% CO₂.
3) Compound Treatment

Monotherapy: After 24 hours of cell plating, each well was supplied with 5µL of test compounds at different concentrations: 1% DMSO, 0.1nM AST-3424 and 5 µM Nutlin-3, shaken gently to ensure even mixing, and then placed at 37°C, 5% CO₂ in an incubator. After continuing culture for 24 hours, the cells were collected.

Combination therapy: After 24 hours of cell plating,
Adding 0.1 nM AST-3424 for pre-treatment for 2 hours, and then adding 5 µM Nutlin-3 for co-treatment for 22 hours;
Adding 1% DMSO and 5 µM MG-132 for co-treatment for 24 hours;
Adding 0.1 nM AST-3424 and 5 µM MG-132 for co-treatment for 24 hours;
Adding 5 µM Nutlin-3 and 5 µM MG-132 for co-treatment for 24 hours;
Adding 0.1 nM AST-3424 for pre-treatment for 2 hour, and then adding 5 µM Nutlin-3 for treatment for 16 hours, and then adding 5µM MG-132 for co-treatment for 6 hours.

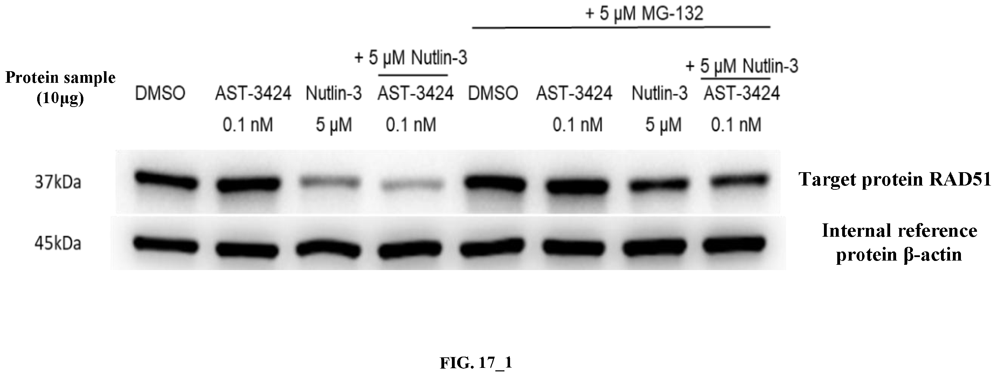

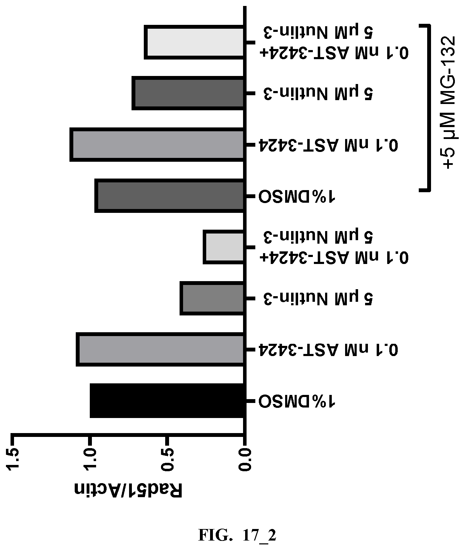

4) Collected the cell protein lysate for WB detection, and the detection results were shown in Figure 17.

MG-132 is a proteasome (26Sproteasome) inhibitor that inhibits the degradation of proteins upon administration.

The administration of MG-132 reversed the downregulation of RAD51 protein induced by the combination of AST-3424 and Nutlin-3, thus suggesting that the reduction in RAD51 protein content caused by the combination therapy is associated with the increase in the ubiquitination degradation of RAD51 protein.

The combination of AST-3424 and Nutlin-3 reduces the half-life of RAD51 protein

### Overview of the experimental process:

1) H460 cell suspension was added to a 24-well plate, with a cell density of 100000 /well and 995 µL of medium per well.
2) The cells were cultured overnight in an incubator at 37°C, 5% CO₂.
3) Compound Treatment

After 24 hours of cell plating, according to the protocol, each cell line experiment was grouped as: Monotherapy group: 1% DMSO treatment group, 5 µM Nutlin-3 treatment group, 0.1 nM AST-3424 treatment group.

### Combination therapy group:

1%DMSO+Cycloheximide 0.5 hour treated group,
1%DMSO+Cycloheximide 1 hour treated group,
1%DMSO+Cycloheximide 2 hour treated group,
1%DMSO+Cycloheximide 4 hour treated group,
0.1nM AST-3424+Cycloheximide 0.5 hour treated group,
0.1nM AST-3424+Cycloheximide 1 hour treated group,
0.1nM AST-3424+Cycloheximide 2 hour treated group,
0.1nM AST-3424+Cycloheximide 4 hour treated group,
5 µM Nutlin-3 + Cycloheximide 0.5 hour treated group,
5 µM Nutlin-3 + Cycloheximide 1 hours treated group,
5 µM Nutlin-3 + Cycloheximide 2 hours treated group,
5 µM Nutlin-3 + Cycloheximide 4 hours treated group,
0.1 nM AST-3424 + 5 µM Nutlin-3 treated group,
0.1 nM AST-3424 + 5 µM Nutlin-3 + Cycloheximide 0.5 hour treated group,
0.1 nM AST-3424 + 5 µM Nutlin-3 + Cycloheximide 1 hour treated group,
0.1 nM AST-3424 + 5 µM Nutlin-3 + Cycloheximide 2 hour treated group,
0.1 nM AST-3424 + 5 µM Nutlin-3 + Cycloheximide 4 hours treated group,

Monotherapy: After 24 hours of cell plating, each well was supplied with 5µL of test compounds at different concentrations, shaken gently to ensure even mixing, and then placed at 37°C, 5% CO₂ in an incubator. After continuing culture for 24 hours, the cells were collected.

### Combination therapy:

After 24 hours of cell plating, each well was supplied with 5µL of 1% DMSO, and then placed at 37°C, 5% CO₂ in an incubator for continuing culture for 24 hours. After that, the cells were added with 5µL of 4µM Cycloheximide. After continuing culture at 37°C, 5% CO₂ in an incubator for 0.5, 1, 2 and 4 hours respectively, the cells were collected.

After 24 hours of cell plating, each well was supplied with 5µL of 0.1nM AST-3424, and then placed at 37°C, 5% CO₂ in an incubator for continuing culture for 24 hours. After that, the cells were added with 5µL of 4µM Cycloheximide. After continuing culture at 37°C, 5% CO₂ in an incubator for 0.5, 1, 2 and 4 hours respectively, the cells were collected.

After 24 hours of cell plating, each well was supplied with 5µL of 5µM Nutlin-3, and then placed at 37°C, 5% CO₂ in an incubator for continuing culture for 24 hours. After that, the cells were added with 5µL of 4µM Cycloheximide. After continuing culture at 37°C, 5% CO₂ in an incubator for 0.5, 1, 2 and 4 hours respectively, the cells were collected.

After 24 hours of cell plating, each well was supplied with 5µL of 0.1 nM AST-3424, and then placed at 37°C, 5% CO₂ in an incubator for continuing culture for 2 hours. After that, the cells were added with 5µL of 5µM Nutlin-3, and then placed at 37°C, 5% CO₂ in an incubator for continuing culture for 24 hours. After that, the cells were collected.

After 24 hours of cell plating, each well was supplied with 5µL of 0.1nM AST-3424, and then placed at 37°C, 5% CO₂ in an incubator for continuing culture for 2 hours. After that, the cells were added with 5µL of 5µM Nutlin-3, shaken gently to ensure even mixing, and then placed at 37°C, 5% CO₂ in an incubator for continuing culture for 24 hours. Then the cells were added with 5µL of 4µM Cycloheximide and placed at 37°C, 5% CO₂ in an incubator for 0.5, 1, 2 and 4 hours respectively. After that, the cells were collected.

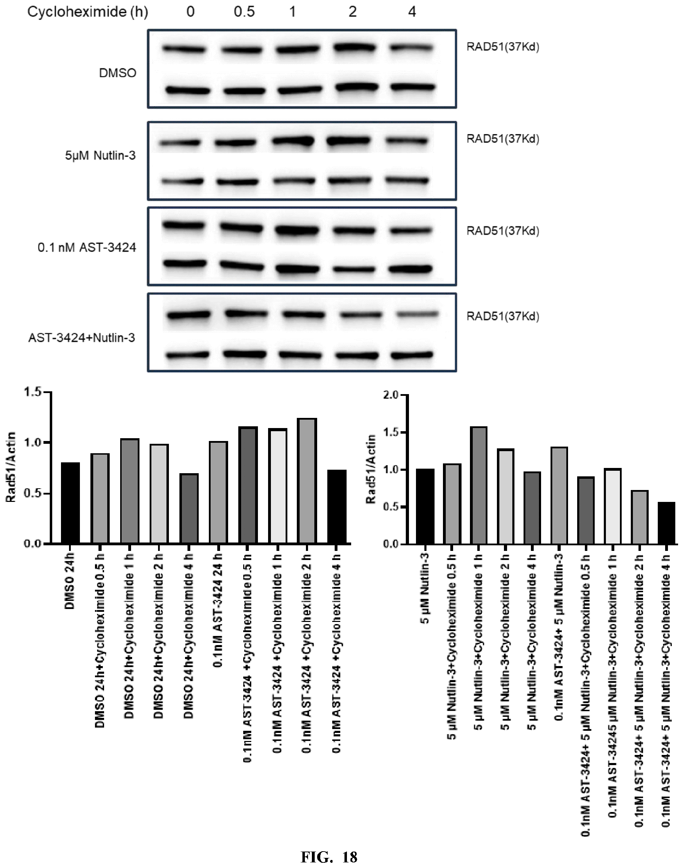

4) Collected the cell protein lysate for WB detection, and the detection results were shown in Figure 18.

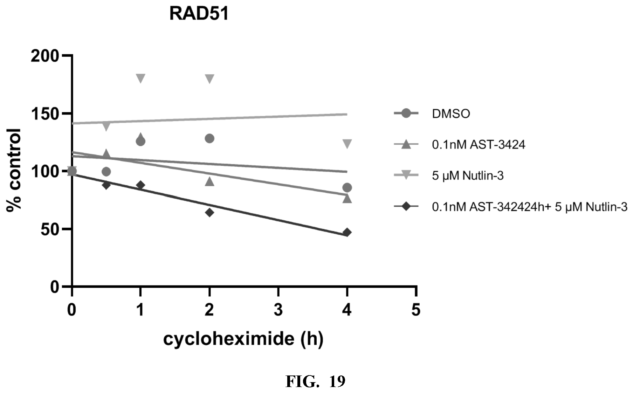

Furthermore, based on the ratio data of the corresponding protein relative to the internal reference protein β-actin in Figure 18, the changes of the relative amount of RAD51 at different time points were calculated and plotted as curves, with the results shown in Figure 19.

Cycloheximide (cycloheximide) is an inhibitor for eukaryotic protein synthesis which terminates the protein synthesis upon administration.

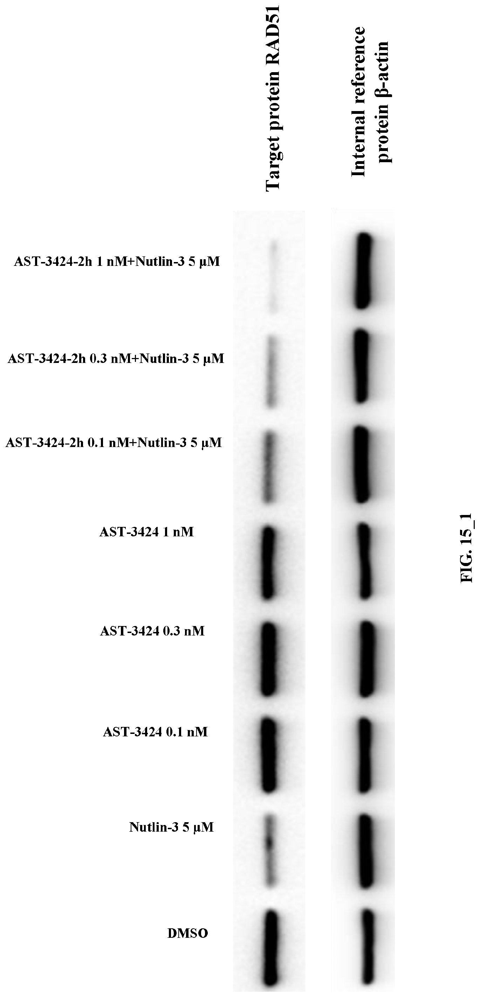

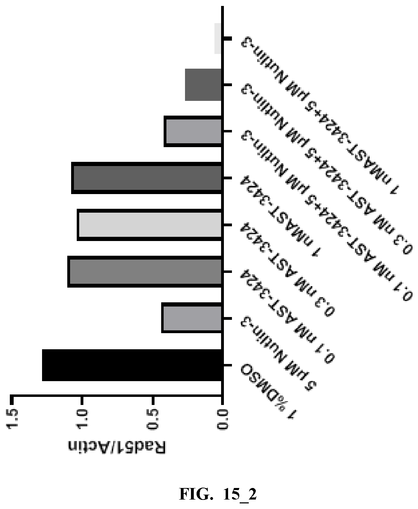

After treating cells with AST-3424 alone, Nutlin-3 alone and the two in combination for 24 hours, no new protein was synthesized after cycloheximide was added. As shown in figure 15, the RAD51 protein amount was reduced in each group. However, compared with each monotherapy, the combination of Nutlin-3 and AST-3424 significantly decreased the RAD51 protein expression amount and its half-life, further confirming that the combination therapy can promote RAD51 degradation.

### Example 7: Effect of AST-3424 in combination with Nutlin-3 on DNA damage process in H460 cells

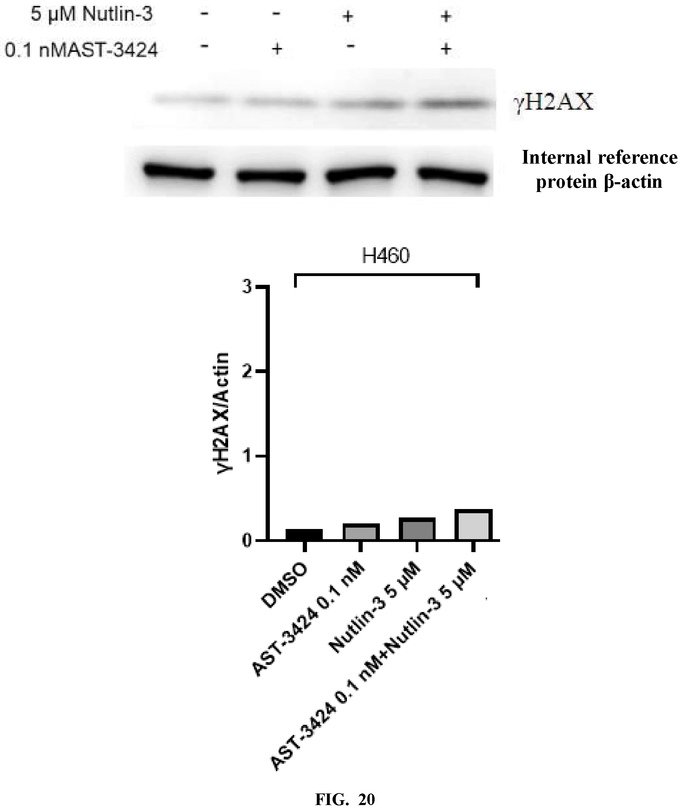

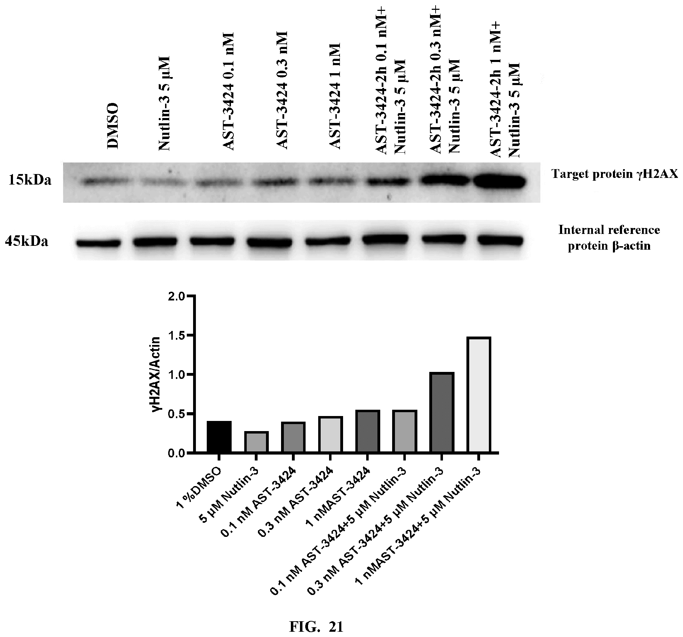

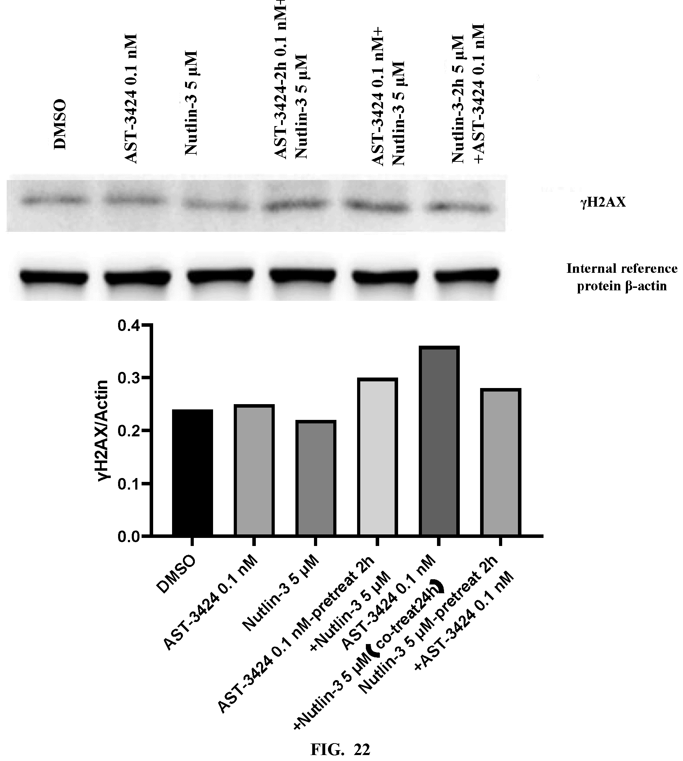

In the first, the second and the third experiment of the effect of AST-3424 in combination with Nutlin-3 on Ser15-p53, Ser20-p53, Total p53, MDM2 and p21 proteins in H460 cells in example 5, the applicant also performed WB detection of γH2AX protein, with the results shown in Figures 20, 21 and 22.

The results showed that AST-3424 could upregulate the amount of γH2AX protein, a biomarker of DNA double-strand damage, in a dose-dependent manner.

Compared with each monotherapy, the combination of AST-3424 and Nutlin-3 further increased the γH2AX protein amount and was AST-3424 dose-dependent. However, the combination of AST-3424 and Nutlin-3 upregulated γH2AX protein more significantly.

### Example 8: Effects of Nutlin-3/AST treatment for 24 hours on the expression of p53, Rad51, MDM2 and p21 proteins in HPAF-II cells

### Overview of the experimental process:

1)HPAF-II cell suspension was added to a 24-well plate, with a cell density of 300000 /well and 995 µL of medium per well.
2) The cells were cultured overnight in an incubator at 37°C, 5% CO₂.
3) Compound treatment. After 24 hours of cell plating, the cells were grouped and dosed according to different cells.

HPAF-II were grouped as: 1% DMSO treated group, 60nM AST treated group, 8 µM Nutlin-3 treated group, AST 60nM + Nutlin-3 8 µM treated group.

Monotherapy: After 24 hours of cell plating, each well was supplied with 5µL of test compounds at different concentrations: 1% DMSO, AST 1.5nM, Nutlin-3 1µM, AST 60nM and Nutlin-3 8µM, shaken gently to ensure even mixing, and then placed at 37°C, 5% CO₂ in an incubator.

Combination therapy: After 24 hours of cell plating, each well was supplied with 5µL of 1µM Nutlin-3 and Nutlin-3 8µM respectively for pre-treatment four 2 hours. Then the corresponding cells were added with 5µM of 1.5nM AST and 60nM AST, shaken gently to ensure even mixing, and then placed at 37°C, 5% CO₂ in an incubator.

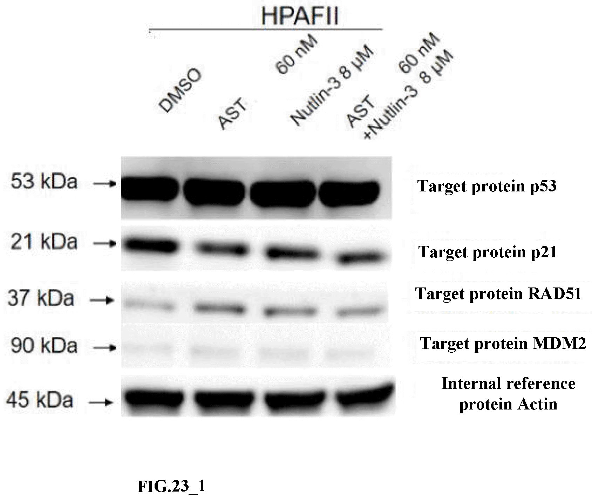

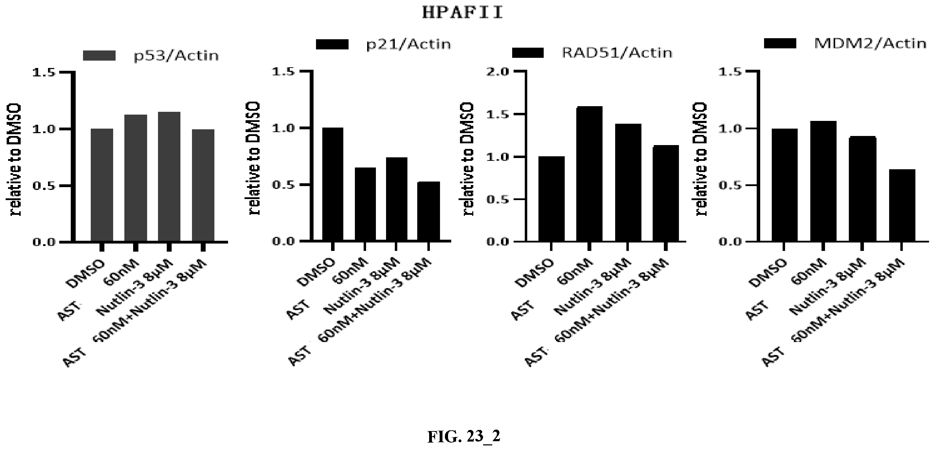

4) After incubating the 24-well plates in a 37°C, 5% CO₂ incubator for 24 hours, the cell protein lysate was collected for WB detection, and the WB detection results were shown in Figure 23.

Nutlin-3 is an inhibitor for combination of p53 and MDM2 protein, activating the function of p53.

The experimental results indicated that 60 nM AST had no effect on the expression of p53, its downstream proteins p21 and MDM2 in HPAF-II cells (cells with p53 pathogenic mutation).

The combination of AST and Nutlin-3 had no effect on the expression of p53, its downstream proteins p21 and MDM2 in HPAF-II cells (with p53 mutation and abnormal protein expression).

AST monotherapy promoted the expression of RAD51 protein in HPAF-II cells (with p53 mutation and abnormal protein expression), but the combination of AST and Nutlin-3 had no synergistic effect.

Example 9: Effects of high concentration Nutlin-3/AST-3424 treatment of HPAF-II cells on Ser15-p53, Ser20-p53, p53, Rad51, MDM2, p21, γH2AX and apoptosis-related proteins Caspase 3 and Cleaved Caspase 3

The aforementioned experiments revealed that for HPAF-II cells with p53 gene mutation, lower concentrations of Nutlin-3/AST-3424 had little effect on related proteins. In order to further verify this finding, these experiments were conducted with high concentrations of Nutlin-3/AST-3424 drug combination: 5 µM Nutlin-3 + 1000 nM AST-3424, to exclude the impact of concentration.

Overview of the experimental process:
1) HPAF-II cell suspension was added to a 24-well plate, with a cell density of 300000 /well and 995 µL of medium per well.
2) The cells were cultured overnight in an incubator at 37°C, 5% CO₂.
3) Compound Treatment

After 24 hours of cell plating, according to the protocol, each cell line experiment was grouped as: Monotherapy: After 24 hours of cell plating, each well was supplied with 5µL of test compounds at different concentrations: 0.5% DMSO, 1000nM AST-3424 and 15 µM Nutlin-3, shaken gently to ensure even mixing, and then placed at 37°C, 5% CO₂ in an incubator.

Combination therapy: After 24 hours of cell plating, each well was supplied with 5µL of test compounds at different concentrations. The cells were pre-treated with 15µM Nutlin-3 for 2 hours and then were added with 1000nM AST-3424, shaken gently to ensure even mixing, and then placed at 37°C, 5% CO₂ in an incubator.

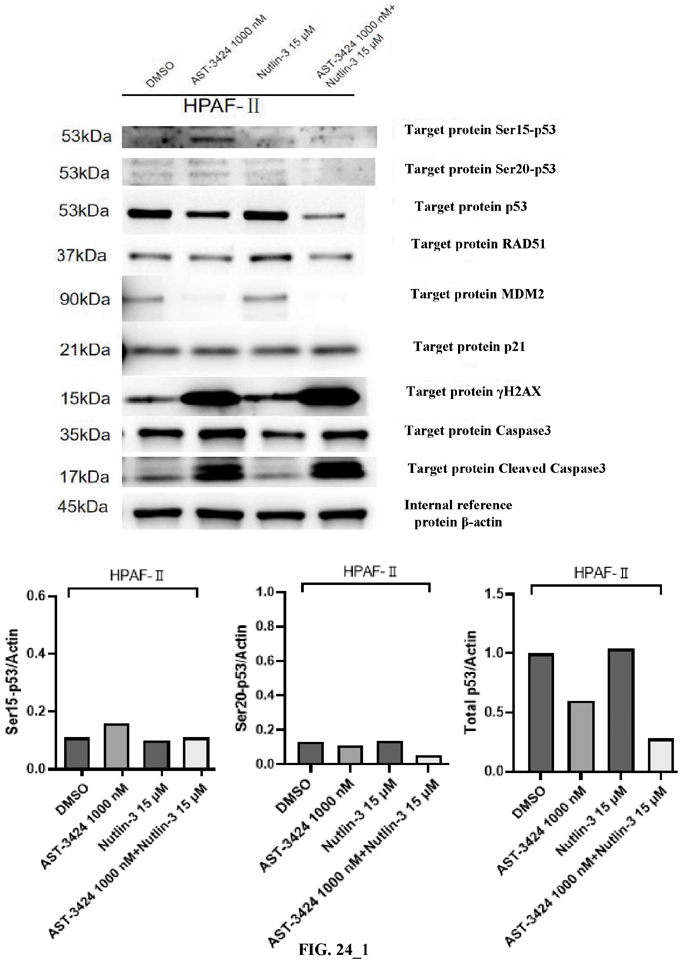

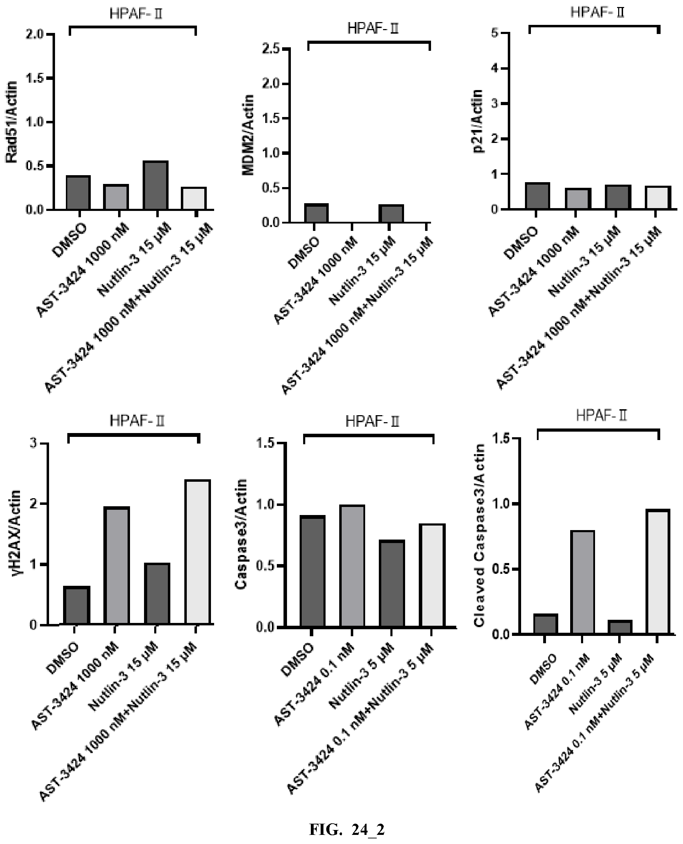

4) Cell protein lysates were collected for WB analysis. The results of the WB detection band are shown in Figure 24.

Clearly, in the case of high concentration administration, any enhancement of protein expression induced by the combination therapy was not observed, or the enhancement of effect induced by the combination therapy was not obvious in HPAF-II cells of p53 mutant cell line.

Based on the above examples 5 to 9, the following conclusions can be drawn:
1. The drugs Nutlin-3/RITA, which upregulate p53 protein expression or activate p53 function, can enhance the *in vitro* toxicity of AST-3424/AST to cancer cells. Moreover, the enhancement is more significant for p53 wild-type cancer cells.
2. The drugs which upregulate p53 protein expression or activate p53 function can enhance the apoptosis caused by AST-3424/AST. Especially for p53 wild-type cancer cells, the apoptosis caused by combination therapy is more significant.
3. The drugs Nutlin-3/RITA, which upregulate p53 protein expression or activate p53 function, can significantly increase the DNA double-strand damage caused by AST-3424/AST. For p53 wild-type cancer cells, the DNA double-strand damage caused by combination therapy is more significant.
4. The drugs Nutlin-3/RITA, which upregulate p53 protein expression or activate p53 function can significantly increase the cell cycle G2/M arrest caused by AST-3424/AST. For p53 wild-type cancer cells, the cell cycle G2/M arrest caused by combination therapy is more significant.
   In summary, the above enhancements reveal that DNA alkylating agents such as AST/AST-3424 have a stronger proliferation inhibitory effect on p53 wild-type cells.
5. Compared with monotherapy, the combination of the drugs Nutlin-3/RITA which upregulate p53 protein expression or activate p53 function, and AST-3424/AST would significantly upregulate p53 phosphorylation, total-p53 expression and the expression of p53 downstream proteins MDM2 and p21, and activate the p53 protein pathway and in turn downregulate the protein expression of RAD51. For p53 wild-type cancer cells, the above phenomena caused by combination therapy are more significant.
6. Compared with monotherapy, the combination of the drugs Nutlin-3/RITA, which upregulate p53 protein expression or activate p53 function, and AST-3424/AST would significantly promote the degradation of RAD51 protein and reduce the half-life of RAD51 protein.

In view of the above, the p53 protein promotes the degradation of homologous recombination repair protein RAD5 1, leading to RAD51 downregulation and reduction of repair capacity for DNA double-strand damage, thereby enhancing the pharmacological activity of DNA alkylating agents such as AST-3424/AST. In other words, the patients with normal p53 protein and p53 gene are more sensitive to the treatment with DNA alkylating agents such as AST-3424/AST due to the above process and pathway involved by p53 protein. The patients may benefit more significantly from the treatment (compared with the patients with low p53 protein expression or p53 gene mutation). Such phenomenon has been preliminarily observed in the clinical trials of AST-3424.

Example 10: Effects of compounds on the in vitro proliferation of H460 cells and p53 knockout cell lines H460 P53 KO #1, H460 P53 KO #7, and H460 P53 KO #12 under normoxia

The p53 CRISPR/Cas9 KO plasmid (human) and p53 HDR plasmid (human) were purchased from Santa Cruz Company to construct H460 P53 KO cell lines, namely p53 knockout (KnockOut) H460 cell lines. Three groups of H460 P53 KO cell lines were constructed with numbers of H460 P53 KO # 1, H460 P53 KO # 7, and H460 P53 KO # 12.

The construction process of p53 gene knockout cell is briefly described as follows:
H460 cells were plated in 6-well plates with 1 × 10⁶/well.
H460 cells were co-transfected with p53 CRISPR/Cas9 KO plasmid (h) (sc-416469) and p53 HDR plasmid (h) (sc-416469-HDR): 125 µL Opti-MEM + 1.25 µg each of the two plasmids + P3000 5 µL; 125 µL Opti-MEM+15 µL (increasing the volume, expected to increase transfection efficiency) liposome 3000. After co-incubation for 15 minutes, they were added dropwise to the culture medium and mixed well.

After 48 h, 2 µg/mL puromycin was added for screening.

Two days later, the medium was replaced with fresh 1640 medium containing 1 µg/mL puromycin, and the medium was changed every two days thereafter.

When the cell colonies grew to a sufficient size, single colonies were picked and cultured.

When the cell number reached a sufficient quantity, samples were collected for WB identification of the colonies. Colonies with no p53 expression were the p53 knockout H460 cell clones.

The experimental procedures of this example can be referred to in the overview of the experimental process in section "Effects of compounds on H460 cell proliferation in vitro under normoxia" in example 2.

### Compound Treatment

Monotherapy: Compounds AST-3424/AST were administered alone in H460 and H460 P53 KO cell lines, respectively.

Combination therapy: Compounds AST-3424/AST were administered in combination with AST-3021, respectively, in H460 and H460 P53 KO cell lines, respectively.

H460 P53 KO cell lines, i.e. H460 P53 KO # 1, H460 P53 KO # 7, H460 P53 KO # 12 cell lines were used in experiments, respectively.

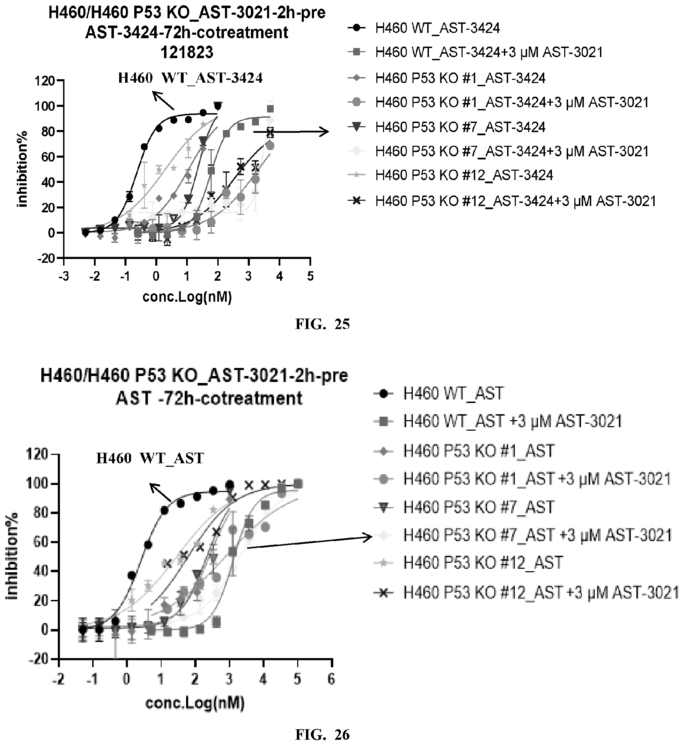

The experimental results of AST-3424 are shown in Table 11. The corresponding in vitro proliferation inhibition rate curves of AST-3424 alone and in combination with AST-3021 on H460 cells and H460 P53 KO cell lines are shown in Figure 25.

The experimental results of AST are shown in Table 12. The corresponding in vitro proliferation inhibition rate curves of AST alone and in combination with AST-3021 on H460 cells and H460 P53KO cell lines are shown in Figure 26.

**Table 11: Inhibition rate results of AST-3424 on in vitro proliferation of H460 WT (H460 wild-type cells) and H460 P53 KO #1, H460 P53 KO #7 and H460 P53 KO #12**

| Cell line/compound combination | IC₅₀ (nM) | Ratio, combination therapy/ monotherapy | Ratio (monotherapy), knockout/wild |
|---|---|---|---|
| H460 WT _ AST-3424 | 0.23 | | |
| H460 WT _ AST-3424 + 3µM AST-3021 | 57.72 | 250.96 | |
| H460 P53 KO # 1 _ AST-3424 | 11.3 | | 49.13 |
| H460 P53 KO # 1 _ AST-3424 + 3µM AST-3021 | 2107 | 186.46 | |
| H460 P53 KO # 7 _ AST-3424 | 23.1 | | 100.43 |
| H460 P53 KO # 7 _ AST-3424 + 3µM AST-3021 | 2709 | 117.27 | |
| H460 P53 KO # 12 _ AST-3424 | 1.99 | | 8.65 |
| H460 P53 KO # 12 _ AST-3424 + 3µM AST-3021 | 344.7 | 173.22 | |

The experimental data shown in Table 11 and Figure 25 show that the sensitivity of H460 P53 KO #1 and #12 to AST-3424 was decreased by 49.13-fold and 8.65-fold, respectively, compared with H460 wild-type cells, which indicates that p53 deficiency resulted in reduced sensitivity to AST-3424 and had no effect on the AKR1C3 protein selectivity of AST-3424. This experiment further corroborated the experimental results of Examples 2 and 3: p53 protein has a determinant effect on the efficacy of AST-3424, and DNA alkylating agents such as AST-3424 exert a stronger proliferation inhibitory effect on cells with negative p53 gene mutation and normal p53 protein.

**Table 12: Inhibition rate results of AST on in vitro proliferation of H460 and H460 P53 KO #1, H460 P53 KO #7 and H460 P53 KO #12.**

| Cell line/compound combination | IC₅₀(nM) | Ratio, combination therapy/ monotherapy | Ratio (monotherapy), knockout/wild |
|---|---|---|---|
| H460 WT _ AST | 2.55 | | |
| H460 WT _ AST+3 µM AST-3021 | 1283 | 503.14 | |
| H460 P53 KO # 1 _ AST | 264 | | 103.53 |
| H460 P53 KO # 1 _ AST+3µM AST-3021 | 745.8 | 2.83 | |
| H460 P53 KO # 7 _ AST | 219.5 | | 86.08 |
| H460 P53 KO # 7 _ AST+3µM AST-3021 | 2166 | 9.87 | |
| H460 P53 KO # 12 _ AST | 27.96 | | 10.96 |
| H460 P53 KO # 12 _ AST+3µM AST-3021 | 60.53 | 2.16 | |

The experimental data shown in Table 12 and Figure 26 show that the sensitivity of H460 P53KO KO #1 and #12 to AST was decreased by 103.53-fold and 10.96-fold, respectively, compared with H460 wild-type cells, which indicates that p53 deficiency resulted in reduced sensitivity to AST. But the p53 deficiency lead to the almost loss of selectivity of AKR1C3 protein by AST. This experiment further corroborated the experimental results of Examples 4: p53 protein has a determinant effect on the efficacy of AST, and DNA alkylating agents such as AST exert a stronger proliferation inhibitory effect on cells with negative p53 gene mutation and normal p53 protein.

### Example 11: Effects of compounds on the in vitro proliferation of H460 cells, H460 P53 KO #1, H460 P53 KO #7, H460 P53 KO #12 cells under hypoxic conditions

### Overview of the experimental process:

1) H460, H460 P53 KO # 1, H460 P53 KO # 7 and H460 P53 KO # 12 cell suspensions were added to two types of 24-well plates at 495 µL per well, with a cell density of 1 × 10⁴ /well. A 24-well plate fitted with glass insert was used for hypoxia experiments, while a normal plastic 24-well plate was used for normoxia experiments.
2) The cells were cultured overnight in an incubator at 37°C, 5% CO₂.
3) Compound Treatment

### Hypoxic Conditions:

The hypoxia workstation was adjusted to a hypoxic environment (O₂ < 0.01%), and the hypoxic condition in the workstation was verified using an oxygen indicator.

After 24 hours of cell plating, the 24-well plate fitted with glass insert was transferred into the hypoxic workstation.

The 24-well plate was placed on the shaker and shaken with the plate lids open for gas exchange for 5 minutes. Each well was added with 5 µL of corresponding compounds at 100 times concentration. Each experimental group set 3 replicate wells.

The plate was shaken gently to ensure even mixing of the compounds. The plate lids were partially opened, and the plates were incubated in the hypoxia workstation for 3 hours.

### Normoxic Conditions:

After 24 hours of cell plating, each well was added with 5 µL of corresponding compounds at 100 times concentration. Each experimental group set 3 replicate wells.

The plate was shaken gently to ensure even mixing of the compounds. The 24-well plate was incubated in an standard incubator at 37°C, 5% CO₂ for 3 hours.

4) All 24-well plates were washed twice with complete medium at 500 µ L per well per wash.

5) 1000 µL of medium was added to each well.

6) The plates were placed in an incubator at 37°C, 5% CO₂ for 72 hours.

7) From each well 800µL of medium was discarded. Then each well was added with 50 µL CTG, shaken for even mixing for 2 min and placed at room temperature away from light for 15 minutes.

8) 100 µL of medium was transferred from each well of the 24-well plates to 96-well white plates.

9) The chemiluminescence signal value was read using a multifunctional microplate reader with a reading time of 1000 ms.

10) The ICso (half maximal inhibitory concentration) values of the compounds were calculated using GraphPad Prism 5 software, with the ICso values derived from the following non-linear fitting equation.

In this example, the above experiments were carried out with the specific compound A of the structural formula (1) and the specific compound B of the structural formula (2), and the structures of the compounds A and B are as follows:

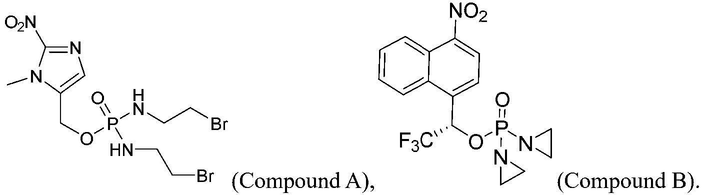

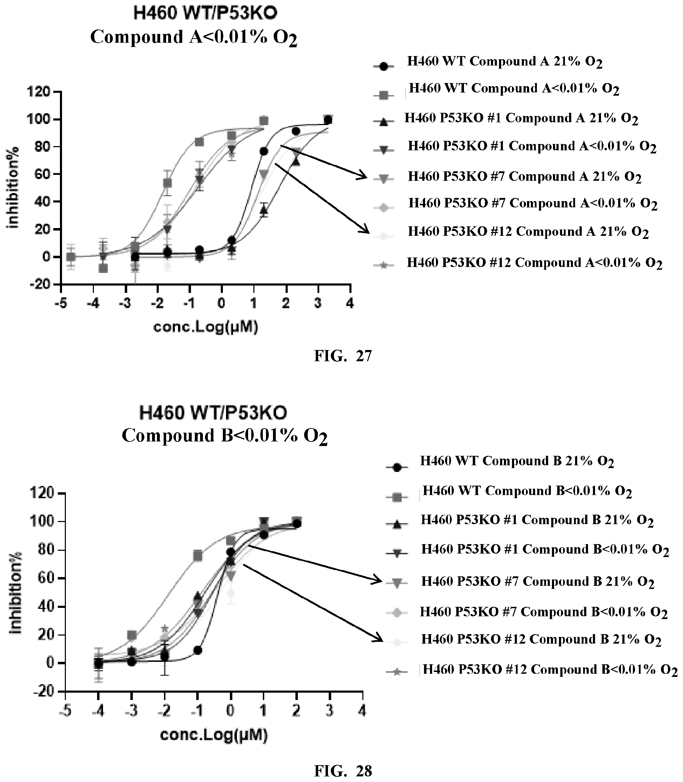

The experimental results of the compound A are shown in Table 13. The corresponding *in vitro* proliferation inhibition rate curves of compound A on H460 cells and H460 P53 KO cells are shown in Figure 27.

The experimental results of the compound B are shown in Table 14. The corresponding *in vitro* proliferation inhibition rate curves of compound B on H460 cells and H460 P53 KO cells are shown in Figure 28.

**Table 13: Inhibition rate results of the compound A on in vitro proliferation of H460 and H460 P53 KO #1, H460 P53 KO #7 and H460 P53 KO #12.**

| Cell line/compound combination | IC₅₀ (uM) | Ratio, 21% O₂/ <0.01% O₂ | Ratio, knockout / wild 21% O₂ | Ratio, knockout / wild<0.01% O₂ |
|---|---|---|---|---|
| H460 WT A 21% O₂ | 8.26 | | | |
| H460 WT A<0.01% O₂ | 0.0144 | 573.61 | | |
| H460 P53KO # 1 A21% O₂ | 61.52 | | 7.45 | |
| H460 P53KO # 1 A<0.01% O₂ | 0.1746 | 352.35 | | 12.13 |
| H460 P53KO # 7 A 21% O₂ | 12.9 | | 1.56 | |
| H460 P53KO # 7 A<0.01% O₂ | 0.1478 | 87.28 | | 10.26 |
| H460 P53KO # 12 A 21% O₂ | 17.37 | | 2.1 | |
| H460 P53KO # 12 A <0.01% O₂ | 0.0951 | 182.65 | | 6.6 |

The experimental data shown in Table 13 and Figure 27 show that under hypoxic conditions, the sensitivity of H460 P53 KO #1, #7 and #12 to the compound A was decreased by 12.13-fold, 10.26-fold and 6.60-fold, compared with H460 P53 WT cells, while these cells still had good hypoxia selectivity. In other words, p53 protein has a determinant effect on the efficacy of the compound A (DNA alkylating agents), which further corroborates the experimental results of the above examples that the DNA alkylating agents exert a stronger proliferation inhibitory effect on cells with negative p53 gene mutation and normal p53 protein.

**Table 14: Inhibition rate results of the compound B on in vitro proliferation of H460 and H460 P53 KO #1, H460 P53 KO #7 and H460 P53 KO #12.**

| Cell line/compound combination | IC₅₀(uM) | Ratio,21% O₂/ <0.01% O₂ | Ratio,0.01% O₂/_{<}21% O2 | Ratio, knockout / wild 21% O₂ | Ratio, knockout / wild < 0.01% O₂ |
|---|---|---|---|---|---|
| H460 WT B 21% O₂ | 0.4 | | | | |
| H460 WT B<0.01% O₂ | 0.013 | 30.77 | | | |
| H460 P53KO # 1 B 21% O₂ | 0.17 | | | 0.43 | |
| H460 P53KO # 1 B < 0.01% O₂ | 0.29 | | 1.71 | | 22.31 |
| H460 P53KO # 7 B 21% O₂ | 0.27 | | | 0.68 | |
| H460 P53KO # 7 B < 0.01% O₂ | 0.36 | | 1.33 | | 27.69 |
| H460 P53KO # 12 B 21% O₂ | 0.47 | | | 1.18 | |
| H460 P53KO # 12 B < 0.01% O₂ | 0.13 | 3.62 | | | 10.00 |

The experimental data shown in Table 14 and Figure 28 show that under hypoxic conditions, the sensitivity of P53 KO #1, #7 and #12 to the compound B was decreased by 22.31-fold, 27.69-fold and 10.00-fold, compared with H460 P53 WT cells, while these cells had reduced hypoxia selectivity. In other words, p53 protein has a determinant effect on the efficacy of the compound B (DNA alkylating agents), which further corroborates the experimental results of the above examples that the DNA alkylating agents exert a stronger proliferation inhibitory effect on cells with negative p53 gene mutation and normal p53 protein.

Example 12: Effects of the presence and absence of Nutlin-3 on Total P53, MDM2, P21, AKR1C3 and Actin proteins in H460 and H460 P53 KO cells

A P53 gene knockout (KnockOut) cell line was constructed with number of H460 P53 KO # 8, by the same method as described in example 10.

### Overview of the experimental process:

1) H460 and H460 P53 KO cell suspensions were added to a 24-well plate, with a cell density of 100000 /well and 995 µL of medium per well.
2) The cells were cultured overnight in an incubator at 37°C, 5% CO₂.
3) Compound Treatment

After 24 hours of cell plating, according to the protocol, each cell line experiment was grouped as:
H460 WT untreated group, H460 P53 KO # 1 untreated group, H460 P53 KO # 7 untreated group, H460 P53 KO # 8 untreated group, H460 P53 KO # 12 untreated group, H460 WT 0.1% DMSO treated group, H460 WT 5 µM Nutlin-3 treated group, H460 P53 KO # 1 5 µM Nutlin-3 treated group, H460 P53 KO # 7 5 µM Nutlin-3 treated group, H460 P53 KO # 8 5 µM Nutlin-3 treated group, H460 P53 KO # 12 5 µM Nutlin-3 treated group.

Monotherapy: After 24 hours of cell plating, each well was supplied with 5µL of test compounds at different concentrations, shaken gently to ensure even mixing, and then placed at 37°C, 5% CO₂ in an incubator.

4) After incubating the 24-well plates in a 37°C, 5% CO₂ incubator for 24h, the cell protein lysate was collected for WB detection.

### WB detection method:

1) The cell plates were taken back, the medium supernatant was discarded, and the plates were gently rinsed with PBS. 30 µL of RIPA protein lysate (RIPA: phosphatase inhibitor = 10: 1) was added to each well. The cells were scraped off with a cell scraper, lysed on ice for 30 min, and centrifuged at 14000 rpm and 4°C for 10 min.
2) Protein quantification was performed using the BCA method. Then protein loading and gel electrophoresis were carried out: protein samples were separated using 4-12% precast SDS-PAGE gels. The protein samples loaded from left to right were as follows: H460 WT untreated group, H460 P53 KO # 1 untreated group, H460 P53 KO # 7 untreated group, H460 P53 KO # 8 untreated group, H460 P53 KO # 12 untreated group, H460 WT 0.1% DMSO treated group, H460 WT 5 µM Nutlin-3 treated group, H460 P53 KO # 1 5 µM Nutlin-3 treated group, H460 P53 KO # 7 5 µM Nutlin-3 treated group, H460 P53 KO # 8 5 µM Nutlin-3 treated group, H460 P53 KO # 12 5 µM Nutlin-3 treated group. The protein loading volume for each sample was 10 µg/12.5 µL. After gel electrophoresis, protein transfer was performed under the conditions of 100 V for 1 h.
3) Antibody incubation and chemiluminescence detection: After protein transfer, the membrane was blocked with 5% non-fat milk on a horizontal shaker for 1 h at room temperature. The respective antibodies were added at an antibody ratio of 1: 1000. The membrane was incubated overnight at 4°C in an antibody incubation box. The next day, the membrane was rewarmed at room temperature for 1 h and then washed three times with TBST for 10 min each time. After washing, corresponding secondary antibodies (diluted at 1:4000) were incubated according to the type of primary antibodies. The incubation time was 2 h. After the incubation, the wash was also performed for 3 three times with TBST for 10 min each time. Solutions A and B of the ECL luminescence solution (SuperSignal West Femto Maximum Sensitivity) were mixed and added to the membrane dried away from water. Chemiluminescence detection was performed using a gel imager.

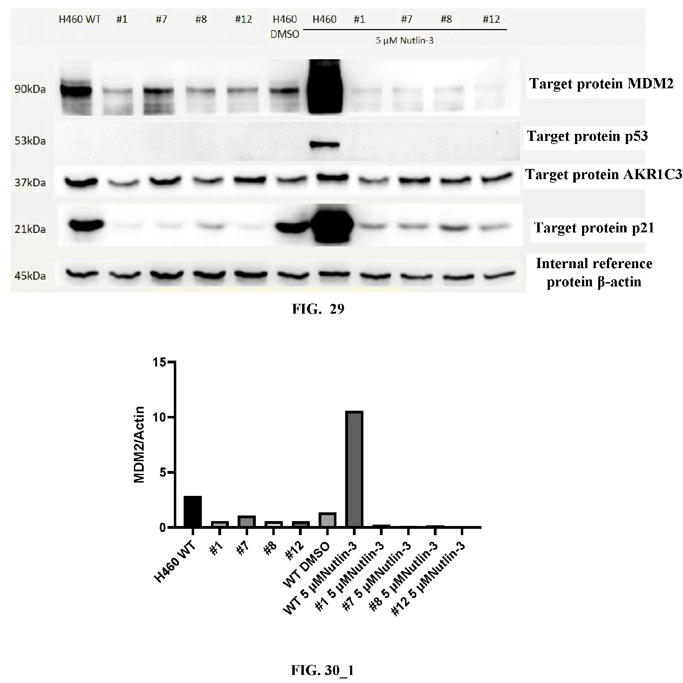

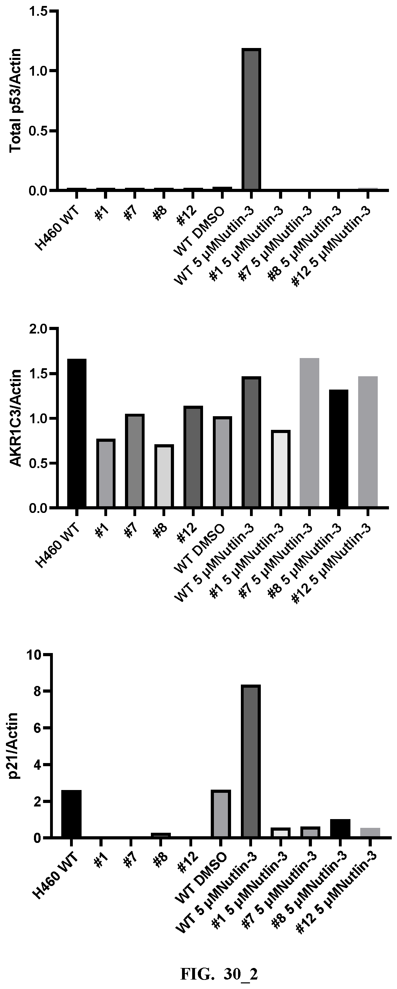

The protein band images from the WB detection of cell protein lysates are shown in Figure 29. The ratios of the corresponding protein to the internal reference protein β-actin are shown in Figure 30.

The internal reference protein actin of all cell lines and under various treatments in the above experiments remained unchanged.

The experimental data shown in Figure 29 and Figure 30 show that, compared with wild-type H460 cells, the cells with p53 knockout had decreased protein expression of AKR1C3 (more significantly in #1) and simultaneously reduced expression of MDM2 and P21. Compared with the H460 wild-type DMSO group, it was found that the expressions of MDM2, P53, P21 and AKR1C3 proteins were all upregulated after treating wild-type H460 with the positive drug Nutlin-3. However, after treatment of wild-type H460 and H460 P53 KO cell line with Nutlin-3, it was found that all the expressions of MDM2, P53, P21 and AKR1C3 proteins were not upregulated in H460 P53 KO cells compared to H460 wild-type DMSO group, especially in H460 P53 KO # 1 cells. These results suggest that H460 P53 KO may downregulate the protein expression of AKR1C3, which is consistent with the experimental results of IC₅₀ values showing that compound AST loses AKR1C3 selectivity in H460 P53 KO cells in Table 12 of example 10 and Figure 26.

### Example 13: Effects of the compound C and AST-3424 on the in vitro proliferation of NCI-H460 cells and NCI-H460 P53KO #1 cells under normoxia conditions

This example further verified the effects of the specific compound C of structural formula (9) on the *in vitro* proliferation of NCI-H460 cells and NCI-H460 P53KO #1 cells under normoxia conditions. The structure of compound C is as follows:

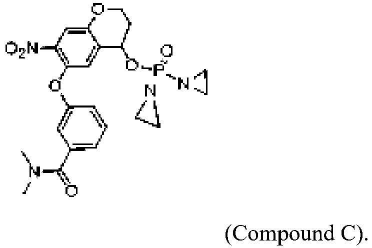

### Overview of the experimental process:

1) NCI-H460, NCI-H460 P53KO # 1 cell suspensions were added to a 96-well plate at 100 µL per well, with a cell density of 2000 /well.
2) The cells were cultured overnight in an incubator at 37°C, 5% CO₂.
3) Compound Treatment

Monotherapy: After 24 hours of cell plating, 99.5 µL of growth medium was supplied to each well. Then each well was added with 0.5µL of test compounds at different concentrations, shaken gently to ensure even mixing, and then placed at 37°C, 5% CO₂ in an incubator.

Combination therapy: After 24 hours of cell plating, 99 µL of medium was supplied to each well. Each well was added with 0.5µL of combined action compound, shaken gently to ensure even mixing, and then placed in an incubator for 2 hours. Then each well was added with 0.5µL of test compounds at different concentrations, shaken gently to ensure even mixing, and then placed at 37°C, 5% CO₂ in an incubator.

4) The cell plate was placed in the incubator for 72 hours.

5) The cell plate to be tested was placed at room temperature to equilibrium for 30 minutes, and 100µL of medium was discarded per well.

6) 25µL of CTG reagent was added to each well, placed at a fast shaker to shake for 2 minutes, and placed at room temperature away from light for 30 minutes.

7) The chemiluminescence signal value was read using a multifunctional microplate reader with a reading time of 1000 ms.

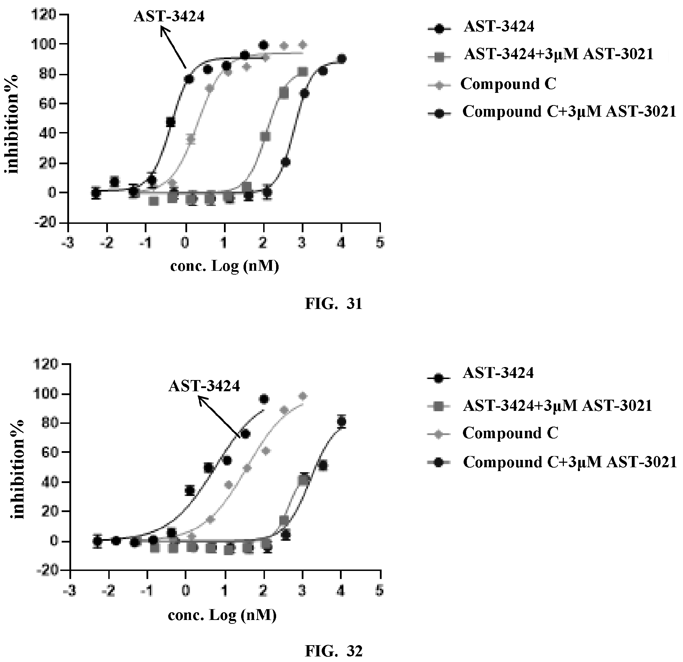

8) The ICso (half maximal inhibitory concentration) values were calculated using GraphPad Prism 5 software. The ICso results detected in the experiment are shown in Table 15. The *in vitro* proliferation inhibition rate curves on NCI-H460 and NCI-H460 P53 KO #1 cells are shown in Figures 31 and 32.

**Table 15: Inhibition rate results of the compound C and AST-3424 on in vitro proliferation of NCI-H460 and NCI-H460 P53 KO #1.**

| Cell line | Compounds | IC₅₀(nM) | Ratio, combination therapy/ monotherapy | Ratio, Compound C/AST-3424 | Ratio, knockout / wild |
|---|---|---|---|---|---|
| NCI-H460 | AST-3424 | 0.43 | | | |
| | AST-3424 + 3 µM AST-3021 | 121.00 | 281.40 | | |
| | Compound C | 2.05 | | 4.77 | |
| | Compound C+3 µM AST-3021 | 626.00 | 305.37 | | |
| NCI-H460 P53KO # 1 | AST-3424 | 5.45 | | | 12.67 |
| | AST-3424 + 3 µM AST-3021 | >1000 | > 183.49 | | |
| | Compound C | 36.08 | | 6.62 | 17.60 |
| | Compound C+3 µM AST-3021 | 1560.00 | 43.24 | | |

The above experimental data show that:
The IC₅₀ of the compound C in NCI-H460 cells is 2.05 nM, which is 4.77 times of the IC₅₀ of AST-3424. There was no significant difference in the selectivity to AKR1C3 between the compound C and AST-3424.

After p53 knockout, IC₅₀ of the Compound C shows 17.60-fold increase, and IC₅₀ of AST-3424 shows 12.67-fold increase, indicating that P53 protein can increase the cytotoxicity of both compound C and AST-3424.

After p53 knockout, the ratio of the ICso the compound C in combination with the AKR1C3 enzyme inhibitor AST-3021 to that of the compound C monotherapy decreased from 305.37-fold to 43.24-fold, indicating that the AKR1C3 selectivity of the compound C was significantly reduced upon p53 knockout.

In other words, the p53 protein has a determinant effect on the efficacy of the compound C (DNA alkylating agents), which further corroborates the experimental results of the above examples that the DNA alkylating agents exert a stronger proliferation inhibitory effect on cells with negative p53 gene mutation and normal p53 protein.

## Claims

1. A treatment method comprising using a DNA alkylating agent prodrug compound-containing single drug or using the single drug and other drugs in combination for treatment of cancer and tumor patients with negative result of p53 gene mutation detection or normal p53 protein expression.

2. The treatment method according to claim 1, wherein the treatment method uses a single drug containing hypoxia-activated DNA alkylating agent prodrug compound, AKR1C3-activated DNA alkylating agent prodrug compound, β-D-Glucosidase- or β-galactosidase-activated DNA alkylating agent prodrug compound or in combination with other drugs for the treatment of cancer and tumor patients with negative result of p53 gene mutation detection or normal p53 protein expression, wherein the hypoxia-activated DNA alkylating agent prodrug compound is selected from formulae 1-3 and salt, ester, solvate, isotopic isomer thereof, the AKR1C3-activated DNA alkylating agent prodrug compound is selected from formulae 4-12 and salt, ester, solvate, isotope isomer thereof, and the β-D-Glucosidase- or β-galactosidase-activated DNA alkylating agent prodrug compound is selected from formula 15: 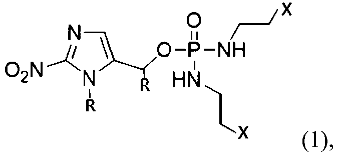 wherein R is each independently selected from H, -CH₃, -CH₂CH₃, -CF₃, and X is each independently selected from leaving groups Cl, Br, MsO, TsO and the like; 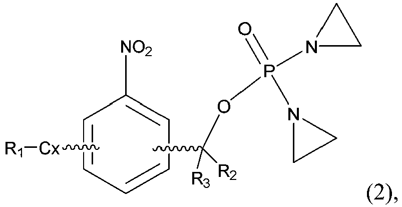 wherein, the definitions of R₁, R₂, R₃, and Cx are as described in the claims of Patent Application PCT/CN2020/114519 with Publication No. WO2021120717A1 (corresponding to the Chinese application No. 2020800673113 with Publication No. CN114466853A); 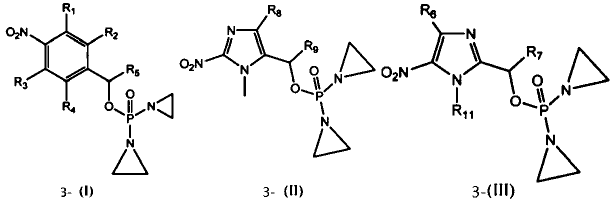 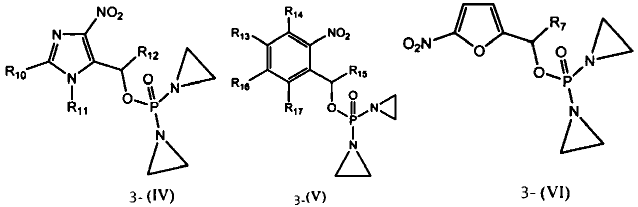 wherein, the definitions of R₁, R₂, R₃, R₄, R₅, R₆, R₇, R₈, R₉, R₁₀, R₁₁, R₁₂, R₁₃, R₁₄, R₁₅, R₁₆, and R₁₇ are as described in the claims of Patent Application PCT/US2016/039092 with Publication No. WO2016210175A1 (corresponding to Chinese application No. 2016800368985 with Publication No. CN108024974A); 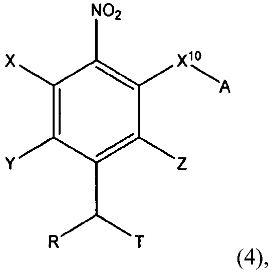 wherein, the definitions of X, Y, Z, R, T, A and X¹⁰ are as described in the claims of Patent Application PCT/US2016/021581 with Publication No. WO2016145092A1 (corresponding to Chinese application No. 2016800150788 with Publication No. CN107530556A); 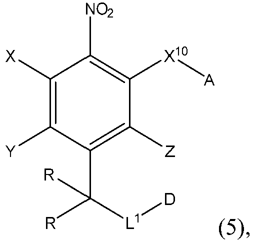 wherein, the definitions of X, Y, Z, R, D, L¹, A and X¹⁰ are as described in the claims of Patent Application PCT/US2016/025665 with Publication No. WO2016161342A3 (corresponding to Chinese application No. 2016800200132 with Publication No. CN108136214A); 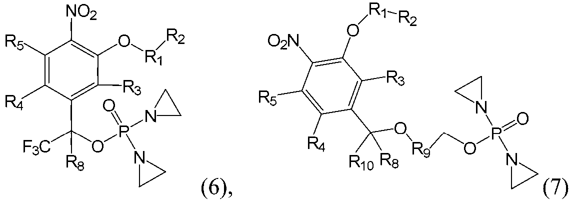 wherein, the definitions of R₁, R₂, R₃, R₄, R₅, R₈, R₉, R₁₀, R₉, and R₁₀ are as described in the claims of Patent Application PCT/CN2020/089692 with Publication No. WO2020228685A9 (corresponding to Chinese application No. 2020800358890 with Publication No. CN113853379A); 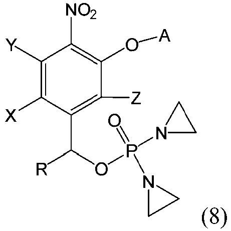 wherein:
A is a substituted or unsubstituted C₆-C₁₀ aryl, a biaryl or substituted biaryl, a 5-15-membered heteroaryl, or-N=CR¹R²; wherein the substituent is selected from the group consisting of halogeno, -CN, -NO₂, -O-(CH₂)-O-, -CO₂H and salt thereof, -OR¹⁰⁰, -CO₂R¹⁰⁰, -CONR¹⁰¹R¹⁰², -NR¹⁰¹R¹⁰², -NR¹⁰⁰SO₂R¹⁰⁰, -SO₂R¹⁰⁰, -SO₂NR¹⁰¹R¹⁰², C₁-C₆ alkyl, and C₃-C₁₀ heterocyclyl;
wherein R¹⁰⁰, R¹⁰¹ and R¹⁰² are each independently hydrogen, C₁-C₈ alkyl, or C₆-C₁₂ aryl; or R¹⁰¹ and R¹⁰² together with the nitrogen atom to which they are attached form a 5-7-membered heterocycle;
wherein the alkyl group and the aryl group are each substituted by 1-3 halogeno groups or 1-3 C₁-C₆ alkyl groups;
R¹ and R² are each independently phenyl or methyl;
X, Y and Z are each independently hydrogen or halogeno; and
R is hydrogen or C₁-C₆ alkyl or halogen-substituted alkyl; 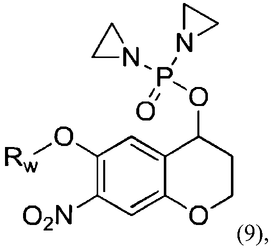
wherein the definitions of Rw are as described in the claims of Patent Application PCT/CN2020/120281 with Publication No. WO2021068952A1 (corresponding to the Chinese Application No. 202080071652.8 with Publication No. CN114555574A); 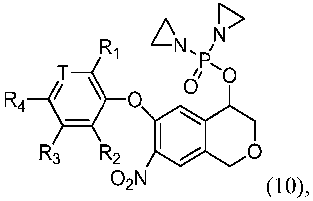
wherein the definitions of R₁, R₂, R₃, R₄ and T are as described in the claims of Patent Application PCT/CN2021/118597 with Publication No.WO2022057838A1; 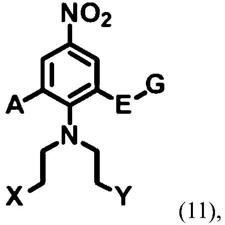
wherein, the definitions of A, E, G, X, and Y are as described in the claims of Patent Application PCT/NZ2019/050030 with Publication No. WO2019190331A1 (corresponding to Chinese application No. 2019800234236 with Publication No. CN111918864A); 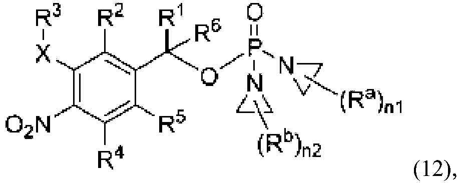 or pharmaceutically acceptable salt thereof,
wherein the definitions of R¹, R², R³, R⁴, R⁵, R⁶, R^{a}, R^{b}, n1 and n2 are as described in the claims of Patent Application PCT/CN2023/123253 with Publication No. WO2024078392A1;
the compound with formula (15) is selected from the compounds with the following structural formulae: 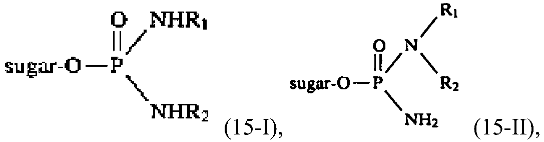
wherein the sugar moiety is attached to a phosphamide mustard residue (15-I) or an ifosfamide mustard residue (15-II), R₁ and R₂, which may be the same or different, are selected from hydrogen, C1-C4 alkyl or C1-C6 haloalkyl,
and the sugar moiety is an isomeric or enantiomeric form of any existing monosaccharides, disaccharides or polysaccharides.

3. The treatment method according to claim 1, wherein the DNA alkylating agent prodrug compound is selected from AKR1C3 activated DNA alkylating agent prodrug compound AST-3424:

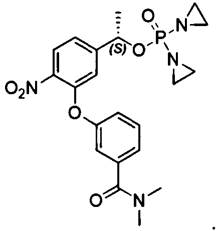

4. The treatment method according to claim 3, wherein AST-3424 is formulated as an aqueous solution for intravenous injection,
where the solute of the aqueous solution consists of the active pharmaceutical ingredient AST-3424, glucose, ethanol, propylene glycol and pH regulator sodium bicarbonate,
wherein the concentration of the active pharmaceutical ingredient AST-3424 in the aqueous solution is 0.004-0.94 mg/ml, the pH is 7.4, the content of glucose is 4.5-5.0% by mass and the solution is an isotonic solution; or
the solute of the aqueous solution solute consists of the active pharmaceutical ingredient AST-3424, sodium chloride, ethanol, propylene glycol and pH regulator sodium bicarbonate,
wherein the concentration of the active pharmaceutical ingredient AST-3424 in the aqueous solution is 0.004-0.94 mg/ml, the pH is 7.4, the content of sodium chloride is 0.81-0.90% by mass, and the solution is an isotonic solution.

5. The treatment method according to claim 3, wherein the AST-3424 dosing regimen is selected from any one of the following regimes:
regimen 1, every 21-day cycle, with one dose administered on Day 1 and Day 8 each, at a dose selected from 12 mg/m², 11 mg/m², 10 mg/m², 9 mg/m², 8 mg/m², 7 mg/m², 6 mg/m², 5 mg/m², 4 mg/m² per administration;
regimen 2, every 21-day cycle, with one dose administered on Day 1, at a dose selected from each 14 mg/m², 13 mg/m², 12 mg/m², 11 mg/m², 10 mg/m², 9 mg/m², 8 mg/m², 7 mg/m², 6 mg/m², 5 mg/m², 4 mg/m², 3 mg/m², 2 mg/m², 1 mg/m² per administration;
regimen 3, every 21-day cycle, with one dose administered once a day from Day 1 to Day 5, at a dose selected from 8 mg/m², 7 mg/m², 6 mg/m², 5 mg/m², 4 mg/m², 3 mg/m², 2 mg/m², 1 mg/m² per administration.

6. The treatment method according to claim 3, wherein the patient is a liver cancer patient.

7. The treatment method according to claim 5, wherein the liver tumor tissue of the patient is strongly positive for AKR1C3 expression, and the liver tumor tissue of the patient is judged to be strongly positive for AKR1C3 expression if one of the following conditions is met:
I. the H score is greater than or equal to 100, preferably greater than or equal to 135, as detected by the AKR1C3 detection method described in WO2022048492;
II. the proportion of tumor cells having a staining intensity of 2 + and/or 3 + is ≥ 70%, as detected by the AKR1C3 detection method described in WO2022048492.

8. The treatment method according to claim 3, wherein the drug containing AST-3424 is used in combination with the drug containing any one selected from abiraterone acetate or an abiraterone drug and a prednisolone combination drug, 5-fluorouracil, sunitinib, gemcitabine, oxaliplatin, a PD-1/L1 inhibitor, apatinib, sorafenib or donafenib, or elemene for the treatment of liver cancer.

9. The use of DNA alkylating agent prodrug compound in the manufacture of a single drug or a drug combined with other drugs for treating cancer and tumor patients with negative detection result of p53 gene mutation or normal expression of p53 protein.

10. The use according to claim 9, wherein the DNA alkylating agent prodrug compound is selected from the group consisting of hypoxia-activated DNA alkylating agent prodrug compounds, AKR1C3-activated DNA alkylating agent prodrug compounds, β-D-Glucosidase- or β-galactosidase-activated DNA alkylating agent prodrug compounds.

11. The use according to claim 10, wherein,
the hypoxia-activated DNA alkylating agent prodrug compound is selected from the group consisting of structural formulae 1-3 and salt, ester, solvate and isotopic isomer thereof,
the AKR1C3 activated DNA alkylating agent prodrug compound is selected from the group consisting of structural formulae 4-12 and salt, ester, solvate, isotopic isomer thereof,
and the β-D-Glucosidase or β-galactosidase activated DNA alkylating agent prodrug compound is selected from the group consisting of structural formula 15: 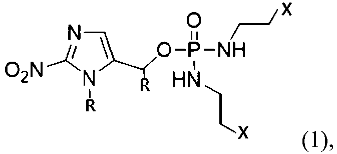
wherein R is each independently selected from H, -CH₃, -CH₂CH₃, -CF₃, and X is each independently selected from leaving groups Cl, Br, MsO, TsO and the like; 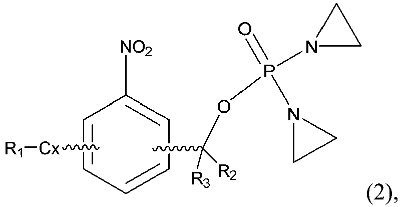
wherein, the definitions of R₁, R₂, R₃, and Cx are as described in the claims of Patent Application PCT/CN2020/114519 with Publication No. WO2021120717A1 (corresponding to the Chinese Patent Application No. 2020800673113 with Publication No. CN114466853A); 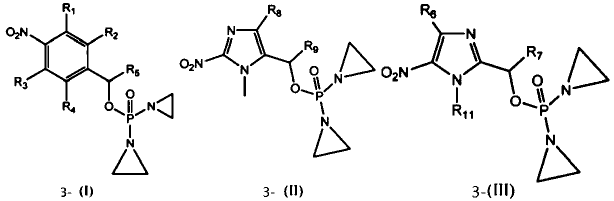 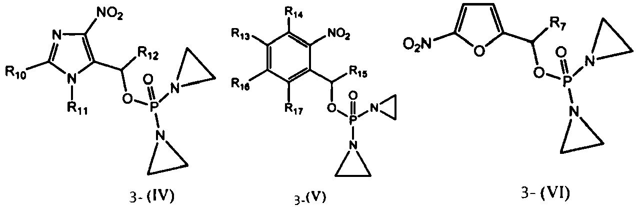
wherein, the definitions of R₁, R₂, R₃, R₄, R₅, R₆, R₇, R₈, R₉, R₁₀, R₁₁, R₁₂, R₁₃, R₁₄, R₁₅, R₁₆, and R₁₇ are as described in the claims of Patent Application PCT/US2016/039092 with Publication No. WO2016210175A1 (corresponding to Chinese Patent Application No. 2016800368985 with Publication No. CN108024974A); 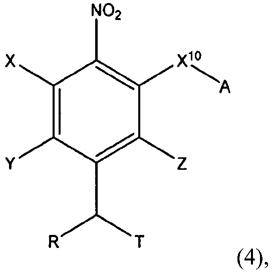
wherein, the definitions of X, Y, Z, R, T, A and X¹⁰ are as described in the claims of Patent Application PCT/US2016/021581 with Publication No. WO2016145092A1 (corresponding to Chinese Patent Application No. 2016800150788 with Publication No. CN107530556A); 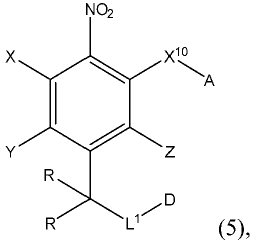
wherein, the definitions of X, Y, Z, R, D, L¹, A and X¹⁰ are as described in the claims of Patent Application PCT/US2016/025665 with Publication No. WO2016161342A3 (corresponding to Chinese Patent Application No. 2016800200132 with Publication No. CN108136214A); 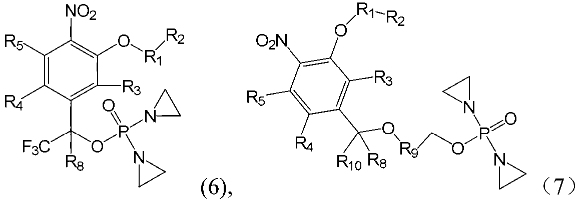
wherein, the definitions of R₁, R₂, R₃, R₄, R₅, R₈, R₉, and R₁₀ are as described in the claims of Patent Application PCT/CN2020/089692 with Publication No. WO2020228685A9 (corresponding to Chinese Patent Application No. 2020800358890 with Publication No. CN113853379A); 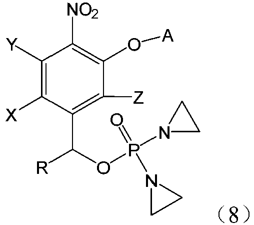
wherein:
A is substituted or unsubstituted C₆-C₁₀ aryl, biaryl or substituted biaryl, 5-15-membered heteroaryl, or - N=CR¹R²; wherein the substituent is selected from the group consisting of halogeno, -CN, -NO₂, -O-(CH₂)-O-, -CO₂H and salt thereof, -OR¹⁰⁰, -CO₂R¹⁰⁰, -CONR¹⁰¹R¹⁰², -NR¹⁰¹R¹⁰², -NR¹⁰⁰SO₂R¹⁰⁰, -SO₂R¹⁰⁰, -SO₂NR¹⁰¹R¹⁰², C₁-C₆ alkyl, and C₃-C₁₀ heterocyclyl;
wherein R¹⁰⁰, R¹⁰¹ and R¹⁰² are each independently hydrogen, C₁-C₈ alkyl, or C₆-C₁₂ aryl; or R¹⁰¹ and R¹⁰² together with the nitrogen atom to which they are attached form a 5-7-membered heterocycle;
wherein the alkyl group and the aryl group are each substituted by 1-3 halogeno groups or 1-3 C₁-C₆ alkyl groups;
R¹ and R² are each independently phenyl or methyl;
X, Y and Z are each independently hydrogen or halogeno; and
R is hydrogen or C₁-C₆ alkyl or halogen-substituted alkyl; 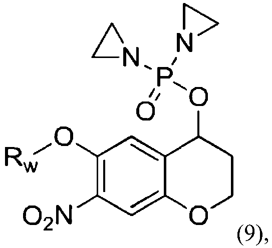
wherein, the definitions of Rw are as described in the claims of Patent Application PCT/CN2020/120281 with Publication No. WO2021068952A1 (corresponding to the Chinese application No. 202080071652.8 with Publication No. CN114555574A); 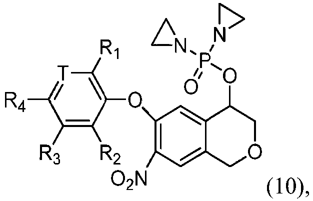
wherein the definitions of R₁, R₂, R₃, R₄ and T are as described in the claims of Patent Application PCT/CN2021/118597 with Publication No. WO2022057838A1; 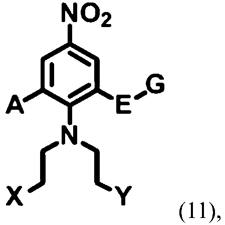
wherein, the definitions of A, E, G, X, and Y are as described in the claims of Patent Application PCT/NZ2019/050030, with Publication No. WO2019190331A1 (corresponding to Chinese Patent Application No. 2019800234236 with Publication No. CN111918864A); 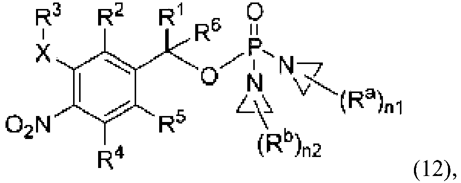
or pharmaceutically acceptable salt thereof, wherein the definitions of R¹, R², R³, R⁴, R⁵, R⁶, R^{a}, R^{b}, n1 and n2 are as described in the claims of Patent Application PCT/CN2023/123253 with Publication No. WO2024078392A1; 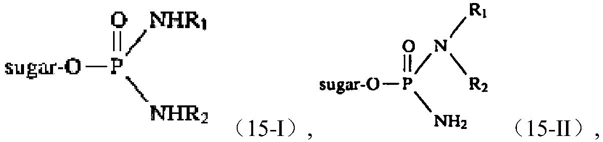
wherein the sugar moiety is attached to a phosphamide mustard residue (15-I) or an ifosfamide mustard residue (15-II), R₁ and R₂, which may be the same or different, are selected from hydrogen, C₁-C₄ alkyl or C₁-C₆ haloalkyl,
and the sugar moiety is an isomeric or enantiomeric form of any existing monosaccharides, disaccharides or polysaccharides.

12. The use according to claim 9, wherein,
the DNA alkylating agent prodrug compound is selected from AKR1C3 activated DNA alkylating agent prodrug compound AST-3424:

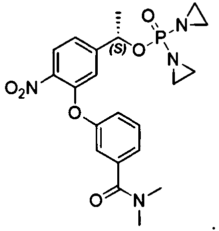

13. The use according to claim 12, wherein the AST-3424 is formulated as an aqueous solution for intravenous injection,
where the solute of the aqueous solution consists of the active pharmaceutical ingredient AST-3424, glucose, ethanol, propylene glycol and pH regulator sodium bicarbonate,
wherein the concentration of the active pharmaceutical ingredient AST-3424 in the aqueous solution is 0.004-0.94 mg/ml, the pH is 7.4, the content of glucose is 4.5-5.0% by mass and the solution is an isotonic solution;
or
the solute of the aqueous solution consists of the active pharmaceutical ingredient AST-3424, sodium chloride, ethanol, propylene glycol and pH regulator sodium bicarbonate,
wherein the concentration of the active pharmaceutical ingredient AST-3424 in the aqueous solution is 0.004-0.94 mg/ml, the pH is 7.4, the content of sodium chloride is 0.81-0.90% by mass, and the solution is an isotonic solution.

14. The use according to claim 12, wherein the AST-3424 dosing regimen is selected from any one of the following regimen:
regimen 1, every 21-day cycle, with one dose administered on Day 1 and Day 8 each, at a dose selected from 12 mg/m², 11 mg/m², 10 mg/m², 9 mg/m², 8 mg/m², 7 mg/m², 6 mg/m², 5 mg/m², 4 mg/m² per administration;
regimen 2, every 21-day cycle, with one dose administered on Day 1, at a dose selected from each 14 mg/m², 13 mg/m², 12 mg/m², 11 mg/m², 10 mg/m², 9 mg/m², 8 mg/m², 7 mg/m², 6 mg/m², 5 mg/m², 4 mg/m², 3 mg/m², 2 mg/m², 1 mg/m² per administration;
regimen 3, every 21-day cycle, with one dose administered once a day from Day 1 to Day 5, at a dose selected from 8 mg/m², 7 mg/m², 6 mg/m², 5 mg/m², 4 mg/m², 3 mg/m², 2 mg/m², 1 mg/m² per administration.

15. The use according to claim 12, wherein the patient is a liver cancer patient.

16. The use according to claim 12, wherein the liver tumor tissue of the patient is strongly positive for AKR1C3 expression, and it is judged that the liver tumor tissue of the patient is strongly positive for AKR1C3 expression if one of the following conditions is met:
1) the H score is greater than or equal to 135 as detected by the AKR1C3 detection method described in WO2022048492;
2) the proportion of tumor cells having a staining intensity of 2 + and/or 3 + is ≥ 70%, as detected by the AKR1C3 detection method described in WO2022048492.

17. The use according to claim 12, wherein the drug containing AST-3424 is use in combination with a drug containing any one selected from abiraterone, prednisolone, 5-fluorouracil, sunitinib, gemcitabine, oxaliplatin, PD-1/L1 inhibitor, apatinib, sorafenib or donafenib, or elemene for the treatment of liver cancer.
